# EUROPEAN PATENT SPECIFICATION

(11) **EP 4 182 323 B1**
(45) Date of publication and mention of the grant of the patent: **24.04.2024**
(21) Application number: 21746406.4
(22) Date of filing: 14.07.2021
(51) Int. Cl.: C07D 498/04, A61K 31/5383, A61P 11/00

(54) **PYRIDO OXAZINE AMINO DERIVATIVES AS ALK5 INHIBITORS**
PYRIDO-OXAZIN-AMINODERIVATE ALS ALK5-INHIBITOREN
DÉRIVÉS DE PYRIDO-OXAZINE AMINO EN TANT QU'INHIBITEURS D'ALK5

(30) Priority: 15.07.2020 EP 20185900
(43) Date of publication of application: 24.05.2023
(73) Proprietor: Chiesi Farmaceutici S.p.A., 43122 Parma (IT)
(72) Inventor: PALA, Daniele, 43122 Parma (IT); PIZZIRANI, Daniela, 43122 Parma (IT); BRUNO, Paolo, 43122 Parma (IT); BIAGETTI, Matteo, 43122 Parma (IT); RONCHI, Paolo, 43122 Parma (IT); GUARIENTO, Sara, 43122 Parma (IT); FIORELLI, Claudio, 43122 Parma (IT); BERTANI, Barbara, 43122 Parma (IT)
(74) Representative: Chiesi Farmaceutici S.p.A.
(86) International application number: PCT/EP2021/069657
(87) International publication number: WO 2022/013312

(56) References cited:
- WO-A1-03/097639
- WO-A1-2011/146287
- WO-A1-2018/215668
- WO-A1-2020/012357

## Description

### FIELD OF THE INVENTION

The present invention generally relates to compounds inhibiting the transforming growth factor β (TGF β) type I receptor (ALK5) (hereinafter ALK5 inhibitors), methods of preparing such compounds, pharmaceutical compositions containing them and therapeutic use thereof.

The compounds of the invention may be useful for instance in the treatment of many diseases, disorders, or conditions associated with ALK5 signaling pathway.

### BACKGROUND OF THE INVENTION

The Transforming Growth Factor β (TGF β) is a protein belonging to the TGF β superfamily.

It is involved in several processes, both cellular, such as proliferation, migration and differentiation, and biological, including wound healing, immunesuppression, cancerogenesis and extracellular matrix production.

The TGF β superfamily also includes, among others, other members known as activins (Acts) (see e.g. Hinck AP, FEBS Letters 586 (2012); 1860-1870).

The binding of the peptide initiates the TGF β signaling cascade through the formation of a heterotetrameric complex composed of two different serine/threonine kinases receptors: type 1 (TGFβR1/ALK5) and type 2 (TGFβR2).

TGFβR1/ALK5 is recruited and activated through the phosphorylation of its intracellular domain by TGFβR2, leading in turn to the phosphorylation of the receptoractivated (R)-Smad family, resulting in the activation of target gene transcription (see e.g. Sheppard D., Proc Am Thorac Soc. (2006);(3):413-417).

Similarly to the TGF β signaling, the type I receptor for activin, ALK4, leads to the activation of target gene transcription (see e.g. Heldin CH et al., Cold Spring Harb Perspect Biol. (2016) Aug 1;8(8)).

Several studies have linked an excessive and/or dysregulated TGFβ activity with many diseases including cancer and fibrosis (see e.g. Syed V, J Cell Biochem. (2016) Jun;117(6):1279-87; Jakowlew SB. Cancer Metastasis Rev. (2006) Sep;25(3):435-57). Among fibrotic disorders, a crucial role of TGFβ has been shown in organs such as lung, heart, liver, and kidney (see e.g. Alhamad EH, J Thorac Dis. (2015);7(3):386-93). In particular, TGFβ expression is increased in fibrotic lung diseases, such as idiopathic pulmonary fibrosis (IPF), and in chronic inflammatory conditions, such as chronic obstructive pulmonary disease and asthma (see e.g. Thomas BJ et al., Am J Respir Cell Mol Biol. (2016);(55):759-766).

In lung, TGFβ is expressed in several cell types, like epithelial cells, endothelial cells, connective tissue cells, macrophages and fibroblasts.

These cell populations may produce excess of TGFβ in IPF human lung tissue. Moreover, high levels of TGFβ have been detected in lung tissue and BAL of IPF patients (see e.g. Bergeron A et al., Eur Respir J (2003);22:69-76).

TGFβ gene expression and TGFβ protein production have been observed to increase in a variety of animal models of pulmonary fibrosis caused by bleomycin, silica, asbestos, and radiation (see e.g. Wei F et al., Int Immunopharmacol. (2017) Jul;48:67-75; Choe JY et al., Inflamm Res. (2010) Mar;59(3):177-88; Wang X et al., Respir Res (2009);10, 36) and it has also been reported how the TGFβ expression is sufficient to induce progressive fibrosis in rodents (see e.g. Sime PJ et al., J Clin Invest (1997);100:768-776; Kim KK et al.).

Contrarily, TGFβ signaling inhibition obtained by employing knockout (KO) animals can inhibit fibrosis development through TGFβ-linked mechanisms (see e.g. Bonniaud P et al., Am J Respir Crit Care Med (2005);171:889-898; 34).

Similar results have been achieved with inhibition of TGFβR1 in mouse bleomycin disease model (see e.g. Wei Y et al., J Clin Invest. (2017);127(10):3675-3688).

Activin signaling dysregulation, similarly to TGFβ, is associated to fibroblasts proliferation, myofibroblasts differentiation and accumulation of extracellular matrix (ECM) (see e.g. Yamashita et al., J. Am. Soc. Nephrol. (2004) 15, 91-101). Moreover, overexpression of activin has been linked to pathological conditions and fibrosis development in different organs, such as liver (see e.g. Patella et al., Am. J. Physiol. Gastrointest. Liver Physiol. (2006) 290, G137-G144), kidney (see e.g. Agapova et al., Kidney Int. (2016) 89, 1231-1243), heart (see e.g. Yndestad et al., Circulation (2004) 109,1379-1385), and lung (see e.g. de Kretser et al., Crit.Care (2013) 17:R263).

Taken together these data suggest the importance of targeting ALK5 receptor to treat pharmacologically the aforementioned diseases, linked to dysregulated TGF signaling pathway.

The TGFβ signaling is strongly involved in the cardiovascular homeostasis (see e.g. van Meeteren LA et al., Springer (2013)). Several studies in humans and mice have shown the main role of TGFβ in angiogenesis and vascular morphogenesis. Moreover, TGFβ plays a key role in the development and functionality of cardiac valves. It is therefore clear the importance of a selective regulation of TGFβ pathway to target the pathological effects avoiding the suppression of the signaling needed for a correct homeostasis.

The answer to this crucial point could be addressed by using the inhalation route to deliver an antiTGFβ drug.

The inhalatory route would allow the treatment of the affected lung compartment bypassing the issue of the heart exposure.

Various compounds have been described in the literature as ALK5 and/or ALK4 inhibitors.

WO2008/006583, WO2009/087212, WO2009/087224, WO2009/087225, WO2009/133070, WO2009/013335 and WO2009/050183 (Novartis) disclose respectively pyrimidine, pyridine, imidazo pyridine, pyrrolo pyrimidine and pyrrolo pyridine, imidazo pyridazine, imidazo pyridine derivatives for the treatment of ALK4 or ALK5 mediated diseases useful for the treatment of inflammatory or obstructive airways diseases, pulmonary hypertension and pulmonary fibrosis.

WO00/61576 and US2003/0149277 (Smithkline Beecham Corp) disclose triarylimidazole derivatives as ALK5 inhibitors useful for the treatment of, among others, renal disease, wound healing, kidney disease, congestive heart failure, ulcers, impaired neurological function and any disease wherein fibrosis is a major component.

WO01/62756 (Smithkline Beecham P.L.C.) discloses pyridinylimidazole derivatives as ALK5 inhibitors useful for the treatment of, among others, renal disease, wound healing, kidney disease, congestive heart failure, ulcers, impaired neurological function and any disease wherein fibrosis is a major component.

WO03/087304 (Biogen Inc.) discloses tri-substituted heteroaryls as ALK5 and/or ALK4 inhibitors useful for the treatment of, among others, idiopathic pulmonary fibrosis, diabetic nephropathy, hepatic fibrosis, pulmonary fibrosis, acute lung injury, postinfarction cardiac fibrosis, fibrotic cancers and fibroma.

WO2013/009140 (SK Chemicals Co) discloses 2-pyridyl substituted imidazole derivatives as ALK5 and/or ALK4 receptors useful for the treatment of, among others, renal-, liver- or pulmonary fibrosis.

Pyrido oxazine amino derivatives have been disclosed in the literature, but not as ALK5 inhibitors.

WO2018/215668 (Glenmark) discloses, among other compounds, pyrido oxazine amino derivatives as inhibitors of MAP4K1, wherein the amino group is linked to a substituted aryl ring. These compounds are disclosed as useful for the treatment of autoimmune, neurodegenerative, neurological, inflammatory, hyperproliferative and cardiovascular diseases.

Of note, inhibition of ALK5 receptor may be useful for the treatment of fibrosis and diseases, disorders and conditions that result from fibrosis.

Several efforts have been done in the past years to develop novel ALK5 receptor inhibitors useful for the treatment of several diseases and some of those compounds have shown efficacy also in humans.

However, there remains a potential for developing inhibitors of receptors ALK5 characterized by good potency, useful for the treatment of diseases or conditions associated with a dysregulation of ALK5 signaling pathway, in particular fibrosis.

In particular, there remains a potential for developing inhibitors of receptor ALK5 useful for the treatment of diseases or conditions associated with a dysregulation of ALK5 signaling in the respiratory field, in particular idiopathic pulmonary fibrosis (IPF), to be administered by the inhalation route and characterized by a good inhalatory profile, that corresponds to a good activity in the lung, a good lung retention and to a low metabolic stability in order to minimize the systemic exposure and correlated safety issues.

In this direction, we have surprisingly found a new series of compounds of general formula (I) that solves the problem of providing potent inhibitors of ALK5 receptor for administration by inhalation, that shows, at the same time, a good inhalatory profile, low metabolic stability, low systemic exposure, improved safety and tolerability, and a good selectivity across the kinome.

### SUMMARY OF THE INVENTION

In a first aspect the present invention relates to compounds of formula (I) 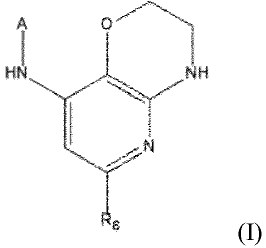 wherein
**A** is selected from the group consisting of **A₁** and **A₂** 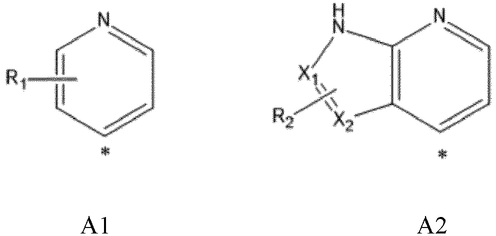
**X₁** and **X₂** are independently C or N;
**R₁** is H or is selected from the group consisting of -NR₃R₄, -C(O)NR₃R₅,-NR₃C(O)R₆ and -C(O)OR₇;
**R₂** is H or is selected from the group consisting of -C(O)OR₃ and -C(O)NR₃R₆; or **R₂** is absent when X₁ or X₂ is N;
**R₃** is H or -(C₁-C₆)alkyl;
**R₄** is H or -(C₁-C₆)alkyl;
**R₅** is H or is selected from the group consisting of -(C₁-C₆)alkyl, -(C₁₋C₆)alkylene-NR_{A}R_{B}, cycloalkyl, heterocycloalkyl optionally substituted by one or more -(C₁₋ C₆)alkyl, and -(C₁₋C₆)alkylene-heterocycloalkyl, wherein said heterocycloalkyl is optionally substituted by one or more groups selected from -(C₁-C₆)alkyl, -C(O)-(C₁₋C₆)alkyl and -SO₂-(C₁₋C₆)alkyl;
**R₆** is selected from the group consisting of -(C₁₋C₆)alkylene-NR₃R₄ and -(C₁₋ C₆)alkylene-heterocycloalkyl, wherein said heterocycloalkyl is optionally substituted by one or more -(C₁-C₆)alkyl;
**R₇** is H or is selected from the group consisting of -(C₁-C₆)alkyl, -(C₁₋C₆)alkylene-NR₃R₄ and -(C₁₋C₆)alkylene-heterocycloalkyl, wherein said heterocycloalkyl is optionally substituted by one or more -(C₁-C₆)alkyl;
**R_{A}** is H or is selected from the group consisting of -(C₁₋C₆)alkyl and -(C₁-C₆)hydroxyalkyl;
**R_{B}** is selected from the group consisting of -(C₁-C₆)alkyl, -(C₁₋C₆)alkylene-C(O)O-(C₁₋C₆)alkyl, heterocycloalkyl and -(C₁-C₆)hydroxyalkyl;
**R₈** is selected from the group consisting of aryl and heteroaryl, wherein said aryl is optionally substituted by one or more groups selected from halogen atoms, -(C₁-C₆)alkyl and -(C₁-C₄)haloalkyl, and said heteroaryl is optionally substituted by one or more groups selected from -(C₁-C₆)alkyl, halogen atoms and -O-(C₁-C₆)alkyl;
and pharmaceutically acceptable salts thereof.

In a second aspect, the invention refers to a pharmaceutical composition comprising a compound of formula (I) and pharmaceutically acceptable salts thereof in admixture with one or more pharmaceutically acceptable carrier or excipient.

In a third aspect, the invention refers to a compound of formula (I) and pharmaceutically acceptable salts or to a pharmaceutical composition comprising a compound of formula (I) and pharmaceutically acceptable salts thereof for use as a medicament.

In a further aspect, the invention refers to a compound of formula (I) and pharmaceutically acceptable salts thereof or to a pharmaceutical composition comprising a compound of formula (I) and pharmaceutically acceptable salts thereof for use in preventing and/or treating a disease, disorder or condition mediated by ALK5 signaling pathway in a mammal.

In a further aspect, the invention refers to a compound of formula (I) and pharmaceutically acceptable salts thereof or to a pharmaceutical composition comprising a compound of formula (I) and pharmaceutically acceptable salts thereof for use in the prevention and/or treatment of fibrosis and/or diseases, disorders, or conditions that involve fibrosis.

In a further aspect, the invention refers to a compound of formula (I) and pharmaceutically acceptable salts thereof or to a pharmaceutical composition comprising a compound of formula (I) and pharmaceutically acceptable salts thereof for use in the prevention and/or treatment idiopathic pulmonary fibrosis (IPF).

### DETAILED DESCRIPTION OF THE INVENTION

### Definitions

Unless otherwise specified, the compound of formula (I) of the present invention is intended to include also tautomer or pharmaceutically acceptable salt or solvate thereof.

The term "pharmaceutically acceptable salts", as used herein, refers to derivatives of compounds of formula (I) wherein the parent compound is suitably modified by converting any of the free acid or basic group, if present, into the corresponding addition salt with any base or acid conventionally intended as being pharmaceutically acceptable.

Suitable examples of said salts may thus include mineral or organic acid addition salts of basic residues such as amino groups, as well as mineral or organic basic addition salts of acid residues such as carboxylic groups.

Cations of inorganic bases which can be suitably used to prepare salts comprise ions of alkali or alkaline earth metals such as potassium, sodium, calcium or magnesium.

Those obtained by reacting the main compound, functioning as a base, with an inorganic or organic acid to form a salt comprise, for example, salts of hydrochloric acid, hydrobromic acid, sulfuric acid, phosphoric acid, methane sulfonic acid, camphor sulfonic acid, acetic acid, oxalic acid, maleic acid, fumaric acid, succinic acid and citric acid.

The term "solvate" means a physical association of a compound of this invention with one or more solvent molecules, whether organic or inorganic. This physical association includes hydrogen bonding. In certain instances, the solvate will be capable of isolation, for example, when one or more solvent molecules are incorporated in the crystal lattice of the crystalline solid. The solvate may comprise either a stoichiometric or nonstoichiometric amount of the solvent molecules.

The term "tautomer" refers to each of two or more isomers of a compound that exist together in equilibrium and are readily interchanged by migration of an atom or group within the molecule.

The term "halogen" or "halogen atoms" or "halo" as used herein includes fluorine, chlorine, bromine, and iodine atom.

The term "(Cₓ-C_{y})alkyl" wherein x and y are integers, refers to a straight or branched chain alkyl group having from x to y carbon atoms. Thus, when x is 1 and y is 6, for example, the term includes methyl, ethyl, n-propyl, isopropyl, n-butyl, isobutyl, sec-butyl, t-butyl, n-pentyl and n-hexyl.

The term "(Cₓ-C_{y})alkylene" wherein x and y are integers, refers to a Cₓ-C_{y}alkyl radical having in total two unsatisfied valencies, such as a divalent methylene radical.

The expressions "(Cₓ-C_{y})haloalkyl" wherein x and y are integers, refer to the above defined "Cₓ-C_{y}alkyl" groups wherein one or more hydrogen atoms are replaced by one or more halogen atoms, which can be the same or different.

Examples of said "(Cₓ-C_{y})haloalkyl" groups may thus include halogenated, poly-halogenated and fully halogenated alkyl groups wherein all hydrogen atoms are replaced by halogen atoms, e.g. trifluoromethyl.

The term "(Cₓ-C_{y})cycloalkyl" wherein x and y are integers, refers to saturated cyclic hydrocarbon groups containing the indicated number of ring carbon atoms. Examples include cyclopropyl, cyclobutyl, cyclopentyl, cyclohexyl, cycloheptyl.

The term "aryl" refers to mono cyclic carbon ring systems which have 6 ring atoms wherein the ring is aromatic. Examples of suitable aryl monocyclic ring systems include, for instance, phenyl.

The term "heteroaryl" refers to a mono- or bi-cyclic aromatic group containing one or more heteroatoms selected from S, N and O, and includes groups having two such monocyclic rings, or one such monocyclic ring and one monocyclic aryl ring, which are fused through a common bond.

The term "(Cₓ-C_{y})heterocycloalkyl" wherein x and y are integers, refers to saturated or partially unsaturated monocyclic (Cₓ-C_{y})cycloalkyl groups in which at least one ring carbon atom is replaced by at least one heteroatom (e.g. N, S or O) or may bear an -oxo (=O) substituent group. Said heterocycloalkyl may be further optionally substituted on the available positions in the ring, namely on a carbon atom, or on an heteroatom available for substitution. Substitution on a carbon atom includes spiro disubstitution as well as substitution on two adjacent carbon atoms, in both cases thus form additional condensed 5 to 6 membered heterocyclic ring.

The term "(Cₓ-C_{y})hydroxyalkyl" wherein x and y are integers, refers to the above defined "(C₁-C₆)alkyl" groups wherein one or more hydrogen atoms are replaced by one or more hydroxy (OH) group.

Throughout the specification the use of an asterisk "*" in the definition of a structural formula, indicates the point of attachment for the radical group to the rest of the molecule.

A dash ("-") that is not between two letters or symbols is meant to represent the point of attachment for a substituent.

The carbonyl group is herein preferably represented as -C(O)- as an alternative to the other common representations such as -CO-, -(CO)- or -C(=O)-.

In general, the bracketed group is a lateral group, not included into the chain, and brackets are used, when deemed useful, to help disambiguating linear chemical formulas; e.g. the sulfonyl group -SO₂- might be also represented as -S(O)₂- to disambiguate e.g. with respect to the sulfinic group -S(O)O-.

The present invention relates to novel compounds differing from the structures disclosed in the art at least for a common new core scaffold. In fact the invention relates to compounds that are [2H,3H,4H-pyrido[3,2-b][1,4]oxazin-8-yl]amino derivatives, wherein the amino group is linked to a pyridine or a pyridine fused to a 5-membered heterocyclic ring, which are inhibitors of receptor ALK5, that have therapeutically desirable characteristics, particularly promising for some fibrosis, including idiopathic pulmonary fibrosis (IPF).

The compounds of the invention are active as inhibitors of ALK5 receptor, they are potent and show improved properties such as a good inhalatory profile, a low metabolic stability, a low systemic exposure, improved safety and tolerability, and a good selectivity across the kinome.

In this respect, the state of the art does not describe or suggest pyrido oxazine amino derivatives of general formula (I) of the present invention having inhibitory activity on receptor ALK5 which represents a solution to the aforementioned need.

In more details, the present invention refers to a series of compounds represented by the general formula (I) as herein below described in details, which are endowed with an inhhibitory activity on receptor ALK5.

Advantageously, the inhibitory action on receptor can be effective in the treatment of those diseases where these receptors play a relevant role in the pathogenesis such as fibrosis and disease, disorder and condition from fibrosis.

Differently from similar compounds of the prior art, the compounds of formula (I) of the present invention are able to act as inhibitors of ALK5 receptor, particularly appreciated by the skilled person when looking at a suitable and efficacious compounds useful for the treatment of fibrosis, in particular idiopathic pulmonary fibrosis.

As indicated in the experimental part, in particular in Table 10, the compounds of formula (I) of the present invention show a notable potency with respect to their inhibitory activity on receptor ALK5, below about 10 nM, confirming that they are able to inhibit ALK5 receptor involved in fibrosis and diseases that result from fibrosis.

As indicated in the experimental part, comparative examples, in particular in Table 11, it is shown that, conversely to the compounds C1, C2 and C3 characterized by a pyrido oxazine ring directly fused to a pyridine ring, in the compounds of formula (I), characterized by an amino group as linker between the pyrido oxazine ring and the pyridine or pyridine condensed to a 5-membered heterocyclic ring, the presence of the amino group as linker between these two groups remarkably and unexpectedly determines a relevant increase in the inhibitory activity on the ALK5 receptor.

Advantageously, the compounds of the present invention are endowed by a very high potency, they could be administered in human at a lower dosage respect to the compounds of the prior art, thus reducing the adverse events that typically occur administering higher dosages of drug.

In addition to being notably potent with respect to their inhibitory activity on receptor ALK5, the compounds of the present invention are also characterized by a good inhalatory profile, that permits to act effectively on the lung compartment and have, at the same time, a low metabolic stability, that allows to minimize the drawbacks associated with the systemic exposure, such as safety and tolerability issues.

As detailed in the experimental part, Table 12, the compounds of the invention show an higher lung exposure compared to the plasma (i.e. systemic) one.

Therefore, the compounds of the present invention are particularly appreciated by the skilled person when looking at a suitable and efficacious compounds useful for the treatment of fibrosis, in particular idiopathic pulmonary fibrosis, administered by the inhalation route and characterized by a good inhalatory profile, that corresponds to a good activity on the lung, a good lung retention and to a low metabolic stability, that minimizes the systemic exposure and correlated safety issues.

Thus, in one aspect the present invention relates to a compound of general formula (I) 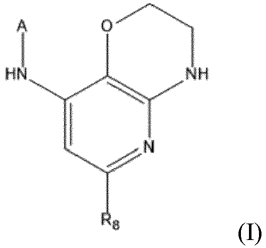 wherein
**A** is selected from the group consisting of **A₁** and **A₂** 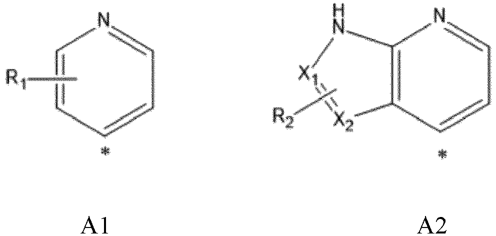
**X₁** and **X₂** are independently C or N;
**R₁** is H or is selected from the group consisting of -NR₃R₄, -C(O)NR₃R₅,-NR₃C(O)R₆ and -C(O)OR₇;
**R₂** is H or is selected from the group consisting of -C(O)OR₃ and -C(O)NR₃R₆; or **R₂** is absent when X₁ or X₂ is N;
**R₃** is H or -(C₁-C₆)alkyl;
**R₄** is H or -(C₁-C₆)alkyl;
**R₅** is H or is selected from the group consisting of -(C₁-C₆)alkyl, -(C₁₋C₆)alkylene-NR_{A}R_{B}, cycloalkyl, heterocycloalkyl optionally substituted by one or more -(C₁₋ C₆)alkyl, and -(C₁₋C₆)alkylene-heterocycloalkyl, wherein said heterocycloalkyl is optionally substituted by one or more groups selected from -(C₁-C₆)alkyl, -C(O)-(C₁₋C₆)alkyl and -SO₂-(C₁₋C₆)alkyl;
**R₆** is selected from the group consisting of -(C₁₋C₆)alkylene-NR₃R₄ and -(C₁₋ C₆)alkylene-heterocycloalkyl, wherein said heterocycloalkyl is optionally substituted by one or more -(C₁-C₆)alkyl;
**R₇** is H or is selected from the group consisting of -(C₁-C₆)alkyl, -(C₁₋C₆)alkylene-NR₃R₄ and -(C₁₋C₆)alkylene-heterocycloalkyl, wherein said heterocycloalkyl is optionally substituted by one or more -(C₁-C₆)alkyl;
**R_{A}** is H or is selected from the group consisting of -(C₁₋C₆)alkyl and -(C₁-C₆)hydroxyalkyl;
**R_{B}** is selected from the group consisting of -(C₁-C₆)alkyl, -(C₁₋C₆)alkylene-C(O)O-(C₁₋C₆)alkyl, heterocycloalkyl and -(C₁-C₆)hydroxyalkyl;
**R₈** is selected from the group consisting of aryl and heteroaryl, wherein said aryl is optionally substituted by one or more groups selected from halogen atoms, -(C₁-C₆)alkyl and -(C₁-C₄)haloalkyl, and said heteroaryl is optionally substituted by one or more groups selected from -(C₁-C₆)alkyl, halogen atoms and -O-(C₁-C₆)alkyl;
and pharmaceutically acceptable salts thereof.

In a particularly preferred embodiment the present invention refers to a compound of formula (I), wherein **A** is group **A1** 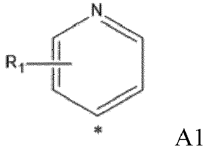 represented by the formula (Ia) 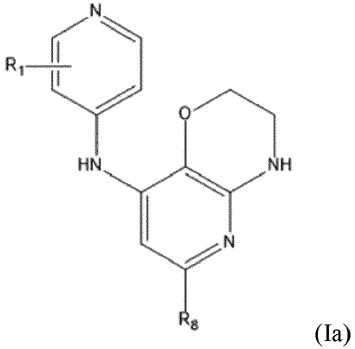
**R₁** is H or is selected from the group consisting of -NR₃R₄, -C(O)NR₃R₅,-NR₃C(O)R₆ and -C(O)OR₇;
**R₃** is H or -(C₁-C₆)alkyl;
**R₄** is H or -(C₁-C₆)alkyl;
**R₅** is H or is selected from the group consisting of -(C₁-C₆)alkyl, -(C₁₋C₆)alkylene-NR_{A}R_{B}, cycloalkyl, heterocycloalkyl optionally substituted by a -(C₁-C₆)alkyl, and -(C₁₋C₆)alkylene-heterocycloalkyl, wherein said heterocycloalkyl is optionally substituted by a group selected from -(C₁-C₆)alkyl, -C(O)-(C₁₋C₆)alkyl and -SO₂-(C₁₋C₆)alkyl;
**R₆** is selected from the group consisting of -(C₁₋C₆)alkylene-NR₃R₄ and -(C₁₋ C₆)alkylene-heterocycloalkyl, wherein said heterocycloalkyl is optionally substituted by a -(C₁-C₆)alkyl;
**R₇** is H or is selected from the group consisting of -(C₁-C₆)alkyl, -(C₁₋C₆)alkylene-NR₃R₄ and -(C₁₋C₆)alkylene-heterocycloalkyl, wherein said heterocycloalkyl is optionally substituted by a -(C₁-C₆)alkyl;
**R_{A}** is H or is selected from the group consisting of -(C₁₋C₆)alkyl and -(C₁-C₆)hydroxyalkyl;
**R_{B}** is selected from the group consisting of -(C₁-C₆)alkyl, -(C₁₋C₆)alkylene-C(O)O-(C₁₋C₆)alkyl, heterocycloalkyl and -(C₁-C₆)hydroxyalkyl;
**R₈** is selected from the group consisting of aryl and heteroaryl, wherein said aryl is optionally substituted by one or more groups selected from halogen atoms, -(C₁-C₆)alkyl and -(C₁-C₄)haloalkyl, and said heteroaryl is optionally substituted by one or more groups selected from -(C₁-C₆)alkyl, halogen atoms and -O-(C₁-C₆)alkyl;
and pharmaceutically acceptable salts thereof.

According to a preferred embodiment, the invention refers to at least one of the compounds of Formula (Ia) listed in the Table 1 below and pharmaceutically acceptable salts thereof.

**Table 1: List of preferred compounds of Formula (Ia)**

| **Example No.** | **Structure** | **Chemical Name** |
|---|---|---|
| 1 | 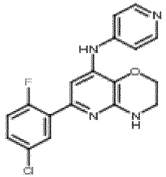 | N-[6-(5-chloro-2-fluorophenyl)-2H,3H,4H-pyrido[3,2-b][1,4]oxazin-8-yl]pyridin-4-amine |
| 2 | 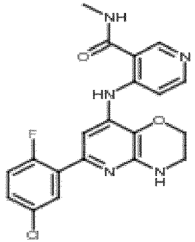 | 4-{[6-(5 -chloro-2-fluorophenyl)-2H,3H,4H-pyrido[3,2-b][1,4]oxazin-8-yl]amino}-N-methylpyridine-3-carboxamide |
| 3 | 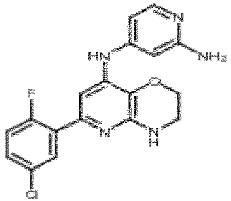 | N4-[6-(5-chloro-2-fluorophenyl)-2H,3H,4H-pyrido[3,2-b][1,4]oxazin-8-yl]pyridine-2,4-diamine |
| 4 | 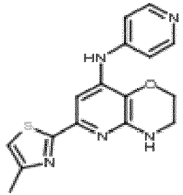 | N-[6-(4-methyl-1,3-thiazol-2-yl)-2H,3H,4H-pyrido[3,2-b][1,4]oxazin-8-yl]pyridin-4-amine |
| 5 | 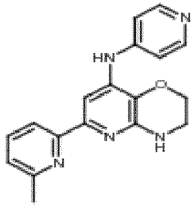 | N-[6-(6-methylpyridin-2-yl)-2H,3H,4H-pyrido[3,2-b][1,4]oxazin-8-yl]pyridin-4-amine |
| 6 | 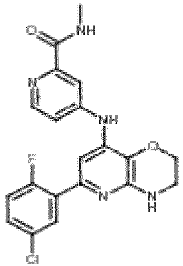 | 4-{[6-(5-chloro-2-fluorophenyl)-2H,3H,4H-pyrido[3,2-b][1,4]oxazin-8-yl]amino}-N-methylpyridine-2-carboxamide |
| 7 | 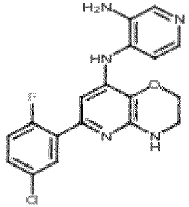 | N4-[6-(5 -chloro-2-fluorophenyl)-2H,3H,4H-pyrido[3,2-b][1,4]oxazin-8-yl]pyridine-3,4-diamine |
| 8 | 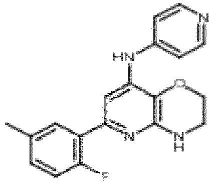 | N-[6-(2-fluoro-5-methylphenyl)-2H,3H,4H-pyrido[3,2-b][1,4]oxazin-8-yl]pyridin-4-amine |
| 9 | 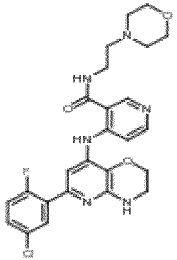 | 4-{[6-(5-chloro-2-fluorophenyl)-2H,3H,4H-pyrido[3,2-b][1,4]oxazin-8-yl]amino}-N-[2-(morpholin-4-yl)ethyl]pyridine-3-carboxamide |
| 10 | 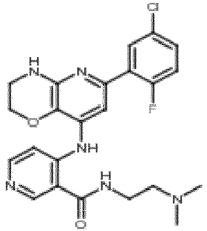 | 4-{[6-(5-chloro-2-fluorophenyl)-2H,3H,4H-pyrido[3,2-b][1,4]oxazin-8-yl]amino}-N-[2-(dimethylamino)ethyl]pyridine-3-carboxamide |
| 11 | 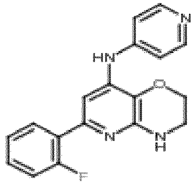 | N-[6-(2-fluorophenyl)-2H,3H,4H-pyrido[3,2-b][1,4]oxazin-8-yl]pyridin-4-amine |
| 12 | 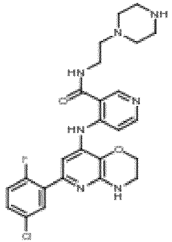 | 4-{[6-(5-chloro-2-fluorophenyl)-2H,3H,4H-pyrido[3,2-b][1,4]oxazin-8-yl]amino}-N-[2-(piperazin-1-yl)ethyl]pyridine-3-carboxamide |
| 13 | 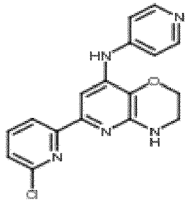 | N-[6-(6-chloropyridin-2-yl)-2H,3H,4H-pyrido[3,2-b][1,4]oxazin-8-yl]pyridin-4-amine |
| 14 | 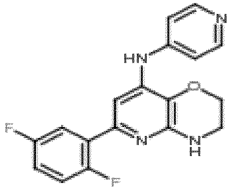 | N-[6-(2,5-difluorophenyl)-2H,3H,4H-pyrido[3,2-b][1,4]oxazin-8-yl]pyridin-4-amine |
| 15 | 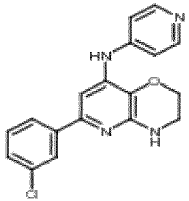 | N-[6-(3-chlorophenyl)-2H,3H,4H-pyrido[3,2-b][1,4]oxazin-8-yl]pyridin-4-amine |
| 16 | 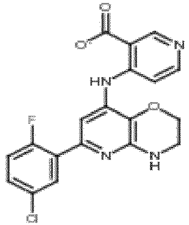 | 4-{[6-(5-chloro-2-fluorophenyl)-2H,3H,4H-pyrido[3,2-b][1,4]oxazin-8-yl]amino}pyridine-3-carboxylate |
| 17 | 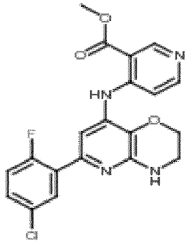 | methyl 4-{[6-(5-chloro-2-fluorophenyl)-2H,3H,4H-pyrido[3,2-b][1,4]oxazin-8-yl]amino }pyridine-3-carboxylate |
| 18 | 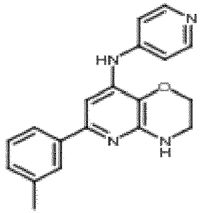 | N-[6-(3-methylphenyl)-2H,3H,4H-pyrido[3,2-b][1,4]oxazin-8-yl]pyridin-4-amine |
| 19 | 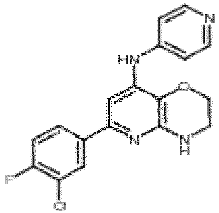 | N-[6-(3-chloro-4-fluorophenyl)-2H,3H,4H-pyrido[3,2-b][1,4]oxazin-8-yl]pyridin-4-amine |
| 20 | 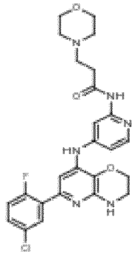 | N-(4-{[6-(5-chloro-2-fluorophenyl)-2H,3H,4H-pyrido[3,2-b][1,4]oxazin-8-yl]amino }pyridin-2-yl)-3-(morpholin-4-yl)propanamide |
| 21 | 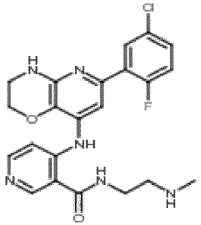 | 4-{[6-(5-chloro-2-fluorophenyl)-2H,3H,4H-pyrido[3,2-b][1,4]oxazin-8-yl]amino}-N-[2-(methylamino)ethyl]pyridine-3-carboxamide |
| 22 | 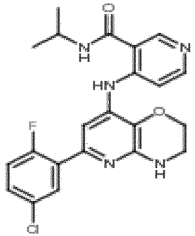 | 4-{[6-(5-chloro-2-fluorophenyl)-2H,3H,4H-pyrido[3,2-b][1,4]oxazin-8-yl]amino}-N-(propan-2-yl)pyridine-3- carboxamide |
| 23 | 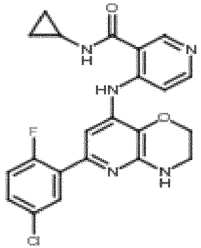 | 4-{[6-(5-chloro-2-fluorophenyl)-2H,3H,4H-pyrido[3,2-b][1,4]oxazin-8-yl]amino}-N-cyclopropylpyridine-3-carboxamide |
| 24 | 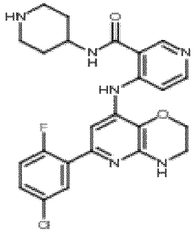 | 4-{[6-(5-chloro-2-fluorophenyl)-2H,3H,4H-pyrido[3,2-b][1,4]oxazin-8-yl]amino}-N-(piperidin-4-yl)pyridine-3-carboxamide |
| 26 | 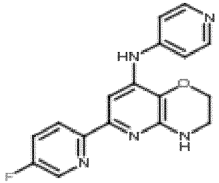 | N-[6-(5-fluoropyridin-2-yl)-2H,3H,4H-pyrido[3,2-b][1,4]oxazin-8-yl]pyridin-4-amine |
| 27 | 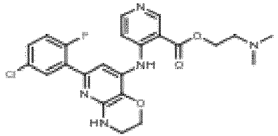 | 2-(dimethylamino)ethyl 4-{[6-(5-chloro-2-fluorophenyl)-2H,3H,4H-pyrido[3,2-b][1,4]oxazin-8-yl]amino }pyridine-3-carboxylate |
| 28 | 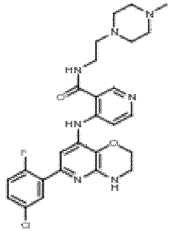 | 4-{[6-(5-chloro-2-fluorophenyl)-2H,3H,4H-pyrido[3,2-b][1,4]oxazin-8-yl]amino}-N-[2-(4-methylpiperazin-1-yl)ethyl]pyridine-3-carboxamide |
| 29 | 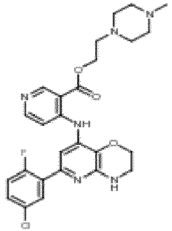 | 2-(4-methylpiperazin-1-yl)ethyl 4-{[6-(5-chloro-2-fluorophenyl)-2H,3H,4H-pyrido[3,2-b][1,4]oxazin-8-yl]amino}pyridine-3-carboxylate |
| 30 | 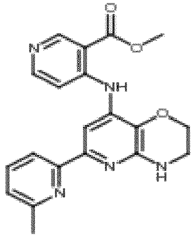 | methyl 4-{[6-(6-methylpyridin-2-yl)-2H,3H,4H-pyrido[3,2-b][1,4]oxazin-8-yl]amino}pyridine-3-carboxylate |
| 32 | 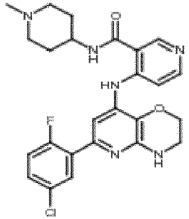 | 4-{[6-(5-chloro-2-fluorophenyl)-2H,3H,4H-pyrido[3,2-b][1,4]oxazin-8-yl]amino}-N-(1-methylpiperidin-4-yl)pyridine-3-carboxamide |
| 33 | 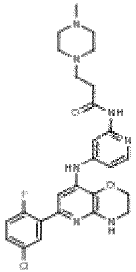 | N-(4-{[6-(5-chloro-2-fluorophenyl)-2H,3H,4H-pyrido[3,2-b][1,4]oxazin-8-yl]amino}pyridin-2-yl)-3-(4-methylpiperazin-1-yl)propanamide |
| 34 | 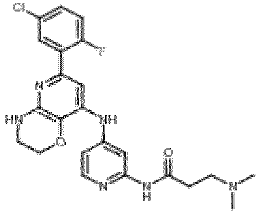 | N-(4-{[6-(5-chloro-2-fluorophenyl)-2H,3H,4H-pyrido[3,2-b][1,4]oxazin-8-yl]amino }pyridin-2-yl)-3-(dimethylamino)propanamide |
| 37 | 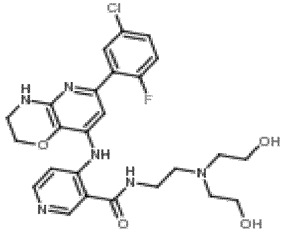 | N-{2-[bis(2-hydroxyethyl)amino]ethyl}-4-{[6-(5-chloro-2-fluorophenyl)-2H,3H,4H-pyrido[3,2-b][1,4]oxazin-8-yl]amino}pyridine-3-carboxamide |
| 39 | 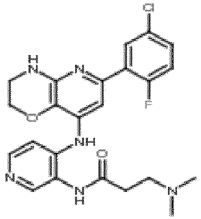 | 4-{[6-(5-chloro-2-fluorophenyl)-2H,3H,4H-pyrido[3,2-b][1,4]oxazin-8-yl]amino}-N-[2-(dimethylamino)ethyl]pyridine-3-carboxamide |
| 42 | 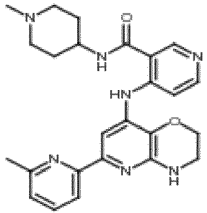 | N-(1-methylpiperidin-4-yl)-4-{[6-(6-methylpyridin-2-yl)-2H,3H,4H-pyrido[3,2-b][1,4]oxazin-8-yl]amino }pyridine-3-carboxamide |
| 43 | 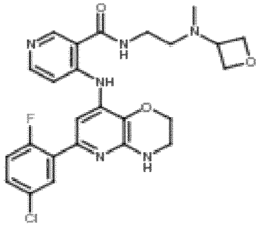 | 4-{[6-(5-chloro-2-fluorophenyl)-2H,3H,4H-pyrido[3,2-b][1,4]oxazin-8-yl]amino}-N-{2-[methyl(oxetan-3-yl)amino] ethyl }pyridine-3-carboxamide |
| 44 | 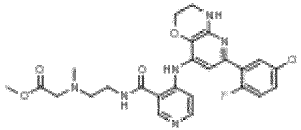 | methyl 2-({2-[(4-{[6-(5-chloro-2-fluorophenyl)-2H,3H,4H-pyrido[3,2-b][1,4]oxazin-8-yl]amino}pyridin-3-yl)formamido]ethyl}(methyl)amino )acetate |
| 46 | 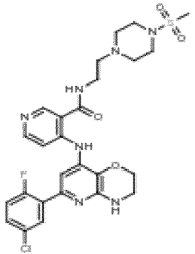 | 4- {[6-(5-chloro-2-fluorophenyl)-2H,3H,4H-pyrido[3,2-b][1,4]oxazin-8-yl]amino}-N-[2-(4-methanesulfonylpiperazin-1- yl)ethyl]pyridine-3-carboxamide |
| 47 | 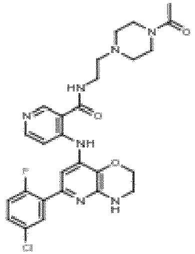 | N-[2-(4-acetylpiperazin-1-yl)ethyl]-4-{[6-(5-chloro-2-fluorophenyl)-2H,3H,4H-pyrido[3,2-b][1,4]oxazin-8-yl]amino}pyridine-3-carboxamide |

According to a even more preferred embodiment, the present invention refers to a compound of formula (Ia), wherein
**R₁** is H or is selected from the group consisting of -C(O)NHR₅, -NHC(O)R₆ and - C(O)OR₇;
**R₅** is selected from the group consisting of methyl, 2-(methylamino)ethyl, cyclopropyl, 2-(4-methylpiperazin-1-yl)ethyl, 2-(dimethylamino)ethyl, 2-(piperazin-1-yl)ethyl, (piperidin-4-yl) and 2-[bis(2-hydroxyethyl)amino]ethyl;
**R₆** is selected from the group consisting of 4-ethyl morpholine, 1-ethyl-4-methylpiperazine and N,N-dimethylethanamine.
**R₇** is H or is selected from the group consisting of methyl and 2-(4-methylpiperazin-1-yl)ethyl.

According to a preferred embodiment, the invention refers to at least one of the compounds of Formula (Ia) listed in the Table 2 below and pharmaceutical acceptable salts thereof. These compounds are particularly active on receptor ALK5, as shown in Table 10.

**Table 2: List of preferred compounds of Formula (Ia)**

| **Example No.** | **Structure** | **Chemical Name** |
|---|---|---|
| 1 | 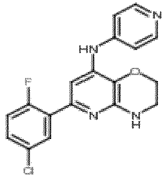 | N-[6-(5-chloro-2-fluorophenyl)-2H,3H,4H-pyrido[3,2-b][1,4]oxazin-8-yl]pyridin-4-amine |
| 2 | 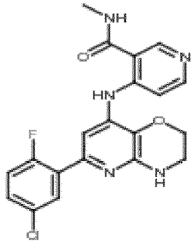 | 4- {[6-(5 -chloro-2-fluorophenyl)-2H,3H,4H-pyrido[3,2-b][1,4]oxazin-8-yl]amino}-N-methylpyridine-3-carboxamide |
| 8 | 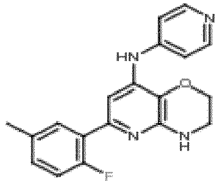 | N-[6-(2-fluoro-5-methylphenyl)-2H,3H,4H-pyrido[3,2-b][1,4]oxazin-8-yl]pyridin-4-amine |
| 10 | 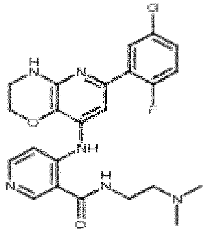 | 4- {[6-(5-chloro-2-fluorophenyl)-2H,3H,4H-pyrido[3,2-b][1,4]oxazin-8-yl]amino}-N-[2-(dimethylamino)ethyl]pyridine-3-carboxamide |
| 12 | 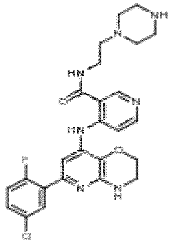 | 4-{[6-(5-chloro-2-fluorophenyl)-2H,3H,4H-pyrido[3,2-b][1,4]oxazin-8-yl]amino}-N-[2-(piperazin-1-yl)ethyl]pyridine-3-carboxamide |
| 16 | 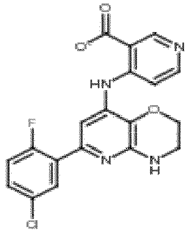 | 4-{[6-(5-chloro-2-fluorophenyl)-2H,3H,4H-pyrido[3,2-b][1,4]oxazin-8-yl]amino}pyridine-3-carboxylate |
| 17 | 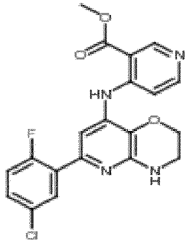 | methyl 4-{[6-(5-chloro-2-fluorophenyl)-2H,3H,4H-pyrido[3,2-b][1,4]oxazin-8-yl]amino}pyridine-3-carboxylate |
| 20 | 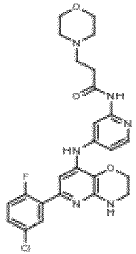 | N-(4-{[6-(5-chloro-2-fluorophenyl)-2H,3H,4H-pyrido[3,2-b][1,4]oxazin-8-yl]amino}pyridin-2-yl)-3-(morpholin-4-yl)propanamide |
| 21 | 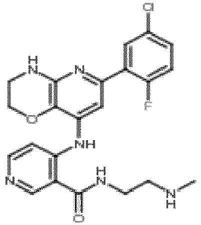 | 4-{[6-(5-chloro-2-fluorophenyl)-2H,3H,4H-pyrido[3,2-b][1,4]oxazin-8-yl]amino}-N-[2-(methylamino)ethyl]pyridine-3-carboxamide |
| 23 | 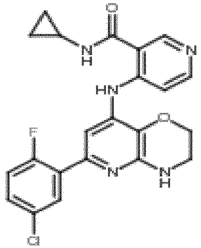 | 4-{[6-(5-chloro-2-fluorophenyl)-2H,3H,4H-pyrido[3,2-b][1,4]oxazin-8-yl]amino}-N-cyclopropylpyridine-3-carboxamide |
| 24 | 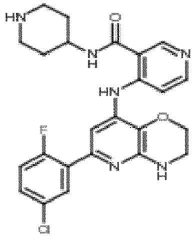 | 4-{[6-(5-chloro-2-fluorophenyl)-2H,3H,4H-pyrido[3,2-b][1,4]oxazin-8-yl]amino}-N-(piperidin-4-yl)pyridine-3 - carboxamide |
| 29 | 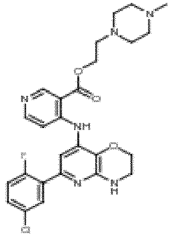 | 2-(4-methylpiperazin-1-yl)ethyl 4-{[6-(5-chloro-2-fluorophenyl)-2H,3H,4H-pyrido[3,2-b][1,4]oxazin-8-yl]amino}pyridine-3-carboxylate |
| 30 | 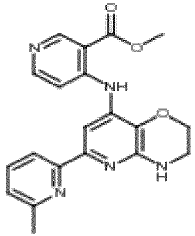 | methyl 4-[[6-(6-methylpyridin-2-yl)-2H,3H,4H-pyrido[3,2-b][1,4]oxazin-8-yl]amino}pyridine-3-carboxylate |
| 32 | 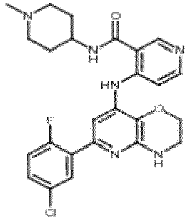 | 4-{[6-(5-chloro-2-fluorophenyl)-2H,3H,4H-pyrido[3,2-b][1,4]oxazin-8-yl]amino}-N-(1-methylpiperidin-4-yl)pyridine-3-carboxamide |
| 33 | 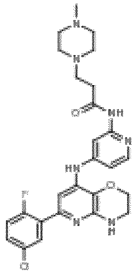 | N-(4-{[6-(5-chloro-2-fluorophenyl)-2H,3H,4H-pyrido[3,2-b][1,4]oxazin-8-yl]amino}pyridin-2-yl)-3-(4-methylpiperazin-1-yl)propanamide |
| 34 | 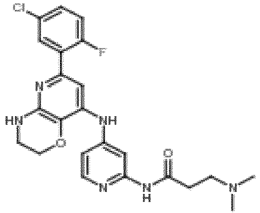 | N-(4-{[6-(5-chloro-2-fluorophenyl)-2H,3H,4H-pyrido[3,2-b][1,4]oxazin-8-yl]amino }pyridin-2-yl)-3-(dimethylamino)propanamide |
| 37 | 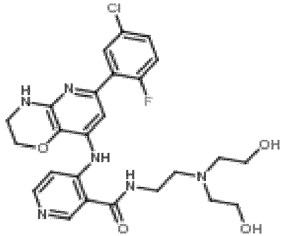 | N-{2-[bis(2-hydroxyethyl)amino]ethyl}-4-{[6-(5-chloro-2-fluorophenyl)-2H,3H,4H-pyrido[3,2-b][1,4]oxazin-8-yl]amino}pyridine-3-carboxamide |

In a more preferred embodiment, the present invention refers to compound of formula (Ia), wherein **A** is **A1a** 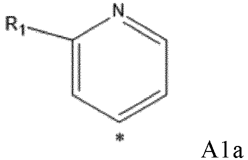 represented by the formula (Iaa) 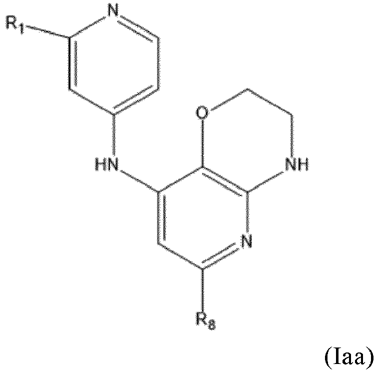
**R₁** is H;
**R₈** is as defined above.

According to a preferred embodiment, the invention refers to at least one of the compounds of Formula (Iaa) listed in the Table 3 below and pharmaceutically acceptable salts thereof.

**Table 3: List of preferred compounds of Formula (Iaa)**

| **Example No.** | **Structure** | **Chemical Name** |
|---|---|---|
| 1 | 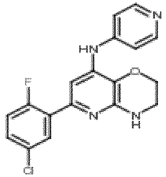 | N-[6-(5-chloro-2-fluorophenyl)-2H,3H,4H-pyrido[3,2-b][1,4]oxazin-8-yl]pyridin-4-amine |
| 4 | 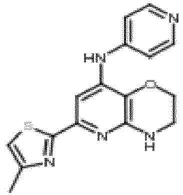 | N-[6-(4-methyl-1,3-thiazol-2-yl)-2H,3H,4H-pyrido[3,2-b][1,4]oxazin-8-yl]pyridin-4-amine |
| 5 | 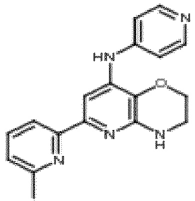 | N-[6-(6-methylpyridin-2-yl)-2H,3H,4H-pyrido[3,2-b][1,4]oxazin-8-yl]pyridin-4-amine |
| 8 | 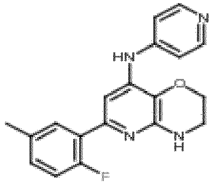 | N-[6-(2-fluoro-5-methylphenyl)-2H,3H,4H-pyrido[3,2-b][1,4]oxazin-8-yl]pyridin-4-amine |
| 11 | 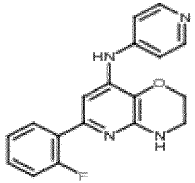 | N-[6-(2-fluorophenyl)-2H,3H,4H-pyrido[3,2-b][1,4]oxazin-8-yl]pyridin-4-amine |
| 13 | 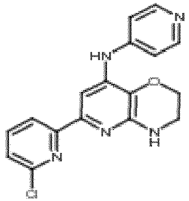 | N-[6-(6-chloropyridin-2-yl)-2H,3H,4H-pyrido[3,2-b][1,4]oxazin-8-yl]pyridin-4-amine |
| 14 | 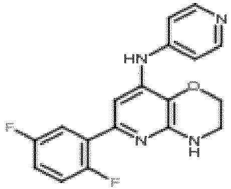 | N-[6-(2,5-difluorophenyl)-2H,3H,4H-pyrido[3,2-b][1,4]oxazin-8-yl]pyridin-4-amine |
| 15 | 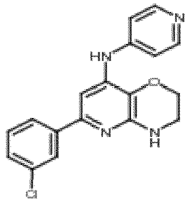 | N-[6-(3-chlorophenyl)-2H,3H,4H-pyrido[3,2-b][1,4]oxazin-8-yl]pyridin-4-amine |
| 18 | 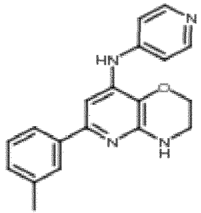 | N-[6-(3-methylphenyl)-2H,3H,4H-pyrido[3,2-b][1,4]oxazin-8-yl]pyridin-4-amine |
| 19 | 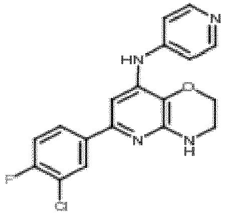 | N-[6-(3-chloro-4-fluorophenyl)-2H,3H,4H-pyrido[3,2-b][1,4]oxazin-8-yl]pyridin-4-amine |
| 26 | 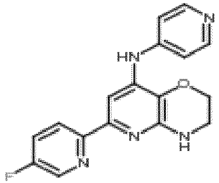 | N-[6-(5-fluoropyridin-2-yl)-2H,3H,4H-pyrido[3,2-b][1,4]oxazin-8-yl]pyridin-4-amine |

In a more preferred embodiment, the present invention refers to compound of Formula (Ia) represented by the formula (Iaa), wherein
**R₁** is H;
**R₈** is phenyl substituted by fluorine and chlorine.

According to a preferred embodiment, the invention refers to at least one of the compounds of Formula (Iaa) listed in the Table 4 below and pharmaceutical acceptable salts thereof. These compounds are particularly active on receptor ALK5, as shown in Table 10.

**Table 4: List of preferred compounds of Formula (Iaa)**

| **Example No.** | **Structure** | **Chemical Name** |
|---|---|---|
| 1 | 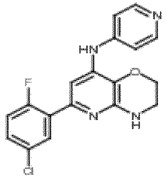 | N-[6-(5-chloro-2-fluorophenyl)-2H,3H,4H-pyrido[3,2-b][1,4]oxazin-8-yl]pyridin-4-amine |
| 8 | 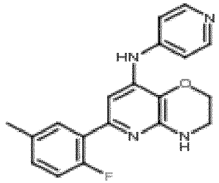 | N-[6-(2-fluoro-5-methylphenyl)-2H,3H,4H-pyrido[3,2-b][1,4]oxazin-8-yl]pyridin-4-amine |

In another embodiment, the present invention refers to a compound of formula (Iaa), wherein
**R₁** is selected from the group consisting of -NR₃R₄, -C(O)NR₃R₅ and -NR₃C(O)R₆;
**R₃** is H or -(C₁-C₆)alkyl;
**R₄** is H or -(C₁-C₆)alkyl;
**R₅** is H or -(C₁-C₆)alkyl;
**R₆** is selected from the group consisting of -(C₁₋C₆)alkylene-NR₃R₄ and -(C₁₋ C₆)alkylene-heterocycloalkyl, wherein said heterocycloalkyl is optionally substituted by a -(C₁-C₆)alkyl;
**R₈** is selected from the group consisting of aryl and heteroaryl, wherein said aryl is optionally substituted by one or more groups selected from halogen atoms, -(C₁-C₆)alkyl and -(C₁-C₄)haloalkyl, and said heteroaryl is optionally substituted by one or more groups selected from -(C₁-C₆)alkyl, halogen atoms and -O-(C₁-C₆)alkyl.

According to a preferred embodiment, the invention refers to at least one of the compounds of Formula (Iaa) listed in the Table 5 below and pharmaceutical acceptable salts thereof.

**Table 5: List of preferred compounds of Formula (Iaa)**

| **Example No.** | **Structure** | **Chemical Name** |
|---|---|---|
| 3 | 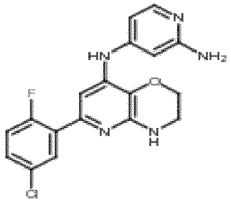 | N4-[6-(5-chloro-2-fluorophenyl)-2H,3H,4H-pyrido[3,2-b][1,4]oxazin-8-yl]pyridine-2,4-diamine |
| 6 | 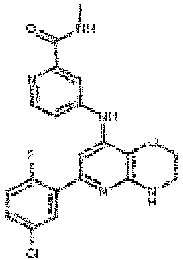 | 4-{[6-(5-chloro-2-fluorophenyl)-2H,3H,4H-pyrido[3,2-b][1,4]oxazin-8-yl]amino}-N-methylpyridine-2-carboxamide |
| 20 | 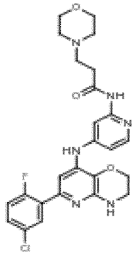 | N-(4-{[6-(5-chloro-2-fluorophenyl)-2H,3H,4H-pyrido[3,2-b][1,4]oxazin-8-yl]amino}pyridin-2-yl)-3-(morpholin-4-yl)propanamide |
| 33 | 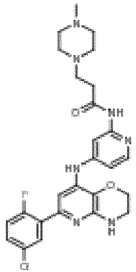 | N-(4-{[6-(5-chloro-2-fluorophenyl)-2H,3H,4H-pyrido[3,2-b][1,4]oxazin-8-yl]amino}pyridin-2-yl)-3-(4-methylpiperazin-1-yl)propanamide |
| 34 | 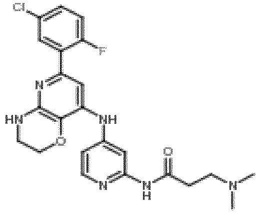 | N-(4-{[6-(5-chloro-2-fluorophenyl)-2H,3H,4H-pyrido[3,2-b][1,4]oxazin-8-yl]amino }pyridin-2-yl)-3-(dimethylamino)propanamide |

In a further more preferred embodiment, the present invention refers to a compound of formula (Iaa), wherein
**R₁** is -NHC(O)R₆;
**R₆** is selected from the group consisting of 4-ethylmorpholine, 1-ethyl-4-methylpiperazine and N,N-dimethylethanamine.

According to preferred embodiment, the invention refers to at least one of the compounds listed of formula (Iaa) in the Table 6 below and pharmaceutical acceptable salts thereof. These compounds are particularly active on receptor ALK5, as shown in Table 10.

**Table 6: List of preferred compounds of Formula (Iaa)**

| **Example No.** | **Structure** | **Chemical Name** |
|---|---|---|
| 20 | 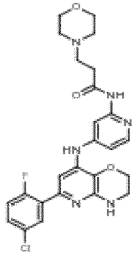 | N-(4-{[6-(5-chloro-2-fluorophenyl)-2H,3H,4H-pyrido[3,2-b][1,4]oxazin-8-yl]amino}pyridin-2-yl)-3-(morpholin-4-yl)propanamide |
| 33 | 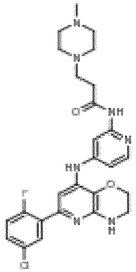 | N-(4-{[6-(5-chloro-2-fluorophenyl)-2H,3H,4H-pyrido[3,2-b][1,4]oxazin-8-yl]amino}pyridin-2-yl)-3-(4-methylpiperazin-1-yl)propanamide |
| 34 | 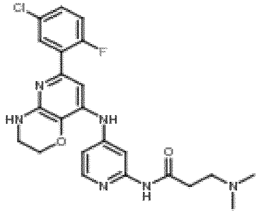 | N-(4-{[6-(5-chloro-2-fluorophenyl)-2H,3H,4H-pyrido[3,2-b][1,4]oxazin-8-yl]amino}pyridin-2-yl)-3-(dimethylamino)propanamide |

In a further preferred embodiment, the present invention refers to a compound of Formula (Ia), wherein **A** is **A1b** 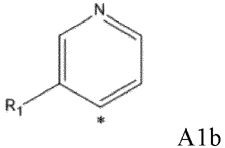 represented by the formula (Iab) 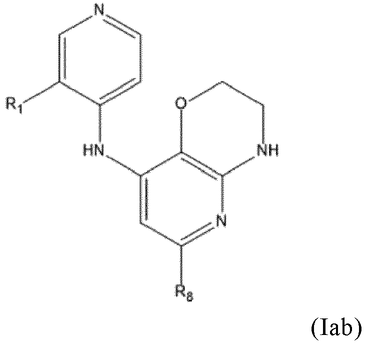
**R₁** is selected from the group consisting of -NR₃R₄, -C(O)NR₃R₅,-NR₃C(O)R₆ and -C(O)OR₇;
**R₃** is H or -(C₁-C₆)alkyl;
**R₄** is H or -(C₁-C₆)alkyl;
**R₅** is H or is selected from the group consisting of -(C₁-C₆)alkyl, -(C₁₋C₆)alkylene-NR_{A}R_{B}, cycloalkyl, heterocycloalkyl optionally substituted by one or more -(C₁₋ C₆)alkyl, and -(C₁₋C₆)alkylene-heterocycloalkyl, wherein said heterocycloalkyl is optionally substituted by one or more groups selected from -(C₁-C₆)alkyl, -C(O)-(C₁₋C₆)alkyl and -SO₂-(C₁₋C₆)alkyl;
**R₆** is selected from the group consisting of -(C₁₋C₆)alkylene-NR₃R₄ and -(C₁₋ C₆)alkylene-heterocycloalkyl, wherein said heterocycloalkyl is optionally substituted by one or more -(C₁-C₆)alkyl;
**R₇** is H or is selected from the group consisting of -(C₁-C₆)alkyl, -(C₁₋C₆)alkylene-NR₃R₄ and -(C₁₋C₆)alkylene-heterocycloalkyl, wherein said heterocycloalkyl is optionally substituted by one or more -(C₁-C₆)alkyl;
**R_{A}** is H or is selected from the group consisting of -(C₁₋C₆)alkyl and -(C₁-C₆)hydroxyalkyl;
**R_{B}** is selected from the group consisting of -(C₁-C₆)alkyl, -(C₁₋C₆)alkylene-C(O)O-(C₁₋C₆)alkyl, heterocycloalkyl and -(C₁-C₆)hydroxyalkyl;
**R₈** is selected from the group consisting of aryl and heteroaryl, wherein said aryl is optionally substituted by one or more groups selected from halogen atoms, -(C₁-C₆)alkyl and -(C₁-C₄)haloalkyl, and said heteroaryl is optionally substituted by one or more groups selected from -(C₁-C₆)alkyl, halogen atoms and -O-(C₁-C₆)alkyl.

According to preferred embodiment, the invention refers to at least one of the compounds of Formula (Iab) listed in the Table 7 below and pharmaceutical acceptable salts thereof.

**Table 7: List of preferred compounds of Formula (Iab)**

| **Example No.** | **Structure** | **Chemical Name** |
|---|---|---|
| 2 | 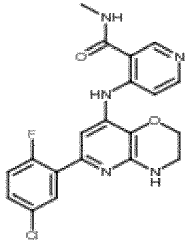 | 4-{[6-(5-chloro-2-fluorophenyl)-2H,3H,4H-pyrido[3,2-b][1,4]oxazin-8-yl]amino}-N-methylpyridine-3-carboxamide |
| 7 | 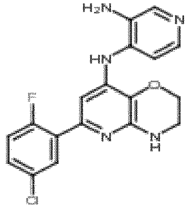 | N4-[6-(5-chloro-2-fluorophenyl)-2H,3H,4H-pyrido[3,2-b][1,4]oxazin-8-yl]pyridine-3,4-diamine |
| 9 | 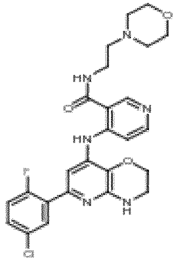 | 4-{[6-(5-chloro-2-fluorophenyl)-2H,3H,4H-pyrido[3,2-b][1,4]oxazin-8-yl]amino}-N-[2-(morpholin-4-yl)ethyl]pyridine-3-carboxamide |
| 10 | 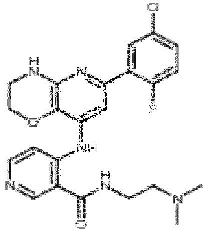 | 4-{[6-(5-chloro-2-fluorophenyl)-2H,3H,4H-pyrido[3,2-b][1,4]oxazin-8-yl]amino}-N-[2-(dimethylamino)ethyl]pyridine-3-carboxamide |
| 12 | 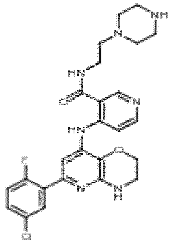 | 4-{[6-(5-chloro-2-fluorophenyl)-2H,3H,4H-pyrido[3,2-b][1,4]oxazin-8-yl]amino}-N-[2-(piperazin-1-yl)ethyl]pyridine-3-carboxamide |
| 16 | 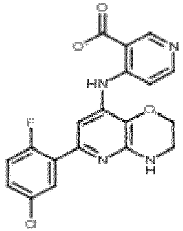 | 4-{[6-(5-chloro-2-fluorophenyl)-2H,3H,4H-pyrido[3,2-b][1,4]oxazin-8-yl]amino}pyridine-3-carboxylate |
| 17 | 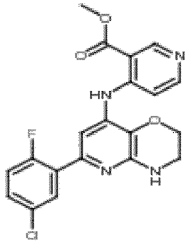 | methyl 4-{[6-(5-chloro-2-fluorophenyl)-2H,3H,4H-pyrido[3,2-b][1,4]oxazin-8-yl]amino}pyridine-3-carboxylate |
| 21 | 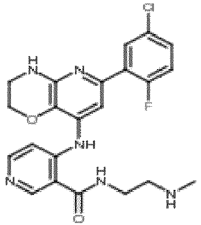 | 4-{[6-(5-chloro-2-fluorophenyl)-2H,3H,4H-pyrido[3,2-b][1,4]oxazin-8-yl]amino}-N-[2-(methylamino)ethyl]pyridine-3-carboxamide |
| 22 | 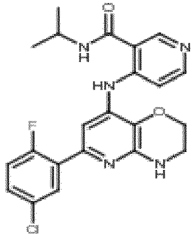 | 4-{[6-(5-chloro-2-fluorophenyl)-2H,3H,4H-pyrido[3,2-b][1,4]oxazin-8-yl]amino}-N-(propan-2-yl)pyridine-3- carboxamide |
| 23 | 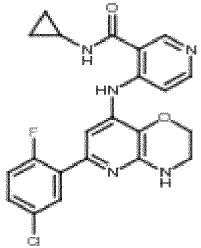 | 4-{[6-(5-chloro-2-fluorophenyl)-2H,3H,4H-pyrido[3,2-b][1,4]oxazin-8-yl]amino}-N-cyclopropylpyridine-3-carboxamide |
| 24 | 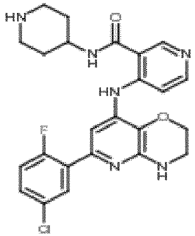 | 4-{[6-(5-chloro-2-fluorophenyl)-2H,3H,4H-pyrido[3,2-b][1,4]oxazin-8-yl]amino}-N-(piperidin-4-yl)pyridine-3-carboxamide |
| 27 | 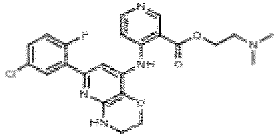 | 2-(dimethylamino)ethyl 4-{[6-(5-chloro-2-fluorophenyl)-2H,3H,4H-pyrido[3,2-b][1,4]oxazin-8-yl]amino}pyridine-3-carboxylate |
| 28 | 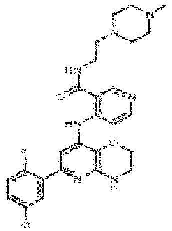 | 4-{[6-(5-chloro-2-fluorophenyl)-2H,3H,4H-pyrido[3,2-b][1,4]oxazin-8-yl]amino}-N-[2-(4-methylpiperazin-1-yl)ethyl]pyridine-3-carboxamide |
| 29 | 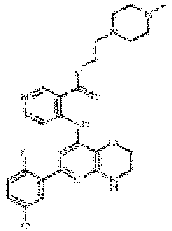 | 2-(4-methylpiperazin-1-yl)ethyl 4-{[6-(5-chloro-2-fluorophenyl)-2H,3H,4H-pyrido[3,2-b][1,4]oxazin-8-yl]amino}pyridine-3-carboxylate |
| 30 | 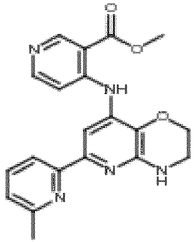 | methyl 4-{[6-(6-methylpyridin-2-yl)-2H,3H,4H-pyrido[3,2-b][1,4]oxazin-8-yl]amino}pyridine-3-carboxylate |
| 32 | 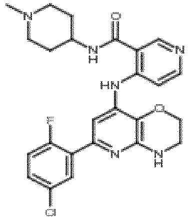 | 4-{[6-(5-chloro-2-fluorophenyl)-2H,3H,4H-pyrido[3,2-b][1,4]oxazin-8-yl]amino}-N-(1-methylpiperidin-4-yl)pyridine-3-carboxamide |
| 37 | 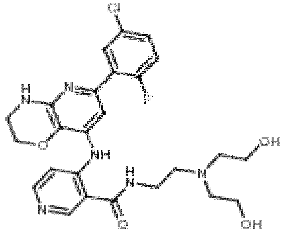 | N-{2-[bis(2-hydroxyethyl)amino]ethyl}-4-{[6-(5-chloro-2-fluorophenyl)-2H,3H,4H-pyrido[3,2-b][1,4]oxazin-8-yl]amino}pyridine-3-carboxamide |
| 39 | 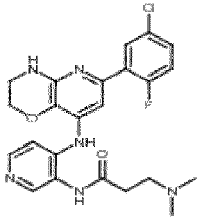 | 4-{[6-(5-chloro-2-fluorophenyl)-2H,3H,4H-pyrido[3,2-b][1,4]oxazin-8-yl]amino}-N-[2-(dimethylamino)ethyl]pyridine-3-carboxamide |
| 42 | 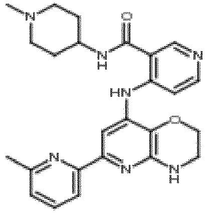 | N-(1-methylpiperidin-4-yl)-4-{[6-(6-methylpyridin-2-yl)-2H,3H,4H-pyrido[3,2-b][1,4]oxazin-8-yl]amino }pyridine-3-carboxamide |
| 43 | 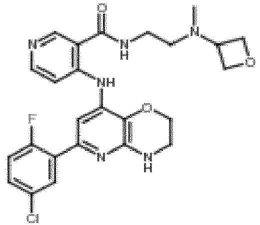 | 4-{[6-(5-chloro-2-fluorophenyl)-2H,3H,4H-pyrido[3,2-b][1,4]oxazin-8-yl]amino}-N-{2-[methyl(oxetan-3- yl)amino]ethyl}pyridine-3-carboxamide |
| 44 | 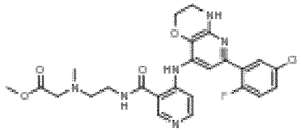 | methyl 2-({2-[(4-{[6-(5-chloro-2-fluorophenyl)-2H,3H,4H-pyrido[3,2-b][1,4]oxazin-8-yl]amino}pyridin-3-yl)formamido]ethyl}(methyl)amino )acetate |
| 46 | 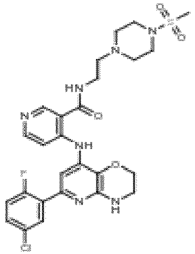 | 4-{[6-(5-chloro-2-fluorophenyl)-2H,3H,4H-pyrido[3,2-b][1,4]oxazin-8-yl]amino}-N-[2-(4-methanesulfonylpiperazin-1-yl)ethyl]pyridine-3-carboxamide |
| 47 | 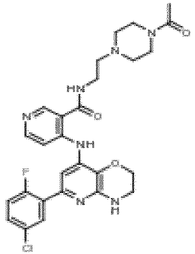 | N-[2-(4-acetylpiperazin-1-yl)ethyl]-4-{[6-(5-chloro-2-fluorophenyl)-2H,3H,4H-pyrido[3,2-b][1,4]oxazin-8-yl]amino}pyridine-3-carboxamide |

In a more preferred embodiment, the present invention refers to a compound of formula (Iab), wherein
**R₁** is selected from the group consisting of -C(O)NR₃R₅ and -C(O)OR₇;
**R₃** is H or -(C₁-C₆)alkyl;
**R₅** is H or is selected from the group consisting of -(C₁-C₆)alkyl, -(C₁₋C₆)alkylene-NR_{A}R_{B}, cycloalkyl, heterocycloalkyl optionally substituted by a -(C₁₋C₆)alkyl and -
(C₁₋C₆)alkylene-heterocycloalkyl, wherein said heterocycloalkyl is optionally substituted by a -(C₁-C₆)alkyl;
**R₇** is H or is selected from the group consisting of -(C₁₋C₆)alkyl and -(C₁₋ C₆)alkylene-heterocycloalkyl, wherein said heterocycloalkyl is optionally substituted by a -(C₁-C₆)alkyl;
**R_{A}** is H or is selected from the group consisting of -(C₁₋C₆)alkyl and -(C₁-C₆)hydroxyalkyl;
**R_{B}** is selected from the group consisting of -(C₁₋C₆)alkyl and -(C₁-C₆)hydroxyalkyl.

In even more preferred embodiment, the present invention refers to a compound of formula (Iab), wherein
**R₁** is selected from the group consisting of -C(O)NHR₅ and -C(O)OR₇;
**R₅** is selected from the group consisting of methyl, 2-(methylamino)ethyl, cyclopropyl, 2-(4-methylpiperazin-1-yl)ethyl, 2-(dimethylamino)ethyl, 2-(piperazin-1-yl)ethyl, (piperidin-4-yl), 1-methylpiperidin-4-yl and 2-[bis(2-hydroxyethyl)amino] ethyl;
**R₇** is H or is selected from the group consisting of methyl and 2-(piperazin-1-yl)ethyl.

According to a preferred embodiment, the invention refers to at least one of the compounds of Formula (Iab) listed in the Table 8 below and pharmaceutical acceptable salts thereof. These compounds are particularly active on receptor ALK5, as shown in Table 10.

**Table 8: List of preferred compounds of Formula (Iab)**

| **Example No.** | **Structure** | **Chemical Name** |
|---|---|---|
| 2 | 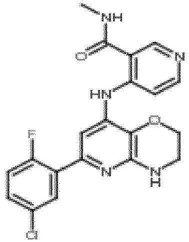 | 4-{[6-(5-chloro-2-fluorophenyl)-2H,3H,4H-pyrido[3,2-b][1,4]oxazin-8-yl]amino}-N-methylpyridine-3-carboxamide |
| 10 | 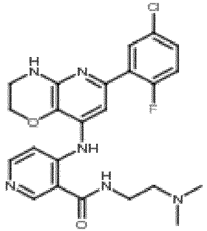 | 4-{[6-(5-chloro-2-fluorophenyl)-2H,3H,4H-pyrido[3,2-b][1,4]oxazin-8-yl]amino}-N-[2-(dimethylamino)ethyl]pyridine-3-carboxamide |
| 12 | 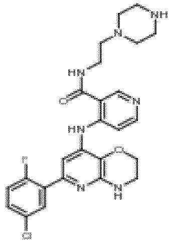 | 4-{[6-(5-chloro-2-fluorophenyl)-2H,3H,4H-pyrido[3,2-b][1,4]oxazin-8-yl]amino}-N-[2-(piperazin-1-yl)ethyl]pyridine-3-carboxamide |
| 16 | 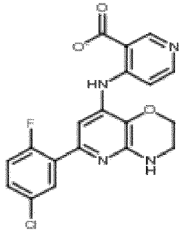 | 4-{[6-(5-chloro-2-fluorophenyl)-2H,3H,4H-pyrido[3,2-b][1,4]oxazin-8-yl]amino}pyridine-3-carboxylate |
| 17 | 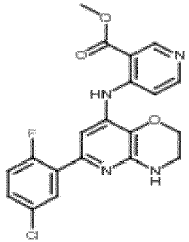 | methyl 4-{[6-(5-chloro-2-fluorophenyl)-2H,3H,4H-pyrido[3,2-b][1,4]oxazin-8-yl]amino}pyridine-3-carboxylate |
| 21 | 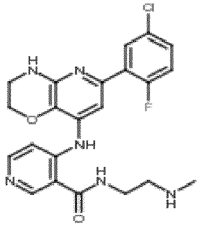 | 4-{[6-(5-chloro-2-fluorophenyl)-2H,3H,4H-pyrido[3,2-b][1,4]oxazin-8-yl]amino}-N-[2-(methylamino)ethyl]pyridine-3-carboxamide |
| 23 | 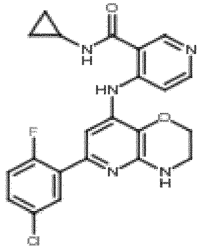 | 4-{[6-(5-chloro-2-fluorophenyl)-2H,3H,4H-pyrido[3,2-b][1,4]oxazin-8-yl]amino}-N-cyclopropylpyridine-3-carboxamide |
| 24 | 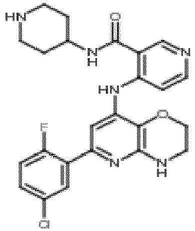 | 4-{[6-(5-chloro-2-fluorophenyl)-2H,3H,4H-pyrido[3,2-b][1,4]oxazin-8-yl]amino}-N-(piperidin-4-yl)pyridine-3-carboxamide |
| 29 | 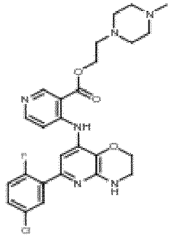 | 2-(4-methylpiperazin-1-yl)ethyl 4-{[6-(5-chloro-2-fluorophenyl)-2H,3H,4H-pyrido[3,2-b][1,4]oxazin-8-yl]amino}pyridine-3-carboxylate |
| 32 | 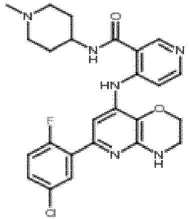 | 4-{[6-(5-chloro-2-fluorophenyl)-2H,3H,4H-pyrido[3,2-b][1,4]oxazin-8-yl]amino}-N-(1-methylpiperidin-4-yl)pyridine-3-carboxamide |
| 37 | 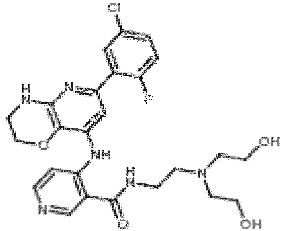 | N-{2-[bis(2-hydroxyethyl)amino]ethyl}-4-{[6-(5-chloro-2-fluorophenyl)-2H,3H,4H-pyrido[3,2-b][1,4]oxazin-8-yl]amino}pyridine-3-carboxamide |

In a equally preferred embodiment the present invention refers to a compound of formula (I), wherein **A** is group **A2** 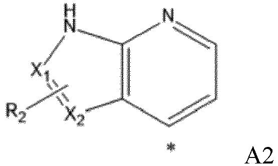 represented by the formula (Ib) 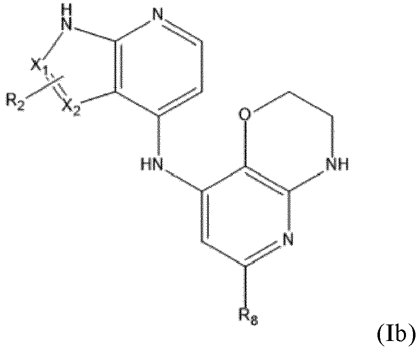
**X₁** and **X₂** are independently C or N;
**R₂** is H or is selected from the group consisting of -C(O)OR₃ and -C(O)NR₃R₆; or **R₂** is absent when X₁ or X₂ is N;
**R₃** is H or -(C₁-C₆)alkyl;
**R₆** is selected from the group consisting of -(C₁₋C₆)alkylene-NR₃R₄ and -(C₁₋ C₆)alkylene-heterocycloalkyl, wherein said heterocycloalkyl is optionally substituted by a -(C₁-C₆)alkyl;
**R₈** is selected from the group consisting of aryl and heteroaryl, wherein said aryl is optionally substituted by one or more groups selected from halogen atoms, -(C₁-C₆)alkyl and -(C₁-C₄)haloalkyl, and said heteroaryl is optionally substituted by one or more groups selected from -(C₁-C₆)alkyl, halogen atoms and -O-(C₁-C₆)alkyl;
and pharmaceutically acceptable salts thereof.

According to a even more preferred embodiment, the invention refers to at least one of the compounds represented by Formula (Ib) listed in the Table 9 below and pharmaceutical acceptable salts thereof. These compounds are particularly active on receptor ALK5, as shown in Table 10.

**Table 9: List of preferred compounds of Formula (Ib)**

| **Example No.** | **Structure** | **Chemical Name** |
|---|---|---|
| 25 | 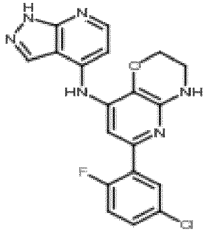 | 6-(5-chloro-2-fluorophenyl)-N-{1H-pyrazolo[3,4-b]pyridin-4-yl}-2H,3H,4H-pyrido[3,2-b][1,4]oxazin-8-amine |
| 31 | 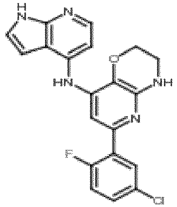 | 6-(5-chloro-2-fluorophenyl)-N-1H-pyrrolo[2,3-b]pyridin-4-yl}-2H,3H,4H-pyrido[3,2-b][1,4]oxazin-8-amine |
| 35 | 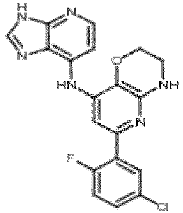 | 6-(5-chloro-2-fluorophenyl)-N-{3H-imidazo[4,5-b]pyridin-7-yl}-2H,3H,4H-pyrido[3,2-b][1,4]oxazin-8-amine |
| 36 | 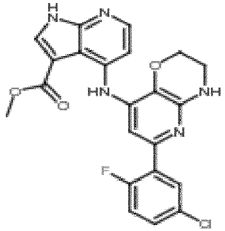 | methyl 4-{[6-(5-chloro-2-fluorophenyl)-2H,3H,4H-pyrido[3,2-b][1,4]oxazin-8-y1]amino}-1H-pyrrolo[2,3-b]pyridine-3-carboxylate |
| 38 | 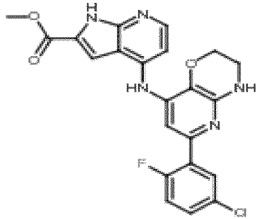 | methyl 4-{[6-(5-chloro-2-fluorophenyl)-2H,3H,4H-pyrido[3,2-b][1,4]oxazin-8-yl]amino}-1H-pyrrolo[2,3-b]pyridine-2-carboxylate |
| 40 | 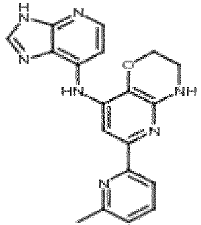 | N-{3H-imidazo[4,5-b]pyridin-7-yl}-6-(6-methylpyridin-2-yl)-2H,3H,4H-pyrido[3,2-b][1,4]oxazin-8-amine |
| 41 | 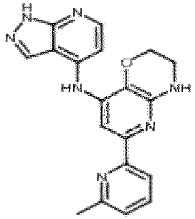 | 6-(6-methylpyridin-2-yl)-N-{1H-pyrazolo[3,4-b]pyridin-4-yl}-2H,3H,4H-pyrido[3,2-b][1,4]oxazin-8-amine |
| 45 | 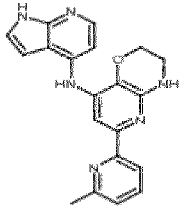 | 6-(6-methylpyridin-2-yl)-N-{1H-pyrrolo[2,3-b]pyridin-4-yl}-2H,3H,4H-pyrido[3,2-b][1,4]oxazin-8-amine |
| 48 | 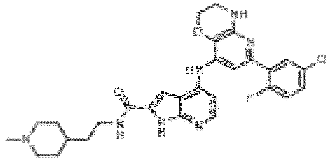 | 4-{[6-(5-chloro-2-fluorophenyl)-2H,3H,4H-pyrido[3,2-b][1,4]oxazin-8-yl]amino}-N-[2-(1-methylpiperidin-4-yl)ethyl]-1H-pyrrolo[2,3-b]pyridine-2-carboxamide |
| 49 | 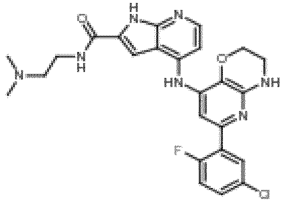 | 4-{[6-(5-chloro-2-fluorophenyl)-2H,3H,4H-pyrido[3,2-b][1,4]oxazin-8-yl]amino}-N-[2-(dimethylamino)ethyl]-1H-pyrrolo[2,3-b]pyridine-2-carboxamide |

In a even more preferred embodiment, the present invention refers to a compound of Formula (Ib), wherein **A** is group **A2a** 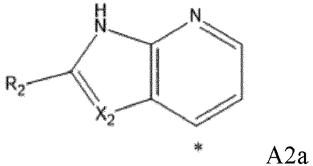 represented by the formula (Iba) 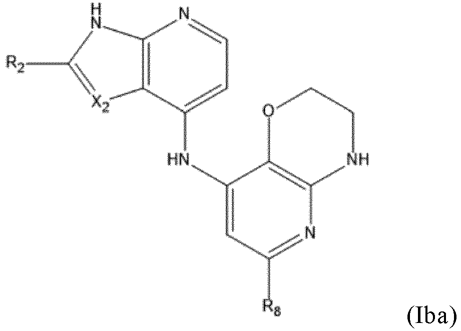 wherein
**X₂** is CH or N;
**R₂** is H or is selected from the group consisting of -C(O)OR₃ and -C(O)NR₃R₆;
**R₃** is H or -(C₁-C₆)alkyl;
**R₆** is selected from the group consisting of -(C₁₋C₆)alkylene-NR₃R₄ and -(C₁₋ C₆)alkylene-heterocycloalkyl, wherein said heterocycloalkyl is optionally substituted by a -(C₁-C₆)alkyl.

In a even more preferred embodiment, the present invention refers to a compound of formula (Iba), wherein
**R₂** is H;
**X₂** is CH.

In another even more preferred embodiment, the present invention refers to a compound of formula (Iba), wherein
**X₂** is CH;
**R₆** is selected from the group consisting of 2-(1-methylpiperidin-4-yl)ethyl and 2-(dimethylamino)ethyl.

In a further more preferred embodiment, the present invention refers to a compound of Formula (Ib), wherein **A** is **A2b** 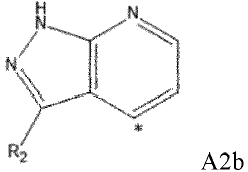 represented by the formula (Ibb) 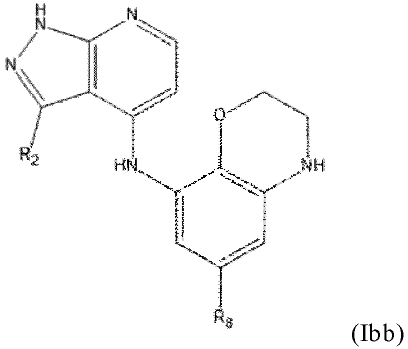 wherein
**R₂** is H or -C(O)OR₃;
**R₃** is H or methyl.

In another preferred embodiment, **R₈** is selected from the group consisting of phenyl, optionally substituted by one or more groups selected from fluorine, chlorine and methyl, and pyridinyl, optionally substituted by one or more groups selected from methyl and chlorine.

The compounds of the invention, including all the compounds here above listed, can be prepared from readily available starting materials using the following general methods and procedures or by using slightly modified processes readily available to those of ordinary skill in the art. Although a particular embodiment of the present invention may be shown or described herein, those skilled in the art will recognize that all embodiments or aspects of the present invention can be obtained using the methods described herein or by using other known methods, reagents and starting materials. When typical or preferred process conditions (i.e. reaction temperatures, times, mole ratios of reactants, solvents, pressures, etc.) are given, other process conditions can also be used unless otherwise stated. While the optimum reaction conditions may vary depending on the particular reactants or solvent used, such conditions can be readily determined by those skilled in the art by routine optimization procedures.

Thus, processes described below and reported in the following schemes should not be viewed as limiting the scope of the synthetic methods available for the preparation of the compounds of the invention.

In some cases a step is needed in order to mask or protect sensitive or reactive moieties, generally known protective groups (PG) could be employed, in accordance to general principles of chemistry (Protective group in organic syntheses, 3rd ed. T. W. Greene, P. G. M. Wuts).

The compounds of formula (I) of the present invention have surprisingly been found to effectively inhibit the receptor ALK5. Advantageously, the inhibition of ALK5 may result in efficacious treatment of the diseases or condition wherein the ALK5 signaling is involved.

In this respect, it has now been found that the compounds of formula (I) of the present invention have an inhibitory drug potency expressed as half maximal inhibitory concentration (IC₅₀) on ALK5 lower or equal than 10 nM as shown in the present experimental part.

Preferably, the compounds of the present invention have an IC₅₀ on ALK5 between 5 and 10 nM.

Even more preferably, the compounds of the present invention have an IC₅₀ on ALK5 lower than 1 nM.

In one aspect, the present invention refers to a compound of formula (I) or a pharmaceutically acceptable salt thereof, for use as a medicament. Thus, the invention refers to a compound of formula (I) in the preparation of a medicament, preferably for use in the prevention and/or treatment of a disease, disorder or condition associated with dysregulated ALK5 signaling pathway.

In a preferred embodiment, the invention refers to a compound of formula (I) or a pharmaceutically acceptable salt thereof, for use in the prevention and/or treatment of a disease, disorder or condition associated with with dysregulated ALK5 signaling pathway. In one embodiment, the present invention refers to a compound of formula (I) useful for the prevention and/or treatment of fibrosis and/or diseases, disorders, or conditions that involve fibrosis.

The terms "fibrosis" or "fibrosing disorder," as used herein, refers to conditions that are associated with the abnormal accumulation of cells and/or fibronectin and/or collagen and/or increased fibroblast recruitment and include but are not limited to fibrosis of individual organs or tissues such as the heart, kidney, liver, joints, lung, pleural tissue, peritoneal tissue, skin, cornea, retina, musculoskeletal and digestive tract.

Preferably, the compounds of formula (I) of the present invention, or a pharmaceutical composition comprising a compound of formula (I), are useful for the treatment and/or prevention of fibrosis such as pulmonary fibrosis, idiopathic pulmonary fibrosis (IPF), hepatic fibrosis, renal fibrosis, ocular fibrosis, cardiac fibrosis, arterial fibrosis and systemic sclerosis.

More preferably, the compounds of formula (I) of the present invention, or a pharmaceutical composition comprising a compound of formula (I), are useful for the treatment of idiopathic pulmonary fibrosis (IPF).

As used herein, "safe and effective amount" in reference to a compound of formula (I) or a pharmaceutically acceptable salt thereof or other pharmaceutically-active agent means an amount of the compound sufficient to treat the patient's condition but low enough to avoid serious side effects and it can nevertheless be routinely determined by the skilled artisan.

The compounds of formula (I) may be administered once or according to a dosing regimen wherein a number of doses are administered at varying intervals of time for a given period of time. Typical daily dosages may vary depending upon the route of administration chosen.

The present invention also refers to a pharmaceutical composition comprising a compound of formula (I) in admixture with at least one or more pharmaceutically acceptable carrier or excipient.

In one embodiment, the invention refers to a pharmaceutical composition of compounds of formula (I) in admixture with one or more pharmaceutically acceptable carrier or excipient, for example those described in Remington's Pharmaceutical Sciences Handbook, XVII Ed., Mack Pub., N.Y., U.S.A.

Administration of the compounds of the invention and their pharmaceutical compositions may be accomplished according to patient needs, for example, orally, nasally, parenterally (subcutaneously, intravenously, intramuscularly, intrasternally and by infusion) and by inhalation.

Preferably, the compounds of the present invention are administered orally or by inhalation.

More preferably, the compounds of the present invention are administered by inhalation.

In one preferred embodiment, the pharmaceutical composition comprising the compound of formula (I) is a solid oral dosage form such as tablets, gelcaps, capsules, caplets, granules, lozenges and bulk powders.

In one embodiment, the pharmaceutical composition comprising the compound of formula (I) is a tablet.

The compounds of the invention can be administered alone or combined with various pharmaceutically acceptable carriers, diluents (such as sucrose, mannitol, lactose, starches) and known excipients, including suspending agents, solubilizers, buffering agents, binders, disintegrants, preservatives, colorants, flavorants, lubricants and the like.

In a further embodiment, the pharmaceutical composition comprising a compound of formula (I) is a liquid oral dosage forms such as aqueous and nonaqueous solutions, emulsions, suspensions, syrups, and elixirs. Such liquid dosage forms can also contain suitable known inert diluents such as water and suitable known excipients such as preservatives, wetting agents, sweeteners, flavorants, as well as agents for emulsifying and/or suspending the compounds of the invention.

In a further embodiment, the pharmaceutical composition comprising the compound of formula (I) is an inhalable preparation such as inhalable powders, propellant-containing metering aerosols or propellant-free inhalable formulations.

For administration as a dry powder, single- or multi-dose inhalers known from the prior art may be utilized. In that case the powder may be filled in gelatine, plastic or other capsules, cartridges or blister packs or in a reservoir.

A diluent or carrier chemically inert to the compounds of the invention, e.g. lactose or any other additive suitable for improving the respirable fraction may be added to the powdered compounds of the invention.

Inhalation aerosols containing propellant gas such as hydrofluoroalkanes may contain the compounds of the invention either in solution or in dispersed form. The propellant-driven formulations may also contain other ingredients such as cosolvents, stabilizers and optionally other excipients.

The propellant-free inhalable formulations comprising the compounds of the invention may be in form of solutions or suspensions in an aqueous, alcoholic or hydroalcoholic medium and they may be delivered by jet or ultrasonic nebulizers known from the prior art or by soft-mist nebulizers.

The compounds of the invention can be administered as the sole active agent or in combination with other pharmaceutical active ingredients.

The dosages of the compounds of the invention depend upon a variety of factors including among others the particular disease to be treated, the severity of the symptoms, the route of administration and the like.

The invention is also directed to a device comprising a pharmaceutical composition comprising a compound of formula (I) according to the invention, in form of a single- or multi-dose dry powder inhaler or a metered dose inhaler.

All preferred groups or embodiments described above for compounds of formula (I) may be combined among each other and apply as well *mutatis mutandis.*

The compounds of formula (I) including all the compounds or at least one of the here above listed can be generally prepared according to the procedure outlined in detail in the Schemes shown below, using generally known methods.

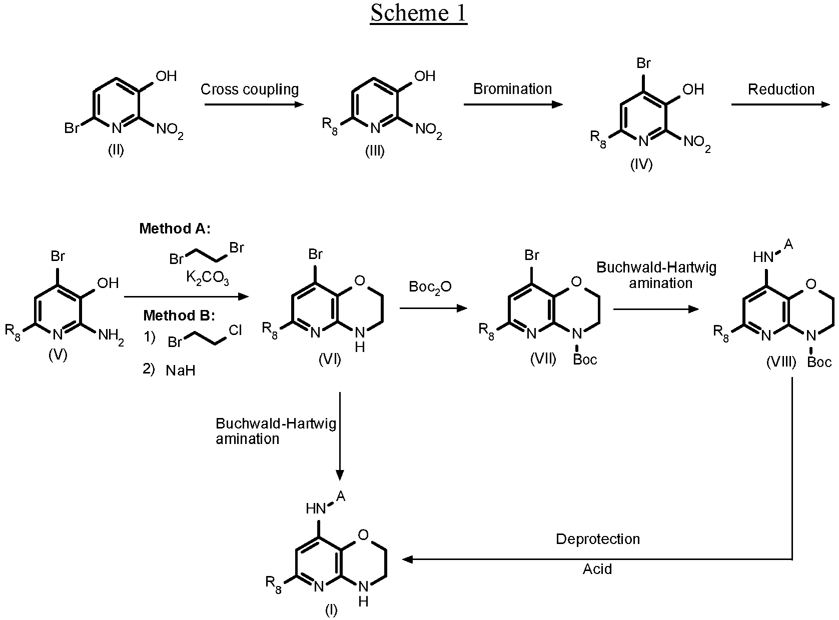

In one embodiment of the present invention, compounds of formula (I) may be prepared as described in Scheme 1, starting from commercially available compound (II).

Compounds (III), wherein Rs is as defined above, may be prepared from compound (II) by cross coupling reaction, such as Suzuki or Stille cross couplings in presence of a Pd catalyst. Typical Suzuki conditions comprise reacting compound (II) with a suitable boronic acid, in the presence of a Pd catalyst, such as Pd(dppf)Cl₂, in a mixture of solvents, such as 1,4-dioxane and water, at an appropriate temperature, such as, for example, 100 °C. Typical Stille cross coupling conditions comprise reacting compound (II) with a suitable organo-tin reagent, in the presence of a Pd catalyst, such as Pd(PPh₃)₄, in a suitable solvent, such as toluene, and at an appropriate temperature, such as for example 100 °C. In some cases, for example wherein R₈ is heteroaryl, the cross coupling may be preceeded by a protection step of OH group as MOM (methoxymethyl), using for example chloromethyl methyl ether in presence of a suitable base, such as N,N-diisopropylethylamine, in a suitable solvent, such as DCM. Typical deprotection conditions to remove a MOM group comprise treatment with acid, such as TFA, in a suitable solvent, such as DCM, at an appropriate temperature, such as at room temperature.

Compounds (IV) may be prepared from compounds (III) by bromination using for example bromine in the presence of sodium methoxide, in a solvent, like for example methanol, at low temperature like, for example 0 °C.

Compounds (V) may be prepared from compouds (IV) by reduction of the nitro group using, for example, sodium borohydride and nickel (II) chloride hexahydrate in MeOH and dry THF as solvents.

Compounds of formula (VI) may be prepared from a compound of formula (V) using a one-step procedure (Method A), by means of an alkylating agent, for example 1,2-dibromoethane, in the presence of a base, for example potassium carbonate, in a solvent, for example DMF, at a temperature, for example, of 80° C. Alternatively, compounds of formula (VI) may be prepared using a two-step alkylation/cyclization sequence (Method B). Typical alkylation conditions comprise reacting a compound of formula (V) with a suitable alkylating agent, such as 1-bromo-2-chloroethane in the presence of a base, such as potassium carboante, in a suitable solvent, such as DMF and at an appropriate temperature, such as, for example, at room temperature. Subsequent intramolecular cyclization may be carried-out, for example, in presence of sodium hydride, in a suitable solvent, such as DMF, and at appropriate temperature, for example, ranging from 0 °C to room temperature.

Compounds of formula (I) may be prepared from compounds of formula (VI) under standard Buchwald-Hartwig amination conditions, for example in the presence of a base, for example sodium *tert*-butoxide, a ligand reagent, for example Xantphos, and a catalyst, for example Pd₂(dba)₃, in a solvent, like 1,4-dioxane and at a temperature, for example, of 90 °C.

Alternatively, protection of the nitrogen of a compound of formula (VI) as *tert-*butoxy carbonyl (Boc), using di-*tert*-butyl dicarbonate (Boc anhydride, BoczO) in the presence of a base, such as lithium bis(trimethylsilyl)amide, in a suitable solvent such as THF, afforded compounds of formula (VII). Introduction of an amine on compound of formula (VII) may be achieved using, for example, the typical Buchwald-Hartwig conditions described above. Finally, removal of the Boc protecting group under acidic conditions, such as, for example, HCl solution in 1,4-dioxane and MeOH at room temperature, allowed to obtain compounds of formula (I).

In another embodiment, compounds of formula (I) can be prepared as described in Scheme 2.

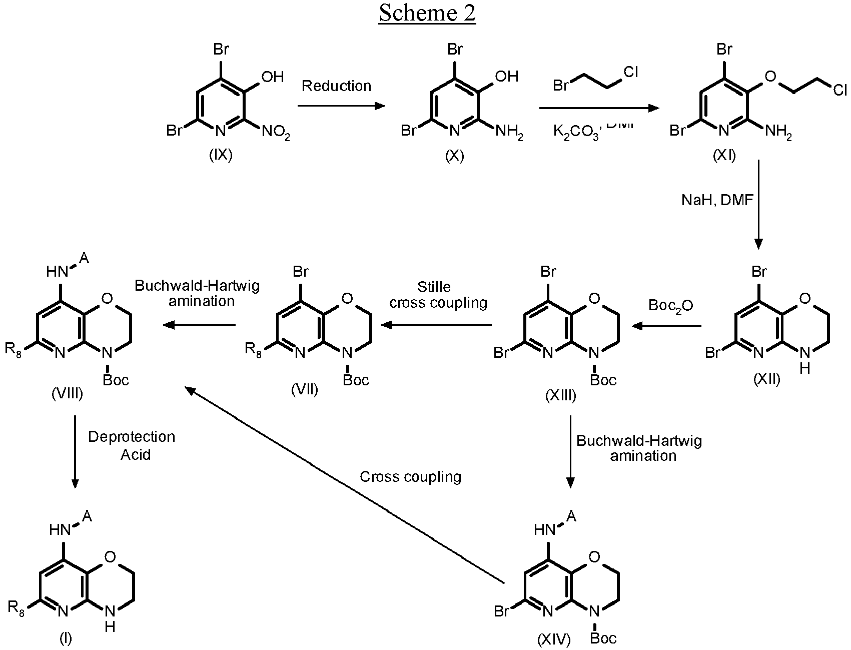

Compound (X) may be prepared from the commercially available 4,6-dibromo-2-nitropyridin-3-ol (IX) by reduction of the nitro group using, for example, sodium borohydride and nickel (II) chloride hexahydrate in a mixture of solvents, for example dry MeOH and dry THF, at a temperature, for example of 0 °C.

Compound (XI) may be prepared by alkylation of compound (X) with 1-bromo-2-chloroethane in the presence of a base, for example potassium carbonate, in dry DMF.

Compound (XII) may be prepared by intramolecular cyclization of compound (XI) in the presence of sodium hydride, in a solvent, for example DMF, at a temperature, for example, ranging from 0 °C to room temperature.

Compound of formula (XIII) may be prepared from compound (XII) by protection of the nitrogen using di-*tert*-butyl dicarbonate (Boc anhydride, BoczO) in the presence of a base, for example lithium bis(trimethylsilyl)amide, in a solvent, for example THF.

Compounds of formula (VII) may be prepared by reaction of compound (XIII) in a cross coupling reaction, for example a Stille cross coupling, using an organo-tin reagent, in the presence of a Pd catalyst, for example Pd(PPh₃)₄, in a solvent, for example toluene at a temperature, for example, of 100 °C. In some cases, a mixture of regioisomers could be observed and the unwanted compound is separated by silica flash chromatography or other well known methods to one skilled in the art.

Compounds of formula (VIII) may be prepared from a compound of formula (VII) by Buchwald-Hartwig amination reaction, in the presence of a base, for example tripotassium phosphate, a ligand reagent, for example Xantphos, and a catalyst, for example Pd₂(dba)₃, in a solvent, for example 1,4-dioxane, at a temperature, for example, of 120 °C.

Compounds of formula (I) may be prepared by removal of the Boc protecting group under acidic conditions, for example, in HCl solution in 1,4-dioxane and MeOH at room temperature.

Compounds of formula (XIV) may be prepared reacting a compound of formula (XIII) under Buchwald-Hartwig amination conditions and separation of the unwanted regioisomer, in the presence of a base, for example tripotassium phosphate, a ligand reagent, for example Xantphos, and a catalyst, for example Pd₂(dba)₃, in a solvent, for example 1,4-dioxane, at a temperature, for example, of 120 °C.

Compounds of formula (VIII) may be prepared from compounds of formula (XIV) by Suzuki cross coupling, by reacting with a boronic acid, in the presence of a Pd catalyst, for example Pd(PPh₃)₄, in a mixture of solvents, for example 1,2-dimethoxyethane and water, at a temperature, for example, of 70 °C. Typical Stille cross coupling conditions comprise reacting a compound of formula (XIV) with a suitable organo-tin reagent, in the presence of Pd catalyst, such as Pd(PPh₃)₄, in a suitable solvent or mixture of solvents, such as toluene and DMF, at an appropriate temperature, for example 100 °C.

Compounds of formula (I) may be prepared from compounds (VIII) as described above.

In some embodiments, compounds of general formula (I) may be prepared as described in Scheme 3.

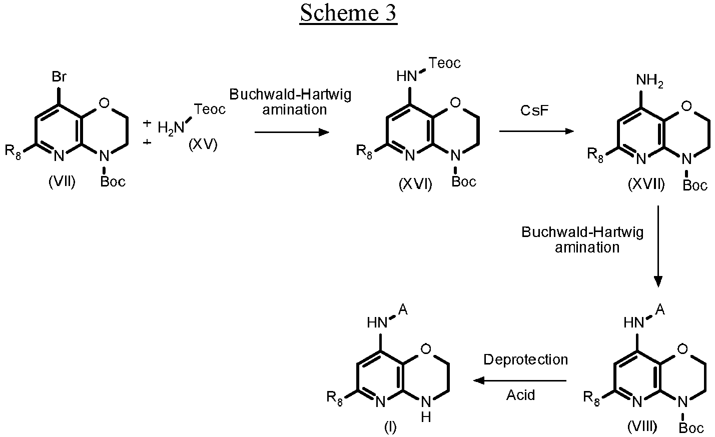

Compounds of formula (XVI) may be prepared reacting a compound of formula (VII) with Teoc-NH₂ (XV, 2-(trimethylsilyl)ethyl carbamate) under Buchwald-Hartwig cross coupling conditions, in the presence of a, for example cesium carbonate, a ligand reagent, for example Xantphos, and a catalyst, for example Pd₂(dba)₃, in a solvent, for example 1,4-dioxane, at a temperature, for example, of 100 °C.

Compounds of formula (XVII) may be prepared by selective cleavage of Teoc (2-(trimethylsilyl)ethoxy carbonyl) using cesium fluoride in DMF.

Compound of general formula (VIII) may be prepared reacting a compound of formula (XVII) with an heteroaryl halide under Buchwald-Hartwig cross coupling conditions, in the presence of a base, for example cesium carbonate, a ligand reagent, for example Xantphos, and a catalyst, for example Pd₂(dba)₃, in a solvent, for example 1,4-dioxane, at a temperature, for example, of 100 °C.

Compounds of formula (I) may be prepared from compound (VIII) by removal of the protecting group under acidic conditions, for example, HCl solution in 1,4-dioxane and MeOH at room temperature.

In a further aspect the present invention relates to the use of compounds of formula (III), (IV), (V), (VI), (VII), (VIII), (XIV), (XVI) and (XII) as intermediates in the preparation of compounds of formula (I) as above described.

The various aspects of the invention described in this application are illustrated by the following examples which are not meant to limit the invention in any way.

### PREPARATIONS OF INTERMEDIATES AND EXAMPLES

Chemical Names of the compounds were generated with Structure To Name Enterprise 10.0 Cambridge Software.

All reagents, for which the synthesis is not described in the experimental part, are either commercially available, or are known compounds or may be formed from known compounds by known methods by a person skilled in the art.

In the procedures that follow, some of the starting materials are identified through an "Intermediate" or "Example" number with indications on step number. This is provided merely for assistance to the skilled chemist.

A "similar" or "analogous" procedure means that such a procedure may involve minor variations, for example reaction temperature, reagent/solvent amount, reaction time, work-up conditions or chromatographic purification conditions.

### ABBREVIATION - MEANING

Cs₂CO₃= Cesium carbonate
CsF= Cesium fluoride
DCM= Dichloromethane
DIPEA= N,N-Diisopropylethylamine
DME= 1,2-Dimethoxyethane
DMF= Dimethylformamide
DMSO= Dimethylsulfoxide
DTBPF= 1,1'-Bis(di-tert-butylphosphino)ferrocene
EtOAc= Ethyl Acetate
FC= flash chromatography
h= hour
hrs= hours
HATU= 1-[Bis(dimethylamino)methylene]-1H-1,2,3-triazolo[4,5-b]pyridinium 3-oxid hexafluorophosphate
HCl= Hydrochloric acid
HCOOH= Formic acid
K₂CO₃= Potassium carbonate
KF= Potassium fluoride
K₃PO₄= Potassium triphosphate
LC-MS= liquid chromatography/mass spectrometry
MeCN= Acetonitrile
MeOH= Methanol
N₂= Nitrogen
NaOH= Sodium hydroxide
Na₂SO₄= Sodium sulfate
Na₂CO₃= Sodium carbonate
NH₄Cl= Ammonium chloride
NH₄OH= Ammonium hydroxide
NBS= N-bromo succinimmide
Pd/C= Palladium on carbon
Pd₂(dba)₃= Tris(dibenzylideneacetone)dipalladium(0)
Pd(dppf)Cl₂ DCM= [1,1'-Bis(diphenylphosphino)ferrocene]dichloropalladium(II), complex with dichloromethane
Pd(PPh₃)₄= Tetrakis(triphenylphosphine)palladium(0)
PL-HCO₃ = Polymer supported hydrogencarbonate
PLS= Parallel Liquid Synthesizer
PPh₃= triphenylphosphine
RT= room temperature
Sat.=saturated
SCX= Strong Cation Exchange
SPE= Solid Phase Extraction
*t*Bu= *tert-*butyl
TEA= Triethylamine
TFA= Trifluoroacetic acid
T3P^{®} = Propylphosphonic anhydride solution
THF= Tetrahydrofuran
Xantphos= 4,5-Bis(diphenylphosphino)-9,9-dimethylxanthene
XPhos Pd G2= Chloro(2-dicyclohexylphosphino-2',4',6'-triisopropyl-1,1'-biphenyl)[2-(2'-amino-1,1'-biphenyl)]palladium(II)

### General Experimental Details and methods

### Analytical method

### Instruments, materials and methods employed for analyses

1H-NMR spectra were performed on a Varian MR-400 spectrometer operating at 400 MHZ (proton frequency), equipped with: a self-shielded Z-gradient coil 5 mm 1H/nX broadband probe head for reverse detection, deuterium digital lock channel unit, quadrature digital detection unit with trans mitter offset frequency shift, or on AgilentVNMRS-500 or on a Bruker Avance 400 spectrometers. Chemical shift are reported as 6 values in ppm relative to trimethylsilane (TMS) as an internal standard. Coupling constants (J values) are given in hertz (Hz) and multiplicities are reported using the following abbreviation (br= broad signal, s= singlet, d= doublet, dd= doublet of doublets, ddd= doublet of doublet of doublets, t= triplet, dt= doublet of triplets, ddt= doublet of doublet of triplets, td= triplet of doublets, q= quartet, qd= quartet of doublets, m= multiplet).

### LC/UV/MS Analytical Methods

LC/MS retention times are estimated to be affected by an experimental error of +0.5 min. LCMS may be recorded under the following conditions: diode array DAD chromatographic traces, mass chromatograms and mass spectra may be taken on UPLC/PDA/MS AcquityTM system coupled with Micromass ZQTM or Waters SQD single quadrupole mass spectrometer operated in positive and/or negative electron spray ES ionization mode and/or Fractionlynx system used in analytical mode coupled with ZQTM single quadrupole operated in positive and/or negative ES ionisation mode. Quality Control methods used operated under low pH conditions or under high pH conditions:
Method 1, low pH conditions column: Acquity CSH C18 2.1x50mm 1.7um, the column temperature was 40°C; mobile phase solvent A was milliQ water+0.1% HCOOH, mobile phase solvent B MeCN+0.1% HCOOH. The flow rate was 1 mL/min.

The gradient table was t=0 min 97% A 3% B, t=1.5 min 0.1% A 99.9% B, t=1.9 min 0.1% A 99.9% B and t=2 min 97% A 3% B. The UV detection range was 210-350 nm and ES+/ES- range was 100 to 1500 AMU.

Method 2, high pH conditions: column: Acquity Kinetex 1.7 um EVO C18 100A, 2.1x50mm, the column temperature was 40°C; mobile phase solvent A was 10 mM aqueous solution of NH4HCO3 adjusted to pH=10 with ammonia, mobile phase solvent B MeCN. The flow rate was 1 mL/min. The gradient table was t=0 min 97% A 3% B, t=1.5 min 0.1% A 99.9% B, t=1.9 min 0.1% A 99.9% B and t=2 min 97% A 3% B. The UV detection range was 210-350 nm and ES+/ES- range was 100 to 1500 AMU.

### PREPARATIONS OF INTERMEDIATES

### Intermediate 1: 6-(5-chloro-2-fluorophenyl)-2-nitropyridin-3-ol

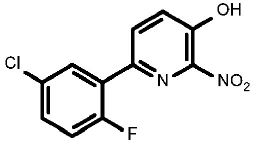

### Method A

A mixture of 6-bromo-2-nitropyridin-3-ol (2.0 g, 9.13 mmol), 5-chloro-2-fluorophenylboronic acid (2.4 g, 13.7 mmol), K₃PO₄ (5.8 g, 27.4 mmol) and XPhos Pd G2 (0.36 g, 0.46 mmol) was dissolved in THF/water (48 mL, 3:1 ratio). The mixture was degassed, then stirred at RT overnight. The reaction was partitioned between EtOAc and acidified brine. The organic phase was separated, dried over Na₂SO₄, filtered and concentrated at reduced pressure. The crude material was purified by FC on Biotage silica gel (from 0% to 50% of DCM in c-Hex as eluent). Evaporation of opportune fractions provided 6-(5-chloro-2-fluorophenyl)-2-nitropyridin-3-ol (1.47 g, 5.49 mmol, 60% yield), as yellow solid.

### Method B

A mixture of 6-bromo-2-nitropyridin-3-ol (1.00 g, 4.57 mmol), 5-chloro-2-fluorophenylboronic acid (0.96 g, 5.48 mmol), Na₂CO₃ (1.45 g, 13.70 mmol) and Pd(dppf)Cl₂ · DCM (0.56 g, 0.68 mmol) was dissolved in 1,4-dioxane/water (45 mL, 2:1 ratio). The mixture was degassed, then heated at 100 °C for 1.5 hrs. The reaction was diluted with EtOAc, filtered through a Celite^{®} pad, washing with EtOAc. The filtrate was washed with acidified brine. The organic phase was filtered through a phase separator and evaporated under vacuum. The residue was purified by FC on Biotage silica gel (100% c-Hex, then 100% of DCM as eluent), affording Intermediate 1 (1.05 g, 3.91 mmol, 86% yield) as yellow solid.

LC-MS (ESI): *m*/*z* (M+1): 269.0 (Method 1)

### Intermediate 2: 4-bromo-6-(5-chloro-2-fluorophenyl)-2-nitropyridin-3-ol

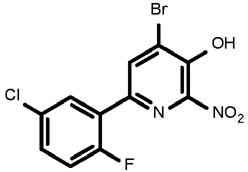

### Method A

To a solution of Intermediate 1 (1.47 g, 5.48 mmol) and DIPEA (1.16 mL, 5.23 mmol) in DCM (30 mL), NBS (1.17 g, 6.58 mmol) was added and the mixture stirred overnight at RT. Further NBS (1 g, 5.62 mmol) was added and stirring continued for 4.5 hrs. Then, the mixture was concentrated under reduced pressure, suspended in the minimum amount of DCM and filtered. The solid was collected and dried at high vacuum to provide 4-bromo-6-(5-chloro-2-fluorophenyl)-2-nitropyridin-3- ol (0.536 g, 1.54 mmol, 28% yield) as a yellow solid. The filtrate was concentrated at reduced pressure and the crude material was by FC on Biotage silica gel (from 0 to 100% of EtOAc in c-Hex as eluent). Pure fractions were evaporated to provide a second batch of title compound (0.8 g, 2.38 mmol, 11%) as an orange solid.

### Method B

To an ice-cooled suspension of Intermediate 1 (5.49 g, 20.42 mmol) in dry MeOH (100 mL), sodium methoxide (~25% solution in MeOH, 5.6 mL, 24.51 mmol) was added and the suspension turned into a yellow solution. Then molecular bromine (1.26 mL, 24.51 mmol) was added dropwise. The solution turned into a suspension and the mixture was stirred at 0 °C for 45 min. The mixture was quenched with water, then extracted with EtOAc (2x). The combined organic layers were washed with 10% aqueous Na₂S₂O₃ (2x) and brine (1x), filtered through a phase separator and concentrated under vacuum. The crude material was taken up with EtOAc and the resulting precipitate was filtered and dried at high vacuum to 4-bromo-6-(5-chloro-2-fluorophenyl)-2-nitropyridin-3-ol (4.03 g, 11.61 mmol, 57% yield), as an orange solid. The material was used in the next step without further purification.

LC-MS (ESI): m/z (M-1): 344.9 (Method 1)

### Intermediate 3: 2-amino-4-bromo-6-(5-chloro-2-fluorophenyl)pyridin-3-ol

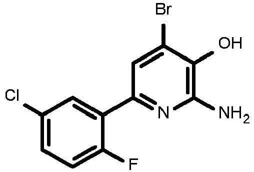

To an ice-cooled solution of 4-bromo-6-(5-chloro-2-fluorophenyl)-2-nitropyridin-3-ol (Intermediate 2, 1.10 g, 3.17 mmol) in a mixture of dry MeOH and dry THF (30 mL, 1:1 ratio), nickel(II) chloride hexahydrate (0.15 g, 0.63 mmol) and sodium borohydride (0.24 g, 6.3 mmol) were subsequently added. The reaction was stirred at 0 °C for 30 min. The mixture was quenched with water and allowed to reach the RT. The pH was adjusted to 3-4 using 2 N aqueous HCl solution and the mixture was extracted with EtOAc (2x). The combined organic layers were washed with brine, filtered through a phase separator and evaporated under vacuum. The residue was dissolved in MeOH and loaded on a SCX column (10 g), washed with MeOH then eluted with 2 N ammonia in MeOH. Proper fractions were evaporated to provide Intermediate 3 (0.53 g, 1.68 mmol, 53% yield) as a brown solid. The material was used in the next step without further purification.

LC-MS (ESI): *m*/*z* (M+1): 316.9 (Method 1)

### Intermediate 4: 4-bromo-6-(5-chloro-2-fluorophenyl)-3-(2-chloroethoxy)pyridin-2-amine

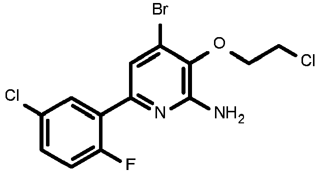

To a mixture of Intermediate 3 (532 mg, 1.68 mmol) and K₂CO₃ (463 mg, 3.35 mmol) in dry DMF (9.3 mL), 1-bromo-2-chloroethane (209 µL, 2.51 mmol) was added and the reaction was stirred at RT overnight. The mixture was diluted with EtOAc and washed with sat. aqueous NaHCO₃ solution (3x) and brine. The organic phase was filtered through a phase separator and concentrated under vacuum. The residue was purified by FC on Biotage Silica-NH gel (from 0% to 30% of EtOAc in c-Hex as eluent) to provide the title compound (425 mg, 1.12 mmol, 67% yield) as colorless oil that slowly solidifies.

LC-MS (ESI): *m*/*z* (M+1): 378.9 (Method 1)

### Intermediate 5: 8-bromo-6-(5-chloro-2-fluorophenyl)-2H,3H,4H-pyrido[3,2-b][1,4]oxazine

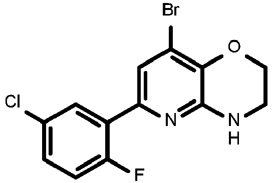

### Method A

Intermediate 3 (0.53 g, 1.68 mmol) and K₂CO₃ (1.86 g, 13.43 mmol) were suspended in dry DMF (55 mL), then 1,2-dibromoethane (0.43 mL, 5.04 mmol) was added and the mixture was stirred at 80 °C overnight. The reaction was diluted with EtOAc and washed with brine. The organic phase was filtered through a phase separator and concentrated under vacuum. The crude material was purified by reverse FC on Biotage C18 cartridge (from 5% to 100% of MeCN + 0.1% HCOOH in water + 0.1% HCOOH as eluent), affording Intermediate 5 (0.16 g, 0.48 mmol, 29% yield).

### Method B

Under N₂ atmosphere, a solution of Intermediate 4 (410 mg, 1.08 mmol) in dry DMF (9 mL) was added dropwise to an ice-cooled suspension of sodium hydride (60% dispersion in mineral oil, 56 mg, 1.40 mmol) in dry DMF (1 mL). A yellow color developed. The mixture was allowed to reach the RT and stirred for 30 min. The reaction was cooled again to 0 °C, quenched with water and diluted with EtOAc. Phases were separated and the organic layer was washed with sat. aqueous NaHCO₃ solution (3x) and brine, filtered through a phase separator and concentrated under vacuum, affording Intermediate 5 (380 mg, recovery assumed quantitative),as pale yellow solid.

LC-MS (ESI): *m*/*z* (M+1): 342.9 (Method 1)

### Intermediate 6: methyl 4-{[6-(5-chloro-2-fluorophenyl)-2H,3H,4H-pyrido[3,2-b][1,4]oxazin-8-yl]amino}pyridine-3-carboxylate

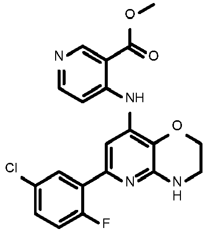

A mixture of Intermediate 5 (70 mg, 0.20 mmol), Pd₂(dba)₃ (11.2 mg, 0.01 mmol), sodium *tert*-butoxide (24.5 mg, 0.25 mmol) and Xantphos (14.2 mg, 0.02 mmol) was suspended in 1,4-dioxane (2 mL). The mixture was degassed (vacuum/Nz) then methyl 4-aminopyridine-3-carboxylate (37.2 mg, 0.24 mmol) was added and the mixture was shaken at 90 °C in a closed vial in a PLS for 30 min. A second addition of reagents was performed: Pd₂(dba)₃ (11.2 mg, 0.01 mmol), sodium *tert*-butoxide (245 mg, 0.25 mmol), Xantphos (14.2 mg, 0.02 mmol) and methyl 4-aminopyridine-3-carboxylate (37.2 mg, 0.24 mmol) and the mixture was shaken at 90 °C in a PLS for additional 30 min. The mixture was filtered, the filtrate was concentrated under reduced pressure and the residue was dissolved in EtOAc washed with brine, filtered through a phase separator and concentrated under reduced pressure. The crude material was purified by reverse FC on Biotage C18 cartridge (from 5% to 35% of MeCN +0.1% HCOOH in water +0.1% HCOOH as eluent). Evaporation of opportune fractions provided Intermediate 6 (30 mg, 0.07 mmol, 36% yield) as a yellow solid.

LC-MS (ESI): *m*/*z* (M+1): 415.1 (Method 1)

### Intermediate 7: N-[6-(5-chloro-2-fluorophenyl)-2H,3H,4H-pyrido[3,2-b][1,4]oxazin-8-yl]-2-nitropyridin-4-amine

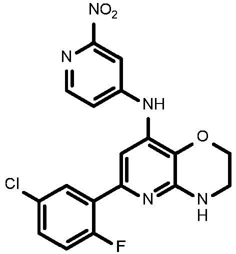

A mixture of Intermediate 5 (50 mg, 0.14 mmol), sodium *tert*-butoxide (32.8 mg, 0.34 mmol), Pd₂(dba)₃ (19.0 mg, 0.04 mmol) and Xantphos (15 mg, 0.02 mmol) was suspended in 1,4-dioxane (1 mL). The mixture was degassed (vacuum/Nz) then 2-nitropyridin-4-amine (45.7 mg, 0.330 mmol) was added and the mixture was shaken at 90 °C for 1 h. The mixture was filtered over a pad of Celite^{®} and the cake was washed with EtOAc. The organic phase was washed with sat. aqueous NaHCO₃ solution, filtered through a phase separator and concentrated at reduced pressure. The crude material was purified by reverse FC on Biotage C18 cartridge (from 5% to 40% of MeCN +0.1% HCOOH in water +0.1% HCOOH as eluent). Evaporation of opportune fractions provided Intermediate 7 (19 mg, 0.05 mmol, 35% yield) as a yellow solid.

LC-MS (ESI): *m*/*z* (M+1): 402.1 (Method 1)

### Intermediate 8: 2-amino-4,6-dibromopyridin-3-ol

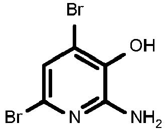

To an ice-cooled solution of 4,6-dibromo-2-nitropyridin-3-ol (3.50 g, 11.75 mmol) and nickel(II) chloride hexahydrate (0.56 g, 2.35 mmol) in a mixture of dry MeOH and dry THF (60 mL, 1:1 ratio), sodium borohydride (1.78 g, 47 mmol) was added portionwise. The reaction was stirred at 0 °C for 30 min. The mixture was quenched with cold water and the pH was adjusted to 4-5 using 3 N aqueous HCl solution and extracted with EtOAc (2x). The combined organic layers were washed with brine, dried over Na₂SO₄ and evaporated under vacuum, affording Intermediate 8 (2.70 g, 10.08 mmol, 86% yield), that was used in the next step without further purification.

LC-MS (ESI): *m*/*z* (M+1): 266.9(Method 1)

### Intermediate 9: 4,6-dibromo-3-(2-chloroethoxy)pyridin-2-amine

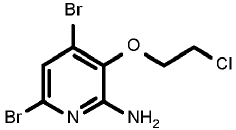

K₂CO₃ (2.27 g, 16.42 mmol) was added to a stirred solution of Intermediate 8 (2.20 g, 8.21 mmol) in dry DMF . The mixture was stirred at RT for 10 min before adding 1-bromo-2-chloroethane (1.77 g, 12.32 mmol). The reaction was stirred at RT for 20 hrs, then it was poured into 5% ice-cold citric acid aqueous solution and extracted with EtOAc. The organic phase was washed with water, dried over Na₂SO₄ and concentrated under vacuum. The residue was purified by FC on Biotage silica gel (20% of EtOAc in c-Hex as eluent), affording the title compound (1.95 g, 5.90 mmol, 72% yield).

LC-MS (ESI): *m*/*z* (M+1): 328.9(Method 1)

### Intermediate 10: 6,8-dibromo-2H,3H,4H-pyrido[3,2-b][1,4]oxazine

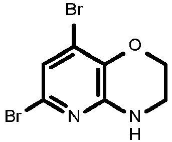

Under N₂ atmosphere, a solution of Intermediate 9 (1.95 g, 5.67 mmol) in dry DMF (35 mL) was added dropwise to an ice-cooled stirred mixture of sodium hydride (60% dispersion in mineral oil , 0.30 g, 7.37 mmol) in dry DMF (5 mL). Once the addition was completed, the reaction was warmed at RT and stirred for 2 hrs, then it was poured into 5% ice-cold citric acid aqueous solution and extracted with EtOAc. The organic phase was washed with water, dried over Na₂SO₄ and concentrated under vacuum, affording Intermediate 10 (1.63 g, 5.55 mmol, 98% yield).

LC-MS (ESI): *m*/*z* (M+1): 292.9 (Method 1)

### Intermediate 11: tert-butyl 6,8-dibromo-2H,3H,4H-pyrido[3,2-b][1,4]oxazine-4-carboxylate

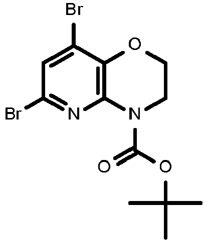

Under N₂ athmosphere, lithium bis(trimethylsilyl)amide (1.0 M solution in THF, 3.5 mL, 3.53 mmol) was added dropwise to an ice-cooled stirred solution of 6,8-dibromo-2H,3H,4H-pyrido[3,2-b][1,4]oxazine (Intermediate 10, 910 mg, 2.94 mmol) in dry THF (29 mL). After 5 min, di-*tert*-butyl dicarbonate (770 mg, 3.53 mmol) was added. The reaction was allowed to reach the RT and stirred for 1 h. The mixture was poured into an ice-cold NH₄Cl aqueous solution and extracted with EtOAc. Organic layer was separated, dried over Na₂SO₄ and evaporated at reduced pressure, providing Intermediate 12 (1.0 g, 2.54 mmol, 86% yield).

LC-MS (ESI): *m*/*z* (M+1): 392.9 (Method 2)

### Intermediate 12: tert-butyl 8-bromo-6-(4-methyl-1,3-thiazol-2-yl)-2H,3H,4H-pyrido[3,2-b][1,4]oxazine-4-carboxylate

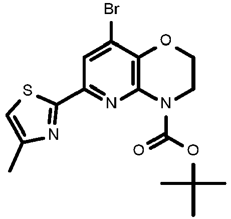

In a suitable vial, a mixture of Intermediate 11 (300 mg, 0.76 mmol) and 4-methyl-2-(tributylstannyl)-1,3-thiazole (325 mg, 0.84 mmol) in dry toluene (6 mL) was degassed by N₂ bubbling, before adding Pd(PPh₃)₄ (88.0 mg, 0.08 mmol). The vial was sealed and heated at 100 °C for 6 hrs. Volatiles were removed under vacuum. The residue material was resuspended in a mixture of Et₂O and 1 N aqueous KF solution and vigorously stirred at RT for 2 hrs. The mixture was filtered through a Celite^{®} pad, then the organic layer was separated, dried over Na₂SO₄ and evaporated at reduced pressure. The residue material was purified by FC on Biotage silica gel (10% of EtOAc in c-Hex as eluent), affording Intermediate 12 (70 mg, 0.17 mmol, 22% yield).

LC-MS (ESI): *m*/*z* (M+1): 412.0 (Method 2)

¹H NMR (500 MHz, *DMSO-d₆*) δ ppm 7.96 (s, 1 H), 7.34 - 7.40 (m, 1 H), 4.42 (t, *J*=4.7 Hz, 2 H), 3.88 - 3.92 (m, 2 H), 2.39 - 2.44 (m, 3 H), 1.45 - 1.57 (m, 9 H).

¹H NMR (500 MHz, *DMSO-d₆*) δ ppm 7.93 (s, 1 H), 7.59 - 7.61 (m, 1 H), 4.49 - 4.55 (m, 2 H), 3.92 - 3.96 (m, 2 H), 2.47 (d, *J*=0.8 Hz, 3 H), 1.50 (s, 9 H).

### Intermediate 13: tert-butyl 6-(4-methyl-1,3-thiazol-2-yl)-8-[(pyridin-4-yl)amino]-2H,3H,4H-pyrido[3,2-b][1,4]oxazine-4-carboxylate

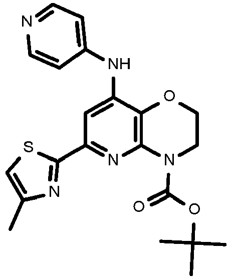

In a suitable vial, a mixture of Intermediate 12 (70 mg, 0.17 mmol), K₃PO₄ (73.1 mg, 0.34 mmol), pyridin-4-amine (17.6 mg, 0.19 mmol), Pd₂(dba)₃ (15.6 mg, 0.02 mmol) and Xantphos (14.7 mg, 0.03 mmol) in 1,4-dioxane (3 mL) was degassed by N₂ bubbling. Then the vial was sealed and submitted to MW cycle at 140 °C for 2.5 hrs. After cooling, the mixture was filtered on a Celite^{®} pad, washing with EtOAc. The filtrate was evaporated under vacuum and the residue material was purified by FC on Biotage silica gel (from 0% to 5% of MeOH in DCM as eluent), affording Intermediate 13 (25 mg, 0.06 mmol, 35% yield).

LC-MS (ESI): *m*/*z* (M+1): 426.2 (Method 2)

### Intermediate 14: 6-bromo-3-(methoxymethoxy)-2-nitropyridine

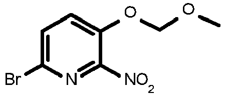

To an ice-cooled stirred solution of 6-bromo-2-nitropyridin-3-ol (5.0 g, 22.83 mmol) in dry DCM (125 mL), DIPEA (8.0 mL, 57.08 mmol) was added, followed by chloromethyl methyl ether (3.12 mL, 41.1 mmol), dropwise. The mixture was stirred at 0 °C for 40 min. The reaction was diluted with DCM, transferred into a separatory funnel and washed with sat. aqueous NaHCO₃ solution (3x) and brine (1x). The organic phase was filtered through a phase separator and concentrated under vacuum. The crude material was purified by FC on Biotage silica-NH (from 0% to 30% of EtOAc in c-Hex as eluent), to give Intermediate 14 (5.98 g, 22.75 mmol, 99% yield) as yellow oil that slowly solidifies.

LC-MS (ESI): *m*/*z* (M+1): 263.1 (Method 1)

### Intermediate 15: 5-(methoxymethoxy)-6'-methyl-6-nitro-2,2'-bipyridine

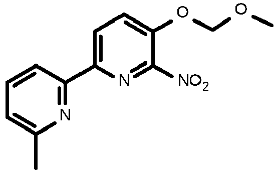

Intermediate 14 (3.0 g, 11.4 mmol) and Pd(PPh₃)₄ (1.3 g, 1.14 mmol) were dissolved in toluene (114 mL) and the mixture was degassed (vacuum/ N₂), before adding 2-methyl-6-(tributylstannyl)pyridine (5.2 g, 13.69 mmol). The reaction was stirred 100 °C for 10 hrs. Volatiles were removed under vacuum, the residue was taken up with EtOAc (30 mL) and KF was added. The mixture was stirred at RT overnight. The suspension was filtered, the filtrate was concentrated under vacuum. The crude material was purified by FC on Biotage silica-NH gel (from 5% to 30% of EtOAc in c-Hex as eluent), affording Intermediate 15 (3.04 g, 11.04 mmol, 97% yield) as yellow solid.

LC-MS (ESI): *m*/*z* (M+1): 276.2 (Method 1)

### Intermediate 16: 6'-methyl-6-nitro-[2,2'-bipyridin]-5-ol

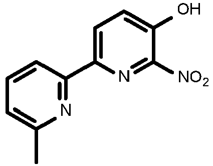

A solution of Intermediate 15 (3.04 g, 11.04 mmol) in a DCM/TFA mixture (110 mL, 9:1 ratio) was stirred at RT for 2 hrs. Volatiles were removed under vacuum and the crude material was purified by SCX (10 g, washing with MeOH, and eluting with 1 N ammonia in MeOH) to give title compound (2.48 g, 10.73 mmol, 97% yield) as yellow solid, that was used without further purification.

LC-MS (ESI): *m*/*z* (M+1): 232.1 (Method 1)

### Intermediate 17: 4-bromo-6'-methyl-6-nitro-[2,2'-bipyridin]-5-ol

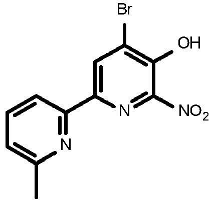

To an ice-cooled suspension of Intermediate 16 (10.73 mmol) in dry MeOH (53.6 mL), sodium methoxide (~30% in MeOH, 2.4 mL, 12.87 mmol) was added and the suspension turned into a yellow solution. Then molecular bromine (0.66 mL, 12.87 mmol) was added dropwise. The reaction was stirred at 0 °C for 30 min The mixture was quenched with 10% Na₂S₂O₃ aqueous solution, diluted with water then extracted with EtOAc (2x). The combined organic layers were washed with brine (1x), filtered through a phase separator and concentrated under vacuum. The residue was triturated with Et₂O affording Intermediate 17 (2.32 g, 7.48 mmol, 70% yield) as a yellow solid.

LC-MS (ESI): *m*/*z* (M+1): 310.1 (Method 1)

### Intermediate 18: 4-bromo-5-(2-chloroethoxy)-6'-methyl-[2,2'-bipyridin]-6-amine

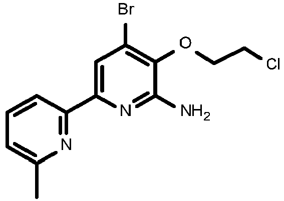

### Step 1

A mixture of Intermediate 17 (1.07 g, 3.44 mmol), bis(pinacolato)diboron (2.71 g, 10.67 mmol) and potassium *tert*-butoxide (0.96 g, 8.6 mmol) in 2-propanol (60 mL) was refluxed for 3 hrs. Volatiles were removed under vacuum, affording 6-amino-4-bromo-6'-methyl-[2,2'-bipyridin]-5-ol (3.8 g, recovery assumed quantitative), that was used in the next step without purification.

### Step 2

To a mixture of 6-amino-4-bromo-6'-methyl-[2,2'-bipyridin]-5-ol (3.44 theoretical mmol) and K₂CO₃ (1.19 g, 8.6 mmol) in dry DMF (35 mL), 1-bromo-2-chloroethane (0.52 mL, 6.19 mmol) was added and the reaction stirred at RT overnight. Further K₂CO₃ (0.59 g, 4.30 mmol) and 1-bromo-2-chloroethane (0.29 mL, 3.10 mmol) were added. After 24 hrs, the reaction was diluted with EtOAc and washed with sat. aqueous NaHCO₃ solution (3x) and brine (1x). The organic phase was filtered through a phase separator and concentrated under vacuum. The crude material was purified by FC on Biotage silica-NH gel (from 0% to 20% of EtOAc in c-Hex as eluent), affording Intermediate 18 (0.66 g, 1.92 mmol, 56% yield) as pale yellow solid.

LC-MS (ESI): *m*/*z* (M+1): 342.0 (Method 1)

### Intermediate 19: tert-butyl 8-bromo-6-(6-methylpyridin-2-yl)-2H,3H,4H-pyrido[3,2-b][1,4]oxazine-4-carboxylate

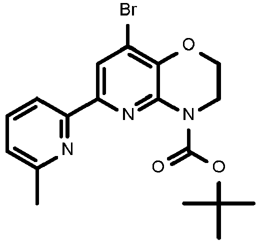

### Method A

Intermediate 19 was prepared as described for Intermediate 12, starting from Intermediate 11 (400 mg, 1.02 mmol) and 2-methyl-6-(tributylstannyl)pyridine (388 mg, 1.02 mmol). The crude material was purified by FC on Biotage silica gel (10% of EtOAc in c-Hex as eluent) and the evaporation of proper fractions provided Intermediate 19 (170 mg, 0.42 mmol, 41% yield).

### Method B

Under N₂ atmosphere, to an ice-cooled suspension sodium hydride (60% dispersion in mineral oil, 98.9 mg, 2.47 mmol) in dry DMF (3.4 mL), a solution of Intermediate 18 (651 mg, 1.9 mmol) in dry DMF (22.5 mL) was added dropwise. A yellow-orange colour developed. The mixture was stirred at RT for 30 min, then it was cooled again to 0 °C and a second portion of sodium hydride (60% dispersion in mineral oil 98.9 mg, 2.47 mmol) was added. After 20 min, di-tert-butyl dicarbonate (498 mg, 2.28 mmol) was added and the reaction was stirred at 0 °C for 1 h. The mixture was quenched with water, diluted with EtOAc and transferred to a separatory funnel. The phases were separated, the organic phase was washed with sat. aqueous NaHCO₃ solution (3x) and brine (1x), then filtered through a phase separator and concentrated under vacuum. The crude material was purified by FC on Biotage silica-NH gel (from 0% to 10% of EtOAc in c-Hex as eluent) affording Intermediate 19 (740 mg, 1.82 mmol, 96% yield) as white solid.

LC-MS (ESI): *m*/*z* (M+1): 406.2 (Method 1)

¹H NMR (500 MHz, *DMSO-d₆*) δ ppm 8.27 (s, 1 H), 8.07 (d, *J*=7.7 Hz, 1 H), 7.83 (t, *J*=7.8 Hz, 1 H), 7.27 (d, *J*=7.5 Hz, 1 H), 4.41 (t, *J*=4.5 Hz, 2 H), 3.93 (t, *J*=4.5 Hz, 2 H), 2.54 (s, 3 H), 1.50 (s, 9 H).

### Intermediate 20: tert-butyl 6-(6-methylpyridin-2-yl)-8-[(pyridin-4-yl)amino]-2H,3H,4H-pyrido [3,2-b] [1,4] oxazine-4-carboxylate

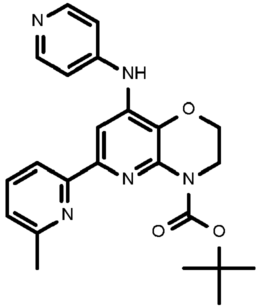

Intermediate 20 was prepared as described for Intermediate 13, starting from Intermediate 19, *tert*-butyl 8-bromo-6-(6-methylpyridin-2-yl)-2H,3H,4H-pyrido[3,2-b][1,4]oxazine-4-carboxylate (100 mg, 0.250 mmol). The crude material was purified by FC on Biotage silica-NH gel (20% of EtOAc in c-Hex as eluent) and the evaporation of proper fractions provided Intermediate 20 (30 mg, 0.07 mmol, 29% yield).

LC-MS (ESI): *m*/*z* (M+1): 420.2 (Method 2)

### Intermediate 21: 4-amino-N-methylpyridine-2-carboxamide

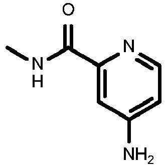

In a suitable vial, methyl 4-aminopyridine-2-carboxylate (75.0 mg, 0.49 mmol) was dissolved in methylamine, 2.0 M solution in MeOH (9.9 mL). The reaction was stirred at 60 °C for 12 hrs. Volatiles were removed under reduced pressure, affording 4-amino-N-methylpyridine-2-carboxamide (79 mg, recovery assumed quantitative) as a pale yellow solid, that was used in the next step without further purification.

LC-MS (ESI): *m*/*z* (M+1): 152 (Method 2)

### Intermediate 22: N-[6-(5-chloro-2-fluorophenyl)-2H,3H,4H-pyrido[3,2-b][1,4]oxazin-8-yl]-3-nitropyridin-4-amine

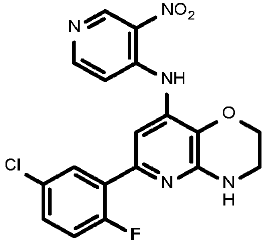

Intermediate 22 was prepared as described for Intermediate 7, starting from Intermediate 5 (100 mg, 0.29 mmol) and 4-amino-3-nitropyridine (40.5 mg, 0.29 mmol). The crude material was purified by FC on Biotage silica gel (from 0% to 50% of EtOAc in c-Hex as eluent) Fractions containing the target product were evaporated and further purified by reverse FC on Biotage C18 cartridge (from 5% to 100% of MeCN in water with ammonia-ammonium bicarbonate buffer (pH=10) as eluent). Evaporation of opportune fractions provided title compound (23 mg, 0.06 mmol, 20% yield) as an orange solid.

LC-MS (ESI): *m*/*z* (M+1): 402.1 (Method 2).

### Intermediate 23: tert-butyl 6-bromo-8-[(pyridin-4-yl)amino]-2H,3H,4H-pyrido[3,2-b] [1,4]oxazine-4-carboxylate

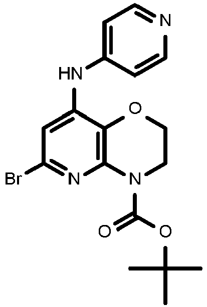

In a suitable vial, a mixture of Intermediate 11 (300 mg, 0.76 mmol), K₃PO₄ (327.8 mg, 1.52 mmol), pyridin-4-amine (78.8 mg, 0.84 mmol), Pd₂(dba)₃ (69.7 mg, 0.08 mmol) and Xantphos (66.1 mg, 0.11 mmol) in 1,4-dioxane (3 mL) was degassed by N₂ bubbling. Then the vial was sealed and submitted to MW cycle at 120 °C for 2.5 hrs. After cooling, the mixture was filtered on a Celite^{®} pad, washing with EtOAc. The filtrate was evaporated under vacuum and the residue material was purified by FC on Biotage silica gel (from 0% to 5% of MeOH in DCM as eluent), affording Intermediate 23 (65 mg, 0.16 mmol, 21% yield).

LC-MS (ESI): *m*/*z* (M+1): 407.1 (Method 1)

¹H NMR (500 MHz, *Chloroform-d*) δ ppm 8.50 (br d, *J*=5.8 Hz, 2 H), 7.21 (s, 1 H), 7.05 (br d, *J*=5.8 Hz, 2 H), 4.34 (t, *J*=4.4 Hz, 2 H), 3.93 (t, *J*=4.4 Hz, 2 H), 1.57 (s, 9H).

### Intermediate 24: tert-butyl 6-(2-fluoro-5-methylphenyl)-8-[(pyridin-4-yl)amino]-2H,3H,4H-pyrido[3,2-b][1,4]oxazine-4-carboxylate

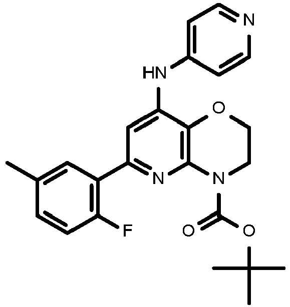

In a suitable vial, a mixture of Intermediate 23 (49 mg, 0.12 mmol), 2-fluoro-5-methylphenylboronic acid (22.2 mg, 0.14 mmol), K₂CO₃ (33.3 mg, 0.24 mmol) and Pd(PPh₃)₄ (7 mg, 0.01 mmol) in DME/water (3.6 mL, 5:1 ratio) was degassed by N₂ bubbling. Then the vial was sealed and stirred at 70 °C for 7 hrs. After cooling, the mixture poured into water and extracted with EtOAc. The organic layer was separated, dried over Na₂SO₄ and evaporated under vacuum. The residue material was purified by FC on Biotage silica gel (from 0% to 2% of MeOH in DCM as eluent), affording Intermediate 24 (40 mg, 0.09 mmol, 76 % yield).

LC-MS (ESI): *m*/*z* (M+1): 437.3 (Method 2)

### Intermediate 25: lithium 4-{[6-(5-chloro-2-fluorophenyl)-2H,3H,4H-pyrido[3,2-b][1,4]oxazin-8-yl]amino}pyridine-3-carboxylate

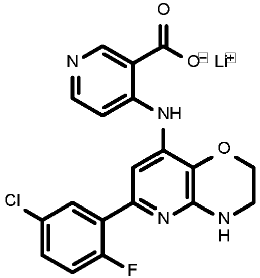

To a solution of Intermediate 6 (130 mg, 0.31 mmol) in THF (4 mL) a solution of lithium hydroxide monohydrate (14.5 mg, 0.34 mmol) in water (1 mL) was added and the reaction was stirred at RT overnight. The mixture was concentrated under reduced pressure and dried at high vacuum to provide Intermediate 25 (153 mg, recovery assumed quantitative) as a yellow solid. The material was used in the next step without further purification.

LC-MS (ESI): *m*/*z* (M+2): 201.2 (Method 1)

### Intermediate 26: tert-butyl 6-(2-fluorophenyl)-8-[(pyridin-4-yl)amino]-2H,3H,4H-pyrido[3,2-b][1,4]oxazine-4-carboxylate

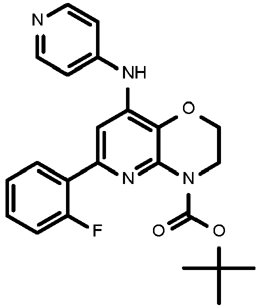

Intermediate 26 was prepared as described for Intermediate 24, starting from Intermediate 23 (55 mg, 0.14 mmol) and 2-fluorophenylboronic acid (22.7 mg, 0.16 mmol). The crude material was purified by FC on Biotage silica gel (from 0% to 2% of MeOH in DCM as eluent). Evaporation of opportune fractions provided title compound (43 mg, 0.10 mmol, 75% yield).

LC-MS (ESI): *m*/*z* (M+1): 423.2 (Method 1)

### Intermediate 27: tert-butyl 4-{2-[(4-{[6-(5-chloro-2-fluorophenyl)-2H,3H,4H-pyrido[3,2-b][1,4]oxazin-8-yl]amino}pyridin-3-yl)formamido]ethyl}piperazine-1-carboxylate

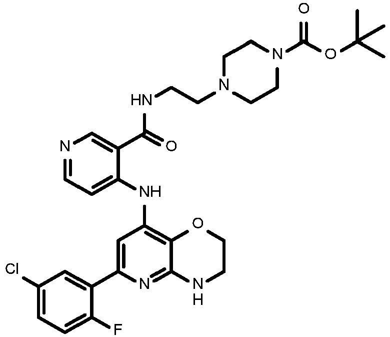

To a suspension of Intermediate 25 (50 mg, 0.12 mmol) in dry DMF (2mL), TEA (0.05 mL, 0.37 mmol) and HATU (56.92 mg, 0.15 mmol) were added and the mixture was stirred for 10 minutes at RT. *Tert*-butyl 4-(2-aminoethyl)-piperazine-1-carboxylate (28.6 mg, 0.12 mmol) was then added and the mixture was stirred for 3 hrs at RT. The mixture was poured in sat. aqueous NaHCO₃ solution and extracted with EtOAc. The organic phase was separated, filtered through a phase separator and concentrated at reduced pressure. The crude material was purified by FC on Biotage silica gel (from 0 to 10% of MeOH in DCM as eluent). Fractions containing the target product were collected and further purified by reverse FC on Biotage C18 cartridge (from 5% to 75% of MeCN in water with ammonia-ammonium bicarbonate buffer (pH=10) as eluent). Evaporation of opportune fractions provided title compound (22 mg, 0.04 mmol, 29% yield) as a yellow solid.

LC-MS (ESI): *m*/*z* (M+1): 612.3 (Method 2)

### Intermediate 28: tert-butyl 6-(6-chloropyridin-2-yl)-8-[(pyridin-4-yl)amino]-2H,3H,4H-pyrido [3,2-b] [1,4] oxazine-4-carboxylate

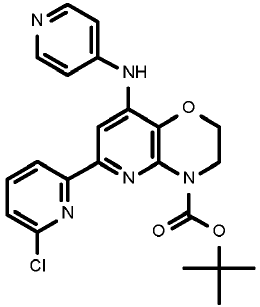

In a suitable vial, a mixture of Intermediate 23 (100 mg, 0.20 mmol) and 2-chloro-6-(tributylstannyl)pyridine (87 mg, 0.22 mmol) in dry toluene (3.6 mL) and DMF (0.6 mL) was degassed by N₂ bubbling, before adding Pd(PPh₃)₄ (22.7 mg, 0.02 mmol). The vial was sealed and heated at 100 °C for 24 hrs. Volatiles were removed under vacuum. The residue material was resuspended in a mixture of Et₂O and 1 N aqueous KF solution and vigorously stirred at RT for 2 hrs. The mixture was filtered through a Celite^{®} pad, then the organic layer was separated, dried over Na₂SO₄ and evaporated at reduced pressure. The residue material was purified by FC on Biotage silica-NH gel (20% of EtOAc in c-Hex as eluent), affording Intermediate 28 (90 mg, 0.20 mmol, quantitative yield).

LC-MS (ESI): *m*/*z* (M+1): 440.2 (Method 2)

### Intermediate 29: tert-butyl 6-(2,5-difluorophenyl)-8-[(pyridin-4-yl)amino]-2H,3H,4H-pyrido[3,2-b][1,4]oxazine-4-carboxylate

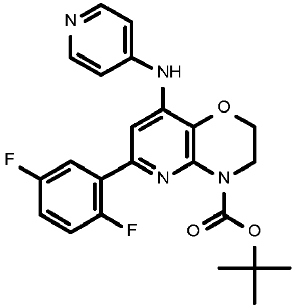

In a suitable vial, a mixture of Intermediate 23 (50 mg, 0.12 mmol), 2,5-difluorophenylboronic acid (20.5 mg, 0.13 mmol), K₂CO₃ (32.6 mg, 0.24 mmol) Pd(PPh₃)₄ (6.81 mg, 0.01 mmol) in DME/water (3.6 mL, 5:1 ratio) was degassed by N₂ bubbling. Then the vial was sealed and stirred at 80 °C for 2 hrs. After cooling, the mixture poured into water and extracted with EtOAc. The organic layer was separated, dried over Na₂SO₄ and evaporated under vacuum affording Intermediate 29 (60 mg, recovery assumed quantitative), that was used without further purification.

LC-MS (ESI): *m*/*z* (M+1): 441.3 (Method 1)

### Intermediate 30: tert-butyl 6-(3-chlorophenyl)-8-[(pyridin-4-yl)amino]-2H,3H,4H-pyrido[3,2-b][1,4]oxazine-4-carboxylate

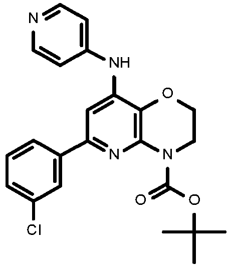

Intermediate 30 (50 mg, 0.11 mmol, 93% yield) was prepared as described for Intermediate 24, starting from Intermediate 23 (50 mg, 0.12 mmol) and 3-chlorophenylboronic acid (21.1 mg, 0.14 mmol).

LC-MS (ESI): *m*/*z* (M+1): 439.2 (Method 2)

### Intermediate 31: tert-butyl 6-(3-methylphenyl)-8-[(pyridin-4-yl)amino]-2H,3H,4H-pyrido[3,2-b][1,4]oxazine-4-carboxylate

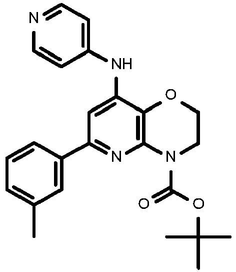

Intermediate 31 (50.0 mg, 0.12 mmol, 54% yield) was prepared as described for Intermediate 24, starting from Intermediate 23 (90 mg, 0.22 mmol) and 3-methylphenylboronic acid (30 mg, 0.22 mmol).

LC-MS (ESI): *m*/*z* (M+1): 419.2 (Method 2)

### Intermediate 32: tert-butyl 6-(3-chloro-4-fluorophenyl)-8-[(pyridin-4-yl)amino]-2H,3H,4H-pyrido[3,2-b][1,4]oxazine-4-carboxylate

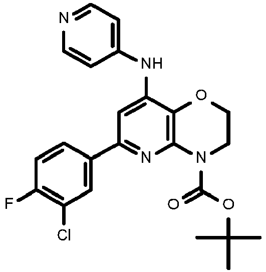

Intermediate 32 (60 mg, 0.13 mmol, 59% yield) was prepared as described for Intermediate 24, starting from Intermediate 23 (90 mg, 0.22 mmol) and 3-chloro-4-fluorophenylboronic acid (38.5 mg, 0.22 mmol).

LC-MS (ESI): *m*/*z* (M+1): 457.2 (Method 2)

### Intermediate 33: N-(4-nitropyridin-2-yl)prop-2-enamide

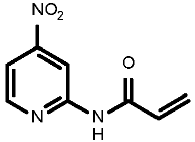

To an ice-cooled solution of 2-amino-4-nitropyridine (500 mg, 3.59 mmol) in dry DCM (20 mL), TEA (1.5 mL, 10.78 mmol) and acryloyl chloride (438 uL, 5.39 mmol) were added. The reaction was stirred at 0 °C for 30 min. Water was added, the phases were separated and the organic phase was filtered through a phase separator and evaporated under vacuum. The crude material was purified by FC on Biotage silica-gel (from 5% to 50% of EtOAc in c-Hex as eluent), affording Intermediate 33 (374 mg, 1.94 mmol, 54% yield) as yellow solid.

LC-MS (ESI): *m*/*z* (M-1): 192.1 (Method 1)

### Intermediate 34: 3-(morpholin-4-yl)-N-(4-nitropyridin-2-yl)propanamide

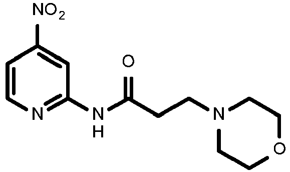

To a solution of Intermediate 33 (80 mg, 0.41 mmol) in THF (2 mL), morpholine (80 µL, 0.91 mmol) was added and the mixture was shaken at 60 °C overnight. Volatiles were removed under vacuum and the crude material was purified by FC on Biotage silica-NH gel (from 5% to 70% of EtOAc in c-Hex as eluent). Evaporation of opportune fractions provided Intermediate 34 (112 mg, 0.40 mmol, 96% yield) as a pale yellow solid.

LC-MS (ESI): *m*/*z* (M+1): 281.2 (Method 2)

### Intermediate 35: N-(4-aminopyridin-2-yl)-3-(morpholin-4-yl)propenamide

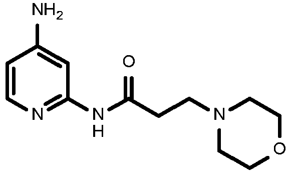

To a mixture of Intermediate 34 (112 mg, 0.40 mmol) and ammonium formate (126 mg, 2 mmol) in ethanol (3 mL), Pd/C (10% loading, 21.3 mg, 0.02 mmol) was added and the mixture was refluxed for 30 minutes. The mixture was allowed to cool to RT, filtered through a pad of Celite^{®} and the filtrate was evaporated under vacuum. The residue material was taken up with DCM and insolubles were filtered and discarded. The filtrate was concentrated at reduced pressure and dried at high vacuum to provide N-(4-aminopyridin-2-yl)-3-(morpholin-4-yl)propanamide (92 mg, 0.37 mmol, 92% yield) as an off-white solid. The material was used in the next step without further purification.

LC-MS (ESI): *m*/*z* (M+1): 251.2 (Method 2)

### Intermediate36:tert-butyl8-bromo-6-(5-chloro-2-fluorophenyl)-2H,3H,4H-pyrido[3,2-b][1,4]oxazine-4-carboxylate

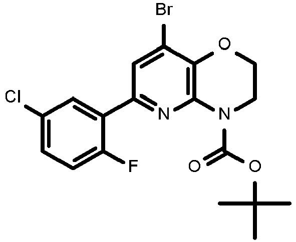

To an ice-cooled solution of Intermediate 5 (150 mg, 0.44 mmol) in THF (5 mL), lithium bis(trimethylsilyl)amide (1.0 M solution in THF, 0.52 mL, 0.52 mmol) was added. After 5 min, di-*tert*-butyl dicarbonate (115 mg, 0.52 mmol) was added and the mixture was stirred at 0 °C for 1 h. The mixture was quenched with sat. NH₄Cl aqueous solution and partitioned between EtOAc and brine. The organic phase was separated, filtered through a phase separator and concentrated under reduced pressure to provide Intermediate 36 (202 mg, 0.45 mmol) as a light brown oil that solidifies upon cooling. The material was used in the next step without further purification.

LC-MS (ESI): *m*/*z* (M+1): 443.1 (Method 1)

### Intermediate 37: tert-butyl 6-(5-chloro-2-fluorophenyl)-8-({2-[3-(morpholin-4-yl)propanamido]pyridin-4-yl}amino)-2H,3H,4H-pyrido[3,2-b][1,4]oxazine-4-carboxylate

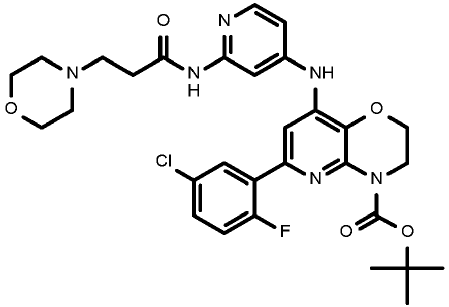

In a suitable vial, a mixture of Intermediate 36 (50 mg, 0.11 mmol), Pd₂(dba)₃ (6.19 mg, 0.01 mmol), Cs₂CO₃ (91.8 mg, 0.28 mmol) and Xantphos (7.82 mg, 0.01 mmol) was suspended in 1,4-dioxane (1 mL). The mixture was degassed (vacuum/Nz) then Intermediate 35 (33.9 mg, 0.14 mmol) was added. The vial was sealed and submitted to MW cycle at 120 °C for 30 min. The mixture was filtered through a Celite^{®} pad washing the cake with EtOAc. The filtrate was concentrated at reduced pressure and partitioned between DCM and sat. aqueous NaHCO₃ solution. The organic phase was separated, filtered through a phase separator and concentrated under vacuum. The crude material was purified by FC on Biotage silica-NH gel (from 0% to 100% of EtOAc in c-Hex as eluent). Evaporation of opportune fractions provided the title compound (35 mg, 0.06 mmol, 51% yield) as a yellow solid.

LC-MS (ESI): *m*/*z* (M+1): 613.4 (Method 2)

### Intermediate 38: tert-butyl N-{2-[(4-{[6-(5-chloro-2-fluorophenyl)-2H,3H,4H-pyrido[3,2-b][1,4]oxazin-8-yl]amino}pyridin-3-yl)formamido]ethyl}-N-methylcarbamate

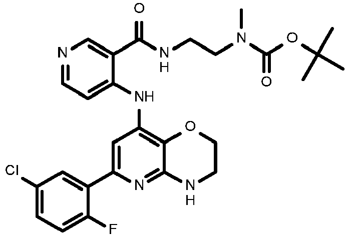

Intermediate 38 was prepared as described for Intermediate 27 starting from Intermediate 25 (33 mg, 0.08 mmol) and *tert*-butyl N-(2-aminoethyl)-N-methylcarbamate (17 mg, 0.10 mmol). The crude material was purified by FC on Biotage silica-NH gel (from 0% to 80% of EtOAc in c-Hex as eluent) and the evaporation of proper fractions provided Intermediate 38 (34 mg, 0.06 mmol, 75% yield) as a white solid.

LC-MS (ESI): *m*/*z* (M+1): 557.3 (Method 2)

### Intermediate 39: methyl 4-({4-[(tert-butoxy)carbonyl]-6-(5-chloro-2-fluorophenyl)-2H,3H,4H-pyrido[3,2-b][1,4]oxazin-8-yl}amino)pyridine-3-carboxylate

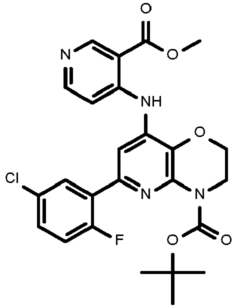

A mixture of Intermediate 36 (400 mg, 0.90 mmol), Pd₂(dba)₃ (43.9 mg, 0.05 mmol), Cs₂CO₃ (734 mg, 2.25 mmol) and Xantphos (55.6 mg, 0.10 mmol) was suspended in 1,4-dioxane (8 mL). The mixture was degassed (vacuum/N₂) then methyl 4-aminopyridine-3-carboxylate (165 mg, 1.08 mmol) was added and the mixture was shaken at 90 °C overnight. The mixture was filtered through a Celite^{®} pad and the filtrate was partitioned between EtOAc and sat. aqueous NaHCO₃ solution. The organic phase was separated, filtered through a phase separator and concentrated at reduced pressure. The crude material was purified by FC on Biotage silica gel (from 0% to 60% of EtOAc in c-Hex as eluent). Evaporation of opportune fractions provided Intermediate 39 (295 mg, 0.57 mmol, 64% yield) as a pale yellow solid.

LC-MS (ESI): *m*/*z* (M+1): 515.4 (Method 1)

### Intermediate 40: lithium 4-({4-[(tert-butoxy)carbonyl]-6-(5-chloro-2-fluorophenyl)-2H,3H,4H-pyrido[3,2-b][1,4]oxazin-8-yl}amino)pyridine-3-carboxylate

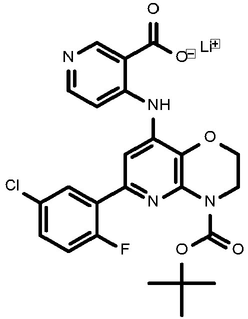

To a solution of Intermediate 39 (295 mg, 0.50 mmol) in THF (8 mL), a solution of lithium hydroxide monohydrate (25.2 mg, 0.600 mmol) in water (2 mL) was added and the mixture was stirred at RT overnight. Solvents were evaporated and the solid was dried under high vacuum to provide title compound (304 mg, recovery assumed quantitative) as a white solid. The material was used in the next step without further purification.

LC-MS (ESI): *m*/*z* (M+1): 501.3 (Method 1)

### Intermediate 41: tert-butyl 6-(5-chloro-2-fluorophenyl)-8-({3-[(propan-2-yl)carbamoyl]pyridin-4-yl}amino)-2H,3H,4H-pyrido[3,2-b][1,4]oxazine-4-carboxylate

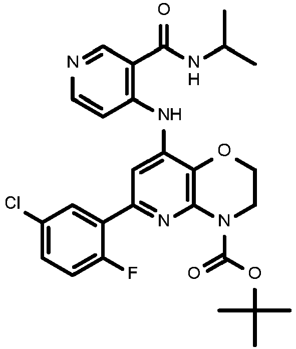

To a solution of Intermediate 40 (50 mg, 0.10 mmol) in DMF (1 mL), TEA (40 µL, 0.30 mmol) was added, followed by HATU (56.3 mg, 0.15 mmol). The mixture was stirred at RT for 10 minutes, before adding isopropylamine (12 µL, 0.15 mmol). The reaction was stirred RT overnight. The mixture was poured into sat. aqueous NaHCO₃ solution and extracted with EtOAc. The organic phase was separated, filtered through a phase separator and concentrated at reduced pressure. The residue material was purified by FC on Biotage silica-NH gel (from 5% to 100% of EtOAc in c-Hex as eluent). Proper fractions were evaporated to provide Intermediate 41 (29.5 mg, 0.05 mmol, 56% yield) as an off-white solid.

LC-MS (ESI): *m*/*z* (M+1): 542.4 (Method 1)

### Intermediate 42: tert-butyl 6-(5-chloro-2-fluorophenyl)-8-{[3-(cyclopropylcarbamoyl)pyridin-4-yl]amino}-2H,3H,4H-pyrido[3,2-b][1,4]oxazine-4-carboxylate

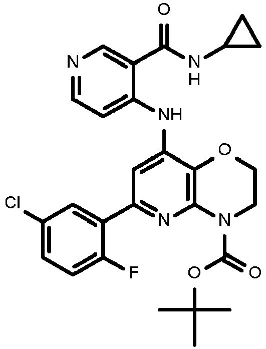

Intermediate 42 was prepared as described for Intermediate 41, starting from Intermediate 40 (50 mg, 0.10 mmol) and cyclopropanamine (10 µL, 0.15 mmol). The crude material was purified by FC on Biotage silica-NH gel (from 5% to 100% of EtOAc in c-Hex as eluent). Proper fractions were evaporated to provide Intermediate 42 (19 mg, 0.03 mmol, 35% yield) as a white solid.

LC-MS (ESI): *m*/*z* (M+1): 540.4 (Method 1)

### Intermediate 43: tert-butyl 4-[4-({4-[(tert-butoxy)carbonyl]-6-(5-chloro-2-fluorophenyl)-2H,3H,4H-pyrido[3,2-b][1,4]oxazin-8-yl}amino)pyridine-3-amido]piperidine-1-carboxylate

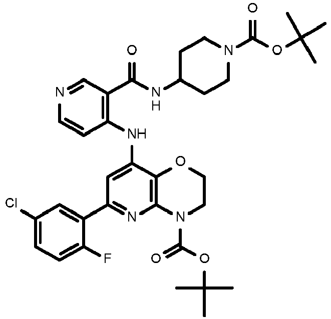

Intermediate 43 was prepared as described for Intermediate 41, starting from Intermediate 40 (50 mg, 0.10 mmol) and *tert*-butyl 4-amino-1-piperidinecarboxylate (29.6 mg, 0.15 mmol). The crude material was purified by FC on Biotage silica-NH gel (from 5% to 70% of EtOAc in c-Hex as eluent) and proper fractions were evaporated to provide the title compound (37 mg, 0.05 mmol, 55% yield) as a yellow solid.

LC-MS (ESI): *m*/*z* (M+1): 683.3 (Method 1)

### Intermediate 44: 4-chloro-1-{[2-(trimethylsilyl)ethoxy]methyl}-1H-pyrazolo[3,4-b]pyridine

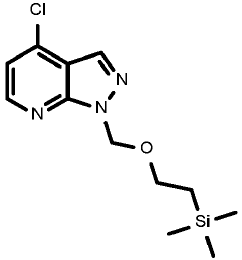

A mixture of 4-chloro-1H-pyrazolo[3,4-b]pyridine (150 mg, 0.98 mmol) and K₂CO₃ (405 mg, 2.93 mmol) in DMF (5 mL) was stirred at RT for 30 min, before adding 2-(trimethylsilyl)ethoxymethyl chloride (0.28 mL, 1.56 mmol). The reaction was stirred at RT overnight. The mixture was poured into brine and extracted with EtOAc. The organic phase was separated, filtered through a phase separator and evaporated at reduced pressure. The crude material was purified by FC on Biotage silica gel (from 0% to 10% of EtOAc in c-Hex as eluent). Evaporation of opportune fractions provided Intermediate 44 (145 mg, 0.51 mmol, 52% yield) as a colorless oil.

LC-MS (ESI): *m*/*z* (M+1): 284.1 (Method 1)

### Intermediate 45: 4-azido-1-{[2-(trimethylsilyl)ethoxy]methyl}-1H-pyrazolo[3,4-b]pyridine

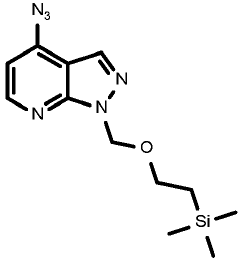

To a solution of Intermediate 44 (145 mg, 0.51 mmol) in DMF (8 mL), sodium azide (49.8 mg, 0.77 mmol) was added and the mixture was stirred at 80 °C overnight. The reaction was allowed to cool to RT and poured into sat. aqueous NaHCO₃ solution and extracted with EtOAc. The organic phase was separated, filtered through a phase separator and concentrated at reduced pressure, affording Intermediate 45 (145 mg, 0.50 mmol, 98% yield) as a yellow oil that solidifies upon cooling. The material was used in the next step without further purification.

LC-MS (ESI): *m*/*z* (M+1): 291.3 (Method 1)

### Intermediate 46: 1-{[2-(trimethylsilyl)ethoxy]methyl}-1H-pyrazolo[3,4-b]pyridin-4-amine

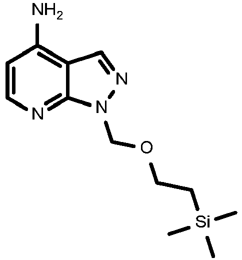

To a solution of Intermediate 45 (145 mg, 0.50 mmol) in THF (5 mL), PPh₃ (131 mg, 0.50 mmol) was added and the mixture was stirred at RT for 1.5 hrs. Then water (5 mL) and acetic acid (cat.) were added and the mixture was stirred at 50 °C overnight. The mixture was partitioned between EtOAc and sat. aqueous NaHCO₃ solution and the organic phase was separated, filtered through a phase separator and concentrated at reduced pressure, affording the title compound (237 mg, recovery assumed quantitative) as a yellow oil, that was used in the next step without further purification.

LC-MS (ESI): *m*/*z* (M+1): 265.3 (Method 1)

### Intermediate 47: tert-butyl 6-(5-chloro-2-fluorophenyl)-8-[(1-{[2-(trimethylsilyl)ethoxy]methyl}-1H-pyrazolo[3,4-b]pyridin-4-yl)amino]-2H,3H,4H-pyrido[3,2-b][1,4]oxazine-4-carboxylate

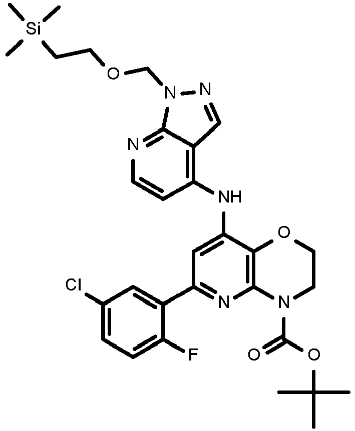

A mixture of Intermediate 36 (100 mg, 0.22 mmol), Pd₂(dba)₃ (12.4 mg, 0.01 mmol), Cs₂CO₃ (184 mg, 0.56 mmol), Xantphos (15.7 mg, 0.03 mmol) and 1-{[2-(trimethylsilyl)ethoxy]methyl}-1H-pyrazolo[3,4-b]pyridin-4-amine (Intermediate 46, 170 mg, 0.27 mmol) was suspended in 1,4-dioxane (2.5 mL). The mixture was degassed (vacuum/Nz) then was shaken overnight at 90 °C in a PLS. The mixture was filtered through a Celite^{®} pad washing the cake with EtOAc. The organic phase was washed with sat. aqueous NaHCO₃ solution, filtered through a phase separator and concentrated at reduced pressure. The crude material was purified by FC on Biotage silica-NH gel (from 0% to 40% of EtOAc in c-Hex as eluent). Evaporation of opportune fractions provided title compound (108 mg, 0.17 mmol, 76% yield) as a yellow oil.

LC-MS (ESI): *m*/*z* (M+1): 627.5 (Method 1)

### Intermediate 48: tert-butyl 6-(5-fluoropyridin-2-yl)-8-[(pyridin-4-yl)amino]-2H,3H,4H-pyrido [3,2-b] [1,4] oxazine-4-carboxylate

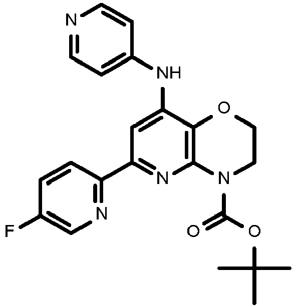

### Step 1

In a suitable vial, to a solution of -2-bromo-5-fluoropyridine (200 mg, 1.14 mmol) in 1,4-dioxane (3 mL), lithium chloride (296 mg, 6.82 mmol), tricyclohexylphosphine (31.9 mg, 0.11 mmol), bis(tributyltin) (0.67 mL, 1.36 mmol) and Pd₂(dba)₃ (52 mg, 0.06 mmol) were added. The mixture was degassed by N₂ bubbling, then the vial was sealed and heated at 100 °C for 6 hrs. Volatiles were removed under vacuum. The residue material was partially purified by FC on Biotage silica gel (10% of EtOAc in c-Hex as eluent) affording 5-fluoro-2-(tributylstannyl)pyridine (200 mg, 0.52 mmol, 46% yield) that was used in the following step without further purification.

### Step 2

In a suitable vial, a mixture of Intermediate 23 (100 mg, 0.20 mmol) and 5-fluoro-2-(tributylstannyl)pyridine (90.1 mg, 0.23 mmol) in dry toluene (4 mL) and DMF (0.4 mL) was degassed by N₂ bubbling, before adding Pd(PPh₃)₄ (26.9 mg, 0.02 mmol). The vial was sealed and heated at 100 °C for 7 hrs. Volatiles were removed under vacuum. The residue material was resuspended in a mixture of Et₂O and 1 N aqueous KF solution and vigorously stirred at RT for 2 hrs. The organic layer was separated, dried over Na₂SO₄ and evaporated at reduced pressure. The residue material was purified by FC on Biotage silica gel (from 0% to 2% of MeOH in DCM as eluent), affording Intermediate 48 (75 mg, 0.18 mmol, 76% yield).

LC-MS (ESI): *m*/*z* (M+1): 424.5 (Method 2)

### Intermediate 49: 4-{[6-(5-chloro-2-fluorophenyl)-2H,3H,4H-pyrido[3,2-b][1,4]oxazin-8-yl]amino}pyridine-3-carboxylic acid hydrochloride

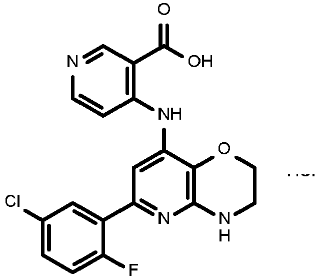

To a solution of Intermediate 25 (60 mg, 0.11 mmol) in MeOH (1 mL), ~ 1.25 M HCl solution in MeOH (1.84 mL, 2.29 mmol) was added and the mixture was stirred at 35 °C for 3 hrs. Volatiles were removed under vacuum, affording the title compound (61 mg, recovery assumed quantitative) as yellow solid, that was used in the next step without further purification.

LC-MS (ESI): *m*/*z* (M+2): 201.2 (Method 1)

### Intermediate 50: tert-butyl 6-(5-chloro-2-fluorophenyl)-8-[(3-{[2-(4-methylpiperazin-1-yl)ethyl]carbamoyl}pyridin-4-yl)amino]-2H,3H,4H-pyrido[3,2-b][1,4]oxazine-4-carboxylate

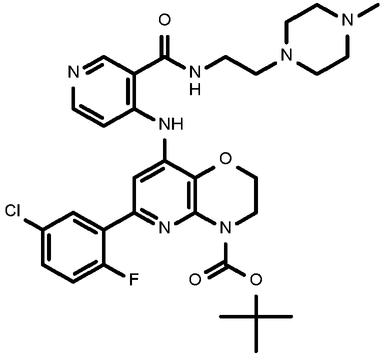

To a mixture of Intermediate 40 (100 mg, 0.20 mmol), 1-(2-aminoethyl)-4-methylpiperazine (42.4 mg, 0.30 mmol) and DIPEA (0.10 mL, 0.60 mmol), HATU (112.5 mg, 0.30 mmol) was added and the reaction was stirred at RT for 1 h. The mixture was poured into sat. aqueous NaHCO₃ solution and extracted with EtOAc. The organic phase was separated, filtered through a phase separator and concentrated at reduced pressure. The crude material was purified by FC on Biotage silica-NH gel (from 0% to 100% of EtOAc in c-Hex, followed by 20% of MeOH in EtOAc as eluent). Evaporation of proper fractions provided title compound (79 mg, 0.13 mmol, 64% yield) as a yellow oil.

LC-MS (ESI): m/z (M+1): 626.4 (Method 2)

### Intermediate 51: methyl 4-({4-[(tert-butoxy)carbonyl]-6-(6-methylpyridin-2-yl)-2H,3H,4H-pyrido[3,2-b][1,4]oxazin-8-yl}amino)pyridine-3-carboxylate

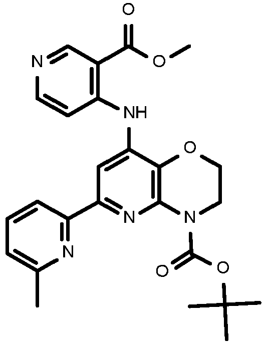

### Method A

In a suitable vial, a mixture of Intermediate 19 (160 mg, 0.39 mmol), Cs₂CO₃ (258 mg, 0.79 mmol), methyl 4-aminopyridine-3-carboxylate (71.9 mg, 0.47 mmol), Pd₂(dba)₃ (18.0 mg, 0.02 mmol) and Xantphos (22.8 mg, 0.04 mmol) in 1,4-dioxane (7 mL) was degassed by N₂ bubbling. The vial was sealed and submitted to MW cycle at 110 °C for 1 h. After cooling, the mixture was filtered on a Celite^{®} pad, washing with EtOAc. The filtrate was evaporated under vacuum and the residue material was purified by FC on Biotage silica gel (from 0% to 5% of MeOH in DCM as eluent, 0.5% of aqueous ammonia as additive), affording Intermediate 51 (40 mg, 0.08 mmol, 21% yield).

### Method B

In a suitable vial, a mixture of Intermediate 19 (200 mg, 0.49 mmol), Cs₂CO₃ (258 mg, 0.79 mmol), methyl 4-aminopyridine-3-carboxylate (89.9 mg, 0.59 mmol), Pd₂(dba)₃ (24.0 mg, 0.03 mmol) and Xantphos (30.3 mg, 0.05 mmol) was suspended in 1,4-dioxane (3.6 mL). The vial was sealed, evacuated and backfilled with N₂ (three times), then heated at 90 °C for 16 hrs in a PLS. The mixture was diluted with EtOAc, filtered over a pad of Celite^{®}. The filtrate was washed with brine (1x). The organic phase was filtered through a phase separator and concentrated under vacuum. The crude material was purified by FC on Biotage silica-NH gel (from 0% to 35% of EtOAc in c-Hex) and on Biotage silica gel (from 0% to 4% of MeOH in DCM) affording the title compound (196 mg, 0.41 mmol, 84% yield) as white solid.

LC-MS (ESI): *m*/*z* (M+1): 478.3 (Method 2)

### Intermediate 52: 4-nitro-1-{[2-(trimethylsilyl)ethoxy]methyl}-1H-pyrrolo[2,3-b]pyridine

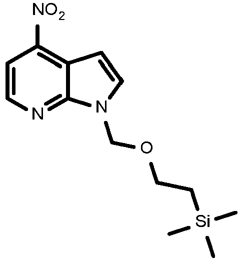

To an ice-cooled suspension of 4-nitro-1H-pyrrolo[2,3-b]pyridine (200 mg, 1.23 mmol) in THF (10 mL), sodium hydride (60% dispersion in mineral oil, 73.6 mg, 1.84 mmol) was added and the mixture was stirred at RT for 30 min, before adding 2-(trimethylsilyl)ethoxymethyl chloride (0.35 mL, 1.96 mmol). The reaction was allowed to warm to RT and stirred overnight. The mixture was poured into brine and extracted with EtOAc. The organic phase was separated, filtered through a phase separator and evaporated at reduced pressure. The crude material was purified by FC on Biotage silica gel (from 0% to 40% of EtOAc in c-Hex as eluent). Evaporation of opportune fractions provided Intermediate 52 (283 mg, 0.96 mmol, 79% yield) as a yellow oil that solidifies upon cooling.

LC-MS (ESI): *m*/*z* (M+1): 294.3 (Method 1)

### Intermediate 53: 1-{[2-(trimethylsilyl)ethoxy]methyl}-1H-pyrrolo[2,3-b]pyridin-4-amine

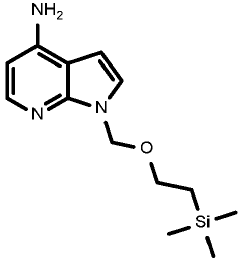

To a mixture of Intemediate 52 (283 mg, 0.96 mmol) and ammonium formate (304 mg, 4.82 mmol) in ethanol (7 mL), Pd/C (10% loading, 51.3 mg, 0.05 mmol) was added and the reaction was refluxed for 30 min. The mixture was allowed to cool to RT and filtered through a pad of Celite^{®}. The filtrate was concentrated and the residue was partitioned between DCM and sat. aqueous NaHCO₃ solution. The organic phase was separated, filtered through a phase separator and concentrated at reduced pressure to provide Intermediate 53 (240 mg, 0.91 mmol, 94% yield) as a pale yellow solid. The material was used in the next step without further purification.

LC-MS (ESI): *m*/*z* (M+1): 264.3 (Method 1)

### Intermediate 54: tert-butyl 6-(5-chloro-2-fluorophenyl)-8-[(1-{[2-(trimethylsilyl)ethoxy]methyl}-1H-pyrrolo[2,3-b]pyridin-4-yl)amino]-2H,3H,4H-pyrido[3,2-b] [1,4]oxazine-4-carboxylate

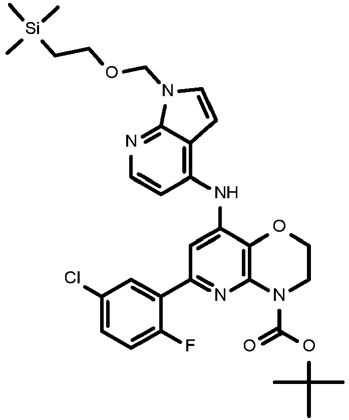

Intermediate 54 was prepared following the procedure described for Intermediate 47, starting from Intermediate 36 (100 mg, 0.22 mmol) and Intermediate 53 (71.3 mg, 0.27 mmol). The crude material was purified by FC on Biotage silica-NH gel (from 0% to 40% of EtOAc in c-Hex as eluent). Evaporation of opportune fractions provided title compound (114 mg, 0.18 mmol, 80% yield) as a yellow solid.

LC-MS (ESI): *m*/*z* (M+1): 626.5 (Method 1)

### Intermediate 55: tert-butyl 6-(5-chloro-2-fluorophenyl)-8-({3-[(1-methylpiperidin-4-yl)carbamoyl]pyridin-4-yllamino)-2H,3H,4H-pyrido[3,2-b][1,4]oxazine-4-carboxylate

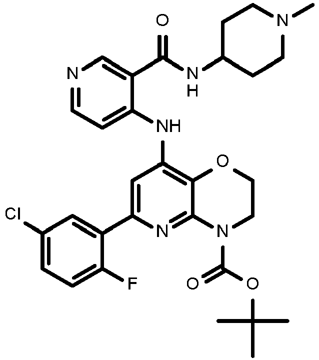

Intermediate 55 was prepared as described for Intermediate 50 starting from Intermediate 40 (95 mg, 0.18 mmol) and 1-methylpiperidin-4-amine (31.5 mg, 0.27 mmol). The crude material was purified by FC on Biotage silica-NH gel (from 0% to 5% of MeOH in DCM as eluent) and proper fractions were evaporated to provide title compound (87 mg, 0.15 mmol, 81% yield) as an off-white solid.

LC-MS (ESI): *m*/*z* (M+1): 597.3 (Method 2)

### Intermediate 56: 3-(4-methylpiperazin-1-yl)-N-(4-nitropyridin-2-yl)propanamide

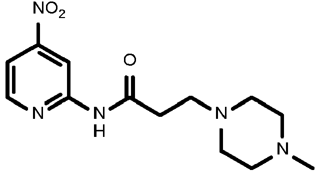

Intermediate 56 was prepared as described for Intermediate 34, starting from Intermediate 33 (120 mg, 0.62 mmol) and 1-methylpiperazine (0.15 mL, 1.37 mmol). The crude material was purified by FC on Biotage silica-NH gel (from 0% to 100% of EtOAc in c-Hex as eluent). Evaporation of opportune fractions provided 3-(4-methylpiperazin-1-yl)-N-(4-nitropyridin-2-yl)propanamide (175 mg, 0.60 mmol, 96% yield) as a pale yellow solid.

LC-MS (ESI): *m*/*z* (M+1): 294.2 (Method 2)

### Intermediate 57: N-(4-aminopyridin-2-yl)-3-(4-methylpiperazin-1-yl)propanamide

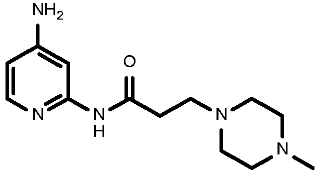

Intermediate 57 was prepared following the procedure described for Intermediate 3, starting from Intermediate 56 (175 mg, 0.60 mmol). The crude material was purified by FC on Biotage silica-NH gel (from 0% to 2% of MeOH in DCM as eluent). Evaporation of opportune fractions provided N-(4-aminopyridin-2-yl)-3-(4-methylpiperazin-1-yl)propanamide (121 mg, 0.46 mmol, 77% yield) as an orange solid.

LC-MS (ESI): *m*/*z* (M+1): 264.2 (Method 2)

### Intermediate 58: tert-butyl 6-(5-chloro-2-fluorophenyl)-8-({2-[3-(4-methylpiperazin-1-yl)propanamido]pyridin-4-yl}amino)-2H,3H,4H-pyrido[3,2-b][1,4]oxazine-4-carboxylate

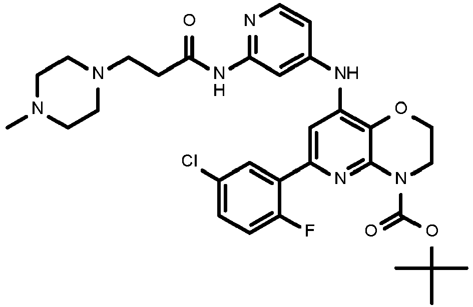

Intermediate 58 was prepared following the procedure described for Intermediate 37, starting from Intermediate 36 (100 mg, 0.22 mmol) and Intermediate 57 (84.8 mg, 0.32 mmol). The crude material was purified by FC on Biotage silica-NH gel (from 0% to 100% of EtOAc in c-Hex, followed by 20% of MeOH in EtOAc as eluent). Evaporation of opportune fractions provided title compound (68 mg, 0.109 mmol, 48% yield) as a yellow oil.

LC-MS (ESI): *m*/*z* (M+1): 626.4 (Method 2)

### Intermediate 59: 3-(dimethylamino)-N-(4-nitropyridin-2-yl)propanamide

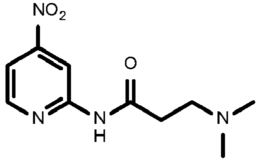

Intermediate 59 was prepared as described for Intermediate 34, starting from Intermediate 33 (120 mg, 0.62 mmol) and dimethylamine (2 M solution in THF, 0.68 mL, 1.37 mmol). The crude material was purified by FC on Biotage silica-NH gel (from 0% to 100% of EtOAc in c-Hex as eluent). Evaporation of opportune fractions provided Intermediate 59 (144 mg, 0.60 mmol, 97% yield) as yellow oil.

LC-MS (ESI): *m*/*z* (M+1): 239.1 (Method 2)

### Intermediate 60: N-(4-aminopyridin-2-yl)-3-(dimethylamino)propanamide

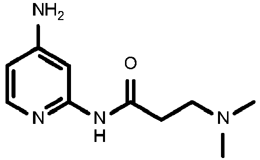

Intermediate 60 was prepared as described for Intermediate 3, starting from Intermediate 59 (144 mg, 0.60 mmol). The crude material was purified by FC on Biotage silica-NH (from 0% to 2% of MeOH in DCM as eluent). Evaporation of opportune fractions provided Intermediate 60 as an orange oil.

LC-MS (ESI): *m*/*z* (M+1): 209.1 (Method 2)

### Intermediate 61: tert-butyl 6-(5-chloro-2-fluorophenyl)-8-({2-[3-(dimethylamino)propanamido]pyridin-4-yl}amino)-2H,3H,4H-pyrido[3,2-b][1,4]oxazine-4-carboxylate

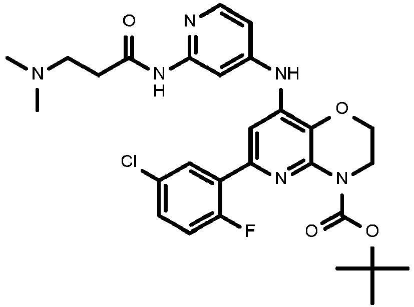

Intermediate 61 was prepared following the procedure described for Intermediate 37, starting from Intermediate 36 (100 mg, 0.22 mmol) and Intermediate 60 (65.7 mg, 0.32 mmol). The crude material was purified by FC on Biotage silica-NH gel (from 0% to 100% of EtOAc in c-Hex as eluent). Evaporation of opportune fractions provided title compound (58 mg, 0.10 mmol, 45% yield) as a yellow oil.

LC-MS (ESI): m/z (M+1): 571.3 (Method 2)

### Intermediate 62: 2-(trimethylsilyl)ethyl carbamate

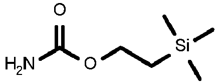

1,1'-Carbonyldiimidazole (9.9 g, 60.9 mmol) was added to a stirred suspension of 2-(trimethylsilyl)ethanol (6.0 g, 50.7 mmol) in dry toluene (50 mL). The reaction was stirred at RT for 5 hrs, before adding ammonium hydroxide solution (28% NH₄OH in water, 10 mL). This mixture was vigorously stirred overnight. Phases were separated, the organic phase was washed with brine, then filtered through a phase separator and concentrated under vacuum. The residue was taken up with EtOAc and washed with brine (5x), then filtered through a phase separator and evaporated, affording 2-(trimethylsilyl)ethyl carbamate (7.5 g, 46.5 mmol, 92% yield) as colorless oil, that solidifies upon cooling.

¹H NMR (400 MHz, *Chloroform-d*) δ ppm 4.52 (br. s., 2 H), 4.10 - 4.22 (m, 2 H), 0.93 - 1.07 (m, 2 H), 0.05 (s, 9 H).

### Intermediate 63: tert-butyl 6-(5-chloro-2-fluorophenyl)-8-({[2-(trimethylsilyl)ethoxy]carbonyl}amino)-2H,3H,4H-pyrido[3,2-b][1,4]oxazine-4-carboxylate

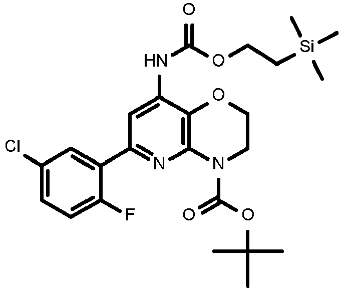

In a suitable vial, a mixture of Intermediate 36 (0.70 g, 1.58 mmol), Xantphos (0.11 g, 0.19 mmol), Cs₂CO₃ (1.03 g, 3.16 mmol), Pd₂(dba)₃ (0.09 g, 0.09 mmol) and Intermediate 62 (0.38 g, 2.37 mmol) was suspended in 1,4-dioxane (10.5 mL). The vial was sealed, evacuated and backfilled with N₂ (three times), then shaken at 100 °C in a PLS overnight. The mixture was diluted with EtOAc, filtered over a pad of Celite^{®}. The filtrate was washed with brine (1x). The organic phase was filtered through a phase separator and concentrated under vacuum. The crude material was purified by FC on Biotage silica gel (from 0% to 20% of EtOAc in c-Hex as eluent), affording title compound (0.70 g, 1.34 mmol, 85% yield) as an ivory solid.

LC-MS (ESI): m/z (M+1): 524.2 (Method 1)

### Intermediate 64: tert-butyl 8-amino-6-(5-chloro-2-fluorophenyl)-2H,3H,4H-pyrido [3,2-b] [1,4] oxazine-4-carboxylate

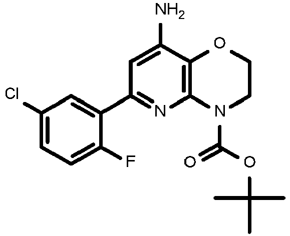

A mixture of Intermediate 63 (0.70 g, 1.34 mmol) and CsF (0.61 g, 4.01 mmol) in DMF (13 mL) was shaken at 45 °C in a PLS for 2 hrs. The mixture was diluted with EtOAc and washed with sat. aqueous NaHCO₃ solution (3x) and brine (1x). The organic phase was filtered through a phase separator and concentrated under vacuum. The crude material was purified by FC on Biotage silica-NH gel (from 0% to 30% of EtOAc in c-Hex as eluent) affording Intermediate 64 (0.49 g, 1.31 mmol, 98% yield) as pale yellow solid.

LC-MS (ESI): m/z (M+1): 380.2 (Method 1)

### Intermediate 65: 7-chloro-3-{[2-(trimethylsilyl)ethoxy]methyl}-3H-imidazo[4,5-b]pyridine

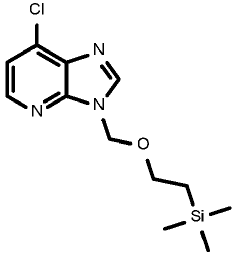

To a solution of 7-chloro-3H-imidazo[4,5-b]pyridine (170 mg, 1.11 mmol) in THF (10 mL), sodium hydride (60 % dispersion in mineral oil, 66.4 mg, 1.66 mmol) was added and the mixture was stirred at RT for 30 min, before adding 2-(trimethylsilyl)ethoxymethyl chloride (0.31 mL, 1.77 mmol). The reaction was stirred at RT overnight, then it was poured into brine and extracted with EtOAc. The organic phase was separated, filtered through a hydrophobic phase separator and evaporated at reduced pressure. The crude material was purified by FC on Biotage silica gel (from 0% to 40% of EtOAc in c-Hex as eluent). Evaporation of opportune fractions provided Intermediate 65 (177 mg, 0.62 mmol, 56% yield) as a colorless oil.

LC-MS (ESI): m/z (M+1): 284.3 (Method 1)

¹H NMR (500 MHz, *Chloroform-d*) δ ppm 8.33 (d, *J*=5.2 Hz, 1 H), 8.25 (s, 1 H), 7.33 (d, *J*=5.2 Hz, 1 H), 5.69 (s, 2 H), 3.59 - 3.65 (m, 2 H), 0.90 - 0.97 (m, 2 H), 0.08 - 0.00 (m, 9 H).

### Intermediate 66: tert-butyl 6-(5-chloro-2-fluorophenyl)-8-[(3-{[2-(trimethylsilyl)ethoxy]methyl}-3H-imidazo[4,5-b]pyridin-7-yl)amino]-2H,3H,4H-pyrido[3,2-b][1,4]oxazine-4-carboxylate

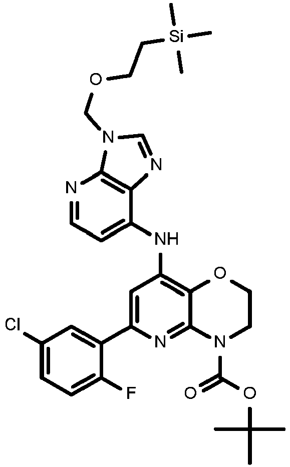

A mixture of Intermediate 64 (100 mg, 0.26 mmol), Pd₂(dba)₃ (14.5 mg, 0.02 mmol), Xantphos (18.3 mg, 0.03 mmol) and Cs₂CO₃ (173 mg, 0.53 mmol) and Intermediate 65 (89.7 mg, 0.32 mmol) was suspended in 1,4-dioxane (2.6 mL). The vial was sealed, evacuated and backfilled with N₂ (three times), then heated at 100 °C overnight. The mixture was diluted with EtOAc and filtered over a pad of Celite^{®}. The filtrate was washed with brine (1x). The organic phase was filtered through a phase separator and concentrated under vacuum. The crude material was purified by FC on Biotage silica-NH gel (from 0% to 50% of EtOAc in c-Hex as eluent), and then on Biotage silica gel (from 10% to 80% of EtOAc in c-Hex as eluent), affording the title compound (148 mg, 0.24 mmol, 91% yield) as white solid.

LC-MS (ESI): m/z (M+1): 627.4 (Method 2)

### Intermediate 67: methyl 4-bromo-1-{[2-(trimethylsilyl)ethoxy]methyl}-1H-pyrrolo[2,3-b]pyridine-3-carboxylate

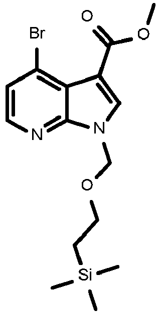

To an ice-cooled suspension of methyl 4-bromo-1H-pyrrolo[2,3-b]pyridine-3-carboxylate (1.5 g, 5.88 mmol) in dry THF (20 mL), sodium hydride (60% dispersion in oil, 0.28 g, 11.76 mmol) was added and the mixture was stirred at 0°C for 30 min, before adding 2-(trimethylsilyl)ethoxymethyl chloride (1.35 mL, 7.64 mmol) dropwise. The reaction mixture was allowed to reach RT and stirred for 3 hrs. Water and EtOAc were added, the product was extracted several times with EtOAc, organic phases were collected, dried and evaporated. The crude material was purified by FC on Biotage silica gel (from 0% to 5% of MeOH in DCM as eluent), affording the title compound (1.05 g, 2.72 mmol, 46% yield) as white wax.

LC-MS (ESI): *m*/*z* (M+1): 385.0 (Method 1)

¹H NMR (400 MHz, *Chloroform-d*) δ ppm 8.15 (d, *J*=5.0 Hz, 1 H), 8.13 (s, 1 H), 7.48 (d, *J*=5.0 Hz, 1 H), 3.92 (s, 3 H), 5.70 (s, 2 H), 3.56 (dd, *J*=8.9, 7.8 Hz, 2 H), 0.88 - 1.01 (m, 2 H), -0.09 - -0.01 (m, 9 H).

### Intermediate 68: tert-butyl 6-(5-chloro-2-fluorophenyl)-8-{[3-(methoxycarbonyl)-1-{[2-(trimethylsilyl)ethoxy]methyl}-1H-pyrrolo[2,3b]pyridin-4-yl]amino)-2H,3H,4H-pyrido[3,2-b][1,4]oxazine-4-carboxylate

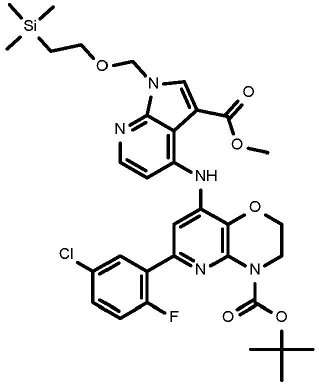

Intermediate 68 was prepared as described for Intermediate 66, starting from Intermediate 64 (100 mg, 0.26 mmol) and Intermediate 67 (122 mg, 0.32 mmol). The crude material was purified by FC on Biotage silica-NH (from 0% to 20% of EtOAc in c-Hex as eluent), affording the title compound (162 mg, 0.24 mmol, 89% yield) as pale yellow solid.

LC-MS (ESI): *m*/*z* (M+1): 684.3 (Method 2)

### Intermediate 69: tert-butyl 8-{[3-({2-[bis(2-hydroxyethyl)amino]ethyl}carbamoyl)pyridin-4-yl]amino}-6-(5-chloro-2-fluorophenyl)-2H,3H,4H-pyrido[3,2-b][1,4]oxazine-4-carboxylate

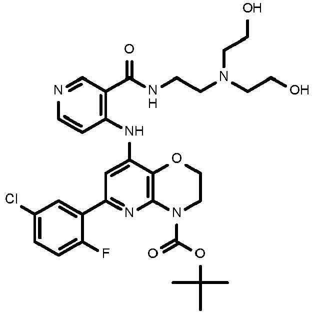

Intermediate 69 was prepared as described for Intermediate 50, starting from Intermediate 40 (150 mg, 0.30 mmol) and 2-[(2-aminoethyl)(2-hydroxyethyl)amino]ethan-1-ol (62 µL, 0.45 mmol). The crude material was purified by FC on Biotage silica-NH gel (from 0% to 5% of MeOH in DCM as eluent). Evaporation of proper fractions provided title compound (58 mg, 0.09 mmol, 31% yield) as a white solid.

LC-MS (ESI): *m*/*z* (M+1): 631.3 (Method 2)

### Intermediate 70: methyl 4-chloro-1-{[2-(trimethylsilyl)ethoxy]methyl}-1H-pyrrolo[2,3-b]pyridine-2-carboxylate

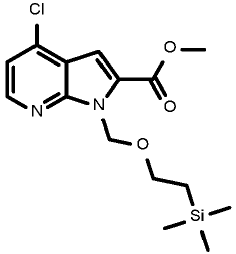

To an ice-cooled suspension of methyl 4-chloro-1H-pyrrolo[2,3-b]pyridine-2-carboxylate (1.0 g, 4.75 mmol) in dry THF (35 mL), sodium hydride (60% dispersion in oil, 0.28 g, 7.12 mmol) was added and the mixture stirred for 30 min before adding 2-(trimethylsilyl)ethoxymethyl chloride (1.09 mL, 6.17 mmol). The reaction mixture was allowed to reach RT and stirred for 3 hrs. The mixture was quenched with sat. aqueous NH₄Cl solution, diluted with EtOAc and washed with brine (1x). The organic phase was dried and concentrated under vacuum and left as solid (yellow), at RT overnight. The day after, the color of the solid was white and UPLC check showed the complete conversion to give the reported regioisomer. The residue was purified by FC on Biotage silica gel (from 0% to 10% of EtOAc in c-Hex), affording Intermediate 70 (820 mg, 2.41 mmol, 51% yield).

LC-MS (ESI): *m*/*z* (M+1): 341.1 (Method 1)

¹H NMR (500 MHz, *Chloroform-d*) δ ppm 8.38 (d, *J*=5.1 Hz, 1 H), 7.40 (s, 1 H), 7.20 (d, *J*=5.1 Hz, 1 H), 6.14 (s, 2 H), 3.97 (s, 3 H), 3.52 - 3.58 (m, 2 H), 0.85 - 0.92 (m, 2 H), -0.11 - -0.05 (m, 9 H).

### Intermediate 71: tert-butyl 6-(5-chloro-2-fluorophenyl)-8-{[2-(methoxycarbonyl)-1-{[2-(trimethylsilyl)ethoxy]methyl}-1H-pyrrolo[2,3-b]pyridin-4-yl]amino)-2H,3H,4H-pyrido[3,2-b][1,4]oxazine-4-carboxylate

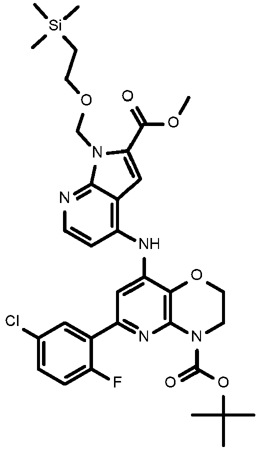

Intermediate 71 was prepared as described for Intermediate 66, starting from Intermediate 64 (70 mg, 0.18 mmol) and Intermediate 70 (75.4 mg, 0.22 mmol). The crude material was purified by FC on Biotage silica-NH gel (from 10% to 30% of EtOAc in c-Hex as eluent). The product was further purified on Biotage silica gel (from 10% to 60% of EtOAc in c-Hex), affording the title compound (110 mg, 0.0.16 mmol, 87% yield) as colorless oil.

LC-MS (ESI): *m*/*z* (M+1): 684.4 (Method 2)

### Intermediate 72: tert-butyl N-{3-[3-(dimethylamino)propanamido]pyridin-4-yl}carbamate

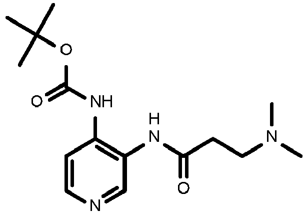

To a solution of *tert-*butyl N-(3-aminopyridin-4-yl)carbamate (250 mg, 1.19 mmol) and 3-(dimethylamino)propanoic acid (210 mg, 1.79 mmol) in dry DMF (3 mL), DIPEA (0.62 mL, 3.58 mmol) and T3P^{®} 50% solution in DMF (1.07 mL, 1.79 mmol) were subsequently added. The reaction was shaken at RT for 1.5 hrs. The mixture was concentrated at reduced pressure and the crude material was purified by FC on Biotage silica-NH gel (from 0% to 5% of MeOH in DCM as eluent). Evaporation of proper fractions provided Intermediate 72 (396 mg, recovery assumed quantitative) as a yellow oil.

LC-MS (ESI): *m*/*z* (M+1): 309.1 (Method 2)

### Intermediate 73: N-(4-aminopyridin-3-yl)-3-(dimethylamino)propanamide

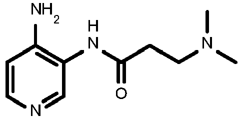

Intermediate 72 (373 mg, 1.21 mmol) was dissolved in a mixture of DCM (10 mL) and TFA (2.8 mL, 36.29 mmol). The solution was shaken at RT overnight. Volatiles were removed at reduced pressure and the residue was dissolved in MeOH, charged on a SCX cartridge (5g), washed with MeOH and eluted with 2 N ammonia in MeOH. Basic fractions were evaporated to provide N-(4-aminopyridin-3-yl)-3-(dimethylamino)propanamide (301 mg, recovery assumed quantitative) as a yellow oil. The material was used in the next step without further purification.

LC-MS (ESI): *m*/*z* (M+1): 209.1 (Method 2)

### Intermediate 74: tert-butyl 6-(5-chloro-2-fluorophenyl)-8-({3-[3-(dimethylamino)propanamido]pyridin-4-yl}amino)-2H,3H,4H-pyrido[3,2-b][1,4]oxazine-4-carboxylate

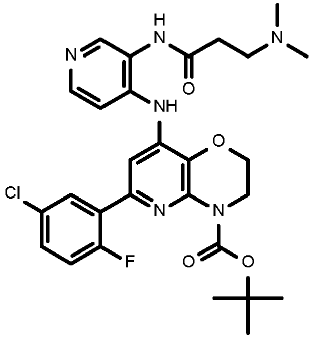

Intermediate 74 was prepared following the procedure described for Intermediate 37, starting from Intermediate 36 (90 mg, 0.20 mmol) and Intermediate 73 (59.1 mg, 0.28 mmol). The crude material was purified by FC on Biotage silica-NH gel (from 0% to 2% of MeOH in DCM as eluent). Evaporation of opportune fractions afforded title compound (61 mg, 0.11 mmol, 53% yield) as a pale yellow solid.

LC-MS (ESI): *m*/*z* (M+1): 571.1 (Method 2)

### Intermediate 75: tert-butyl 6-(6-methylpyridin-2-yl)-8-({[2-(trimethylsilyl)ethoxy]carbonyl}amino)-2H,3H,4H-pyrido[3,2-b][1,4]oxazine-4-carboxylate

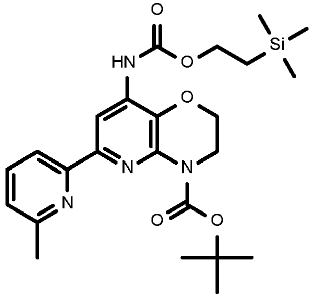

Intermediate 75 was prepared following the procedure described for Intermediate 63, starting from Intermediate 19 (510 mg, 1.26 mmol) and Intermediate 62 (213 mg, 1.32 mmol). The crude material was purified by FC on Biotage silica gel (from 0% to 2% of MeOH in DCM as eluent) and then on Biotage silica-NH gel (from 0% to 35% of EtOAc in c-Hex as eluent) affording Intermediate 75 (500 mg, 1.03 mmol, 82% yield) as ivory solid.

LC-MS (ESI): *m*/*z* (M+1): 487.2 (Method 2)

### Intermediate 76: tert-butyl 8-amino-6-(6-methylpyridin-2-yl)-2H,3H,4H-pyrido[3,2-b][1,4]oxazine-4-carboxylate

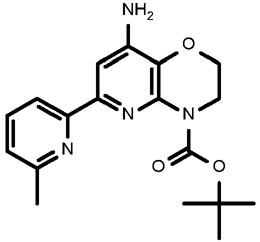

Intermediate 76 was prepared following the procedure described for Intermediate 64, starting from: Intermediate 75 (500 mg, 1.03 mmol). Title compound (359 mg, recovery assumed quantitative) was obtained as white solid.

LC-MS (ESI): *m*/*z* (M+1): 343.1 (Method 2)

### Intermediate 77: tert-butyl 6-(6-methylpyridin-2-yl)-8-[(3-{[2-(trimethylsilyl)ethoxy] methyl}-3H -imidazo [4,5-b] pyridin-7 -yl)amino]-2H,3H,4H-pyrido[3,2-b][1,4]oxazine-4-carboxylate

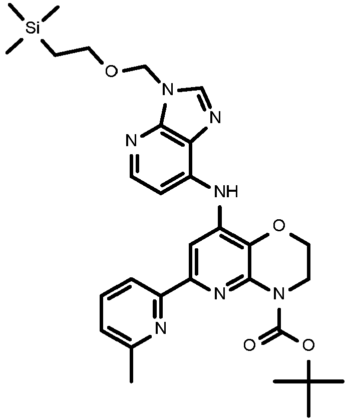

A mixture of Intermediate 76 (70 mg, 0.20 mmol), Pd₂(dba)₃ (11.23 mg, 0.01 mmol), Xantphos (14.2 mg, 0.02 mmol), Cs₂CO₃ (134.05 mg, 0.41 mmol) and Intermediate 65 (75.4 mg, 0.27 mmol) was suspended in 1,4-dioxane (1.8 mL). The vial was sealed, evacuated and backfilled with N₂ (three times), then heated at 120 °C for 24 hrs.

The mixture was diluted with EtOAc, filtered over a pad of Celite^{®}. The filtrate was washed with brine (1x). The organic phase was filtered through a phase separator and concentrated under vacuum. The crude material was purified by FC on Biotage silica-NH gel (from 0% to 43% of EtOAc in c-Hex as eluent) and then on Biotage silica gel (from 0% to 3% of MeOH in DCM as eluent), affording the title compound (100 mg, 0.17 mmol, 83% yield) as white solid.

LC-MS (ESI): *m*/*z* (M+1): 590.3 (Method 1)

### Intermediate 78: tert-butyl 6-(6-methylpyridin-2-yl)-8-[(1-{[2-(trimethylsilyl)ethoxy]methyl}-1H-pyrazolo[3,4-b]pyridin-4-yl)amino]-2H,3H,4H-pyrido[3,2-b][1,4]oxazine-4-carboxylate

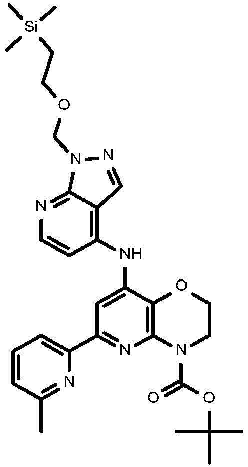

Intermediate 78 was prepared following the procedure described for Intermediate 77, starting from Intermediate 76 (70 mg, 0.20 mmol) and Intermediate 44 (69.6 mg, 0.25 mmol). The crude material was purified by FC on Biotage silica-NH gel (from 0% to 30% of EtOAc in c-Hex as eluent) and then on Biotage silica gel (from 0% to 3% of MeOH in DCM as eluent), affording title compound (100 mg, 0.17 mmol, 83% yield) as white solid.

LC-MS (ESI): *m*/*z* (M+1): 590.3 (Method 1)

### Intermediate 79: lithium 4-({4-[(tert-butoxy)carbonyl]-6-(6-methylpyridin-2-yl)-2H,3H,4H-pyrido[3,2-b][1,4]oxazin-8-yl}amino)pyridine-3-carboxylate

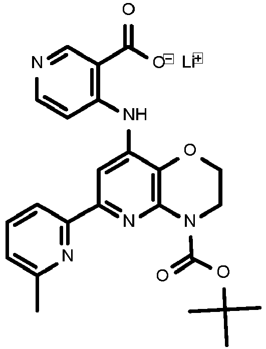

Intermediate 79 was prepared following the procedure described for Intermediate 40, starting from Intermediate 51 (96 mg, 0.41 mmol). Title compound (190 mg, 0.40 mmol, 99% yield) was obtained as pale yellow solid.

LC-MS (ESI): *m*/*z* (M+1): 464.2 (Method 1)

### Intermediate 80: tert-butyl 8-({3-[(1-methylpiperidin-4-yl)carbamoyl]pyridin-4-yl}amino)-6-(6-methylpyridin-2-yl)-2H,3H,4H-pyrido[3,2-b][1,4]oxazine-4-carboxylate

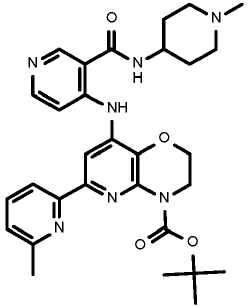

Intermediate 80 was prepared following the procedure described for Intermediate 50, starting from Intermediate 79 (70.0 mg, 0.150 mmol) and 4-amino-1-methylpiperidine (28 µL, 0.220 mmol).The crude material was purified by FC on Biotage silica-NH gel (from 20% to 100% of EtOAc in c-Hex as eluent), affording the title compound (69 mg, 0.12 mmol, 82% yield) as colorless oil.

LC-MS (ESI): *m*/*z* (M+2): 280.7 (Method 1)

### Intermediate 81: tert-butyl N-{2-[methyl(oxetan-3-yl)amino]ethyl}carbamate

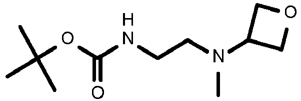

To a solution of *tert*-butyl N-(2-bromoethyl)carbamate (200 mg, 0.89 mmol) in MeCN (7 mL), N-Methyl-3-aminooxetane (0.2 mL, 2.23 mmol) was added and the mixture was stirred overnight at RT. Volatiles were removed at reduced pressure and the crude material was purified by FC on Biotage silica gel (from 0% to 3% of MeOH in DCM as eluent). Evaporation of opportune fractions provided Intermediate 81 (43 mg, 0.19 mmol, 21% yield) as a colorless oil.

¹H NMR (400 MHz, *Chloroform-d*) δ ppm 5.00 (br. s., 1 H), 4.62 - 4.67 (m, 2 H), 4.56 (t, *J*=6.27 Hz, 2 H), 3.58 (quin, *J*=6.55 Hz, 1 H), 3.20 (q, *J*=5.72 Hz, 2 H), 2.32 (t, *J*=6.05 Hz, 2 H), 2.12 (s, 3 H), 1.48 (s, 9 H).

### Intermediate 82: tert-butyl 6-(5-chloro-2-fluorophenyl)-8-{[3-({2-[methyl(oxetan-3-yl)amino]ethyl}carbamoyl)pyridin-4-yl]amino}-2H,3H,4H-pyrido[3,2-b][1,4]oxazine-4-carboxylate

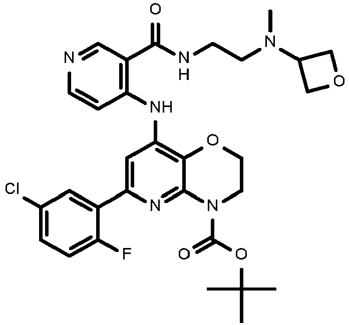

### Step 1

Intermediate 81 (43 mg, 0.19 mmol) was dissolved in a mixture of DCM (1.8 mL) and TFA (0.21 mL, 2.8 mmol) and the solution was shaken at RT for 1 h. Volatiles were removed at reduced pressure and the residue dried at high vacuum to provide N-(2-aminoethyl)-N-methyloxetan-3-amine, 2,2,2-trrifluoroacetate (48 mg, recovery assumed quantitative) as a colorless oil.

### Step 2

Intermediate 82 was prepared as described for Intermediate 50 starting from Intermediate 40 (90 mg, 0.18 mmol) and N-(2-aminoethyl)-N-methyloxetan-3-amine, 2,2,2-trrifluoroacetate (48 mg, 0.19 theoretical mmol). The crude material was purified by FC on Biotage silica-NH gel (from 0% to 100% of EtOAc in c-Hex, followed by 30% of MeOH in EtOAc as eluent). Evaporation of proper fractions provided title compound (53 mg, 0.09 mmol, 49% yield) as a pale yellow solid.

LC-MS (ESI): *m*/*z* (M-tBu+2): 279.2 (Method 1)

### Intermediate 83: methyl 2-[(2-{[(tert-butoxy)carbonyl]amino}ethyl)(methyl)amino]acetate

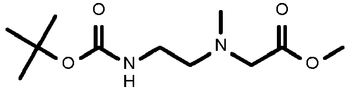

To a solution of *tert*-butyl N-[2-(methylamino)ethyl]carbamate (400 mg, 2.3 mmol) and DIPEA (0.8 mL, 4.59 mmol) in MeCN (15 mL), methyl bromoacetate (0.2 mL, 2.07 mmol) was added and the mixture was stirred at RT overnight. Volatiles were removed at reduced pressure and the crude material was purified by FC on Biotage silica gel (from 0% to 10% of MeOH in DCM as eluent). Evaporation of opportune fractions provided title compound (415 mg, 1.68 mmol, 73.4% yield) as a colorless oil.

¹H NMR (400 MHz, *Chloroform-d*) δ ppm 5.14 (br. s., 1 H), 3.73 (s, 3 H), 3.30 (s, 2 H), 3.21 (q, *J*=5.14 Hz, 2 H), 2.63 (t, *J*=5.94 Hz, 2 H), 2.39 (s, 3 H), 1.46 (s, 9 H).

### Intermediate 84: tert-butyl 6-(5-chloro-2-fluorophenyl)-8-{[3-({2-[(2-methoxy-2-oxoethyl)(methyl)amino]ethyl}carbamoyl)pyridin-4-yl]amino}-2H,3H,4H-pyrido[3,2-b][1,4]oxazine-4-carboxylate

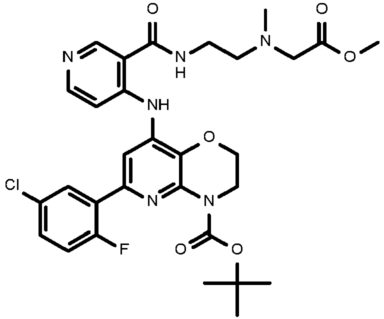

### Step 1

Intermediate 83 (55.0 mg, 0.22 mmol) was dissolved in a mixture of DCM (1.8 mL) and TFA (0.21 mL, 2.8 mmol) and the solution was shaken at RT for 1 h. Volatiles were removed at reduced pressure and the residue dried at high vacuum to provide methyl 2-[(2-aminoethyl)(methyl)amino]acetate, 2,2,2-trrifluoroacetate (60 mg, recovery assumed quantitative) as colorless oil.

### Step 2

Intermediate 84 was prepared as described for Intermediate 50 starting from Intermediate 40 (90 mg, 0.18 mmol) and methyl 2-[(2-aminoethyl)(methyl)amino]acetate, 2,2,2-trrifluoroacetate (58 mg, 0.22 theoretical mmol). The crude material was purified by FC on Biotage silica-NH gel (from 5% to 100% of EtOAc in c-Hex as eluent). Evaporation of proper fractions provided title compound (80 mg, 0.13 mmol, 71% yield) as a yellow solid.

LC-MS (ESI): *m*/*z* (M+1): 629.3 (Method 2)

### Intermediate 85: 4-chloro-1-{[2-(trimethylsilyl)ethoxy]methyl}-1H-pyrrolo[2,3-b]pyridine

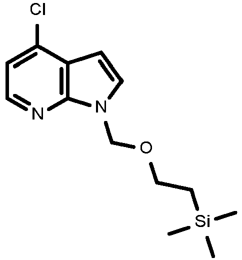

To an iced-cooled suspension of 4-chloro-1H-pyrrolo[2,3-b]pyridine (500 mg, 3.28 mmol) in DMF (12 mL), sodium hydride (60 % dispersion in mineral oil, 157 mg, 3.93 mmol) was added and the mixture was stirred at 0 °C for 30 min, before adding 2-(trimethylsilyl)ethoxymethyl chloride (0.75 mL, 4.26 mmol) dropwise. The reaction mixture was allowed to reach the RT and stirred for 3 hrs. Water and EtOAc were added, the product was extracted several times with EtOAc, organic phases were collected, dried and evaporated. The crude material was purified by FC on Biotage silica gel (from 0% to 6% of EtOAc in c-Hex as eluent), affording Intermediate 85 (600 mg, 2.12 mmol, 65% yield) as oil.

LC-MS (ESI): *m*/*z* (M+1): 283.2 (Method 1)

### Intermediate 86: tert-butyl 6-(6-methylpyridin-2-yl)-8-[(1-{[2-(trimethylsilyl)ethoxy]methyl}-1H-pyrrolo[2,3-b]pyridin-4-yl)amino]-2H,3H,4H-pyrido[3,2-b] [1,4]oxazine-4-carboxylate

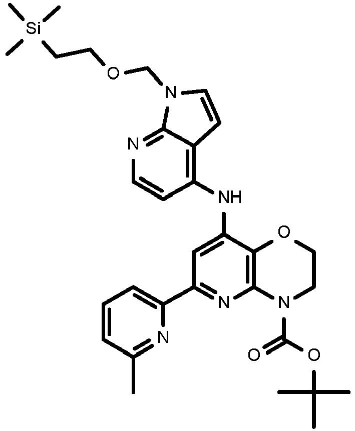

Intermediate 86 was prepared following the procedure described for Intermediate 77, starting from Intermediate 76 (70 mg, 0.20 mmol) and Intermediate 85 (69.6 mg, 0.25 mmol). The crude material was purified by FC on Biotage silica-NH gel (from 0% to 30% of EtOAc in c-Hex as eluent) and then on Biotage silica gel (from 0% to 7% of MeOH in DCM as eluent), affording title compound (115 mg, 0.19 mmol, 96% yield) as white solid.

LC-MS (ESI): *m*/*z* (M+1): 589.3 (Method 2)

### Intermediate 87: tert-butyl 6-(5-chloro-2-fluorophenyl)-8-[(3-{[2-(4-methanesulfonylpiperazin-1-yl)ethyl]carbamoyl}pyridin-4-yl)amino]-2H,3H,4H-pyrido[3,2-b][1,4]oxazine-4-carboxylate

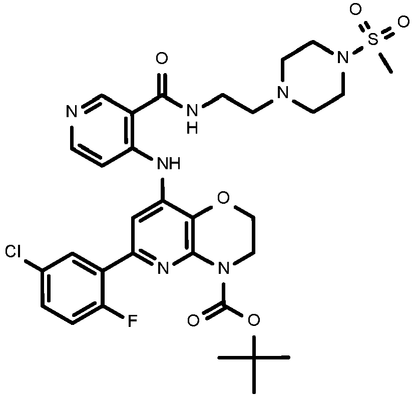

Intermediate 87 was prepared as described for Intermediate 50, starting from Intermediate 40 (80 mg, 0.16 mmol) and 2-(4-methanesulfonylpiperazin-1-yl)ethan-1-amine (49.1 mg, 0.24 mmol) The crude material was purified by FC on Biotage silica-NH gel (from 0% to 1% of MeOH in DCM as eluent) affording the title compound (105 mg, 0.152 mmol, 96% yield) as colorless oil that slowly solidifies.

LC-MS (ESI): *m*/*z* (M-tBu+2): 317.7 (Method 1)

### Intermediate 88: tert-butyl 8-[(3-{[2-(4-acetylpiperazin-1-yl)ethyl]carbamoyl}pyridin-4-yl)amino]-6-(5-chloro-2-fluorophenyl)-2H,3H,4H-pyrido[3,2-b][1,4]oxazine-4-carboxylate

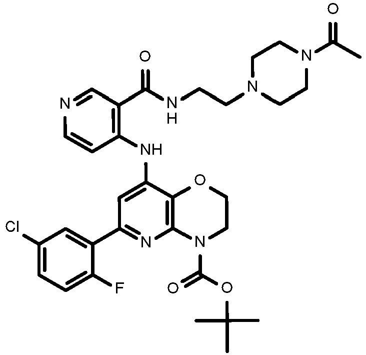

Intermediate 88 was prepared as described for Intermediate 50, starting from Intermediate 40 (70 mg, 0.14 mmol) and 1-[4-(2-aminoethyl)piperazin-1-yl]ethan-1-one (35.5 mg, 0.21 mmol). The crude material was purified by FC on Biotage silica-NH gel (from 0% to 2% of MeOH in DCM as eluent) affording the title compound (74 mg, 0.11 mmol, 82% yield) as colorless oil that slowly solidifies.

LC-MS (ESI): *m*/*z* (M-tBu+2): 299.7 (Method 1)

### Intermediate 89: lithium 4-({4-[(tert-butoxy)carbonyl]-6-(5-chloro-2-fluorophenyl)-2H,3H,4H-pyrido[3,2-b][1,4]oxazin-8-yl}amino)-1-{[2-(trimethylsilyl)ethoxy]methyl}-1H-pyrrolo[2,3-b]pyridine-2-carboxylate

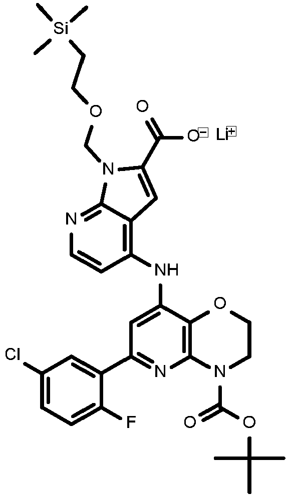

Intermediate 89 was prepared following the procedure described for Intermediate 40, starting from Intermediate 71 (1.0 g, 1.2 mmol). Title compound (0.79 mg, 1.18 mmol, 98% yield) was obtained as pale yellow solid.

LC-MS (ESI): m/z (M+1): 670.3 (Method 1)

### Intermediate 90: tert-butyl 6-(5-chloro-2-fluorophenyl)-8-[(2-{[2-(1-methylpiperidin-4-yl)ethyl]carbamoyl}-1-{[2-(trimethylsilyl)ethoxy]methyl}-

### 1H-pyrrolo[2,3-b]pyridin-4-yl)amino]-2H,3H,4H-pyrido[3,2-b] [1,4]oxazine-4-carboxylate

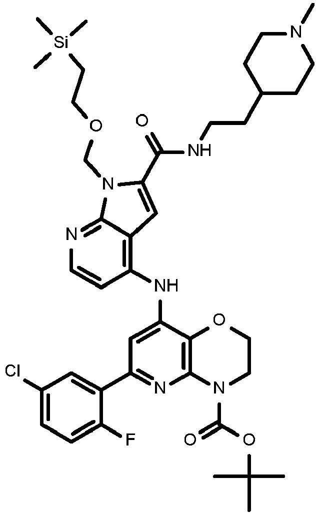

Intermediate 90 was prepared as described for Intermediate 50, starting from Intermediate 89 (120 mg, 0.18 mmol) and 2-(1-methylpiperidin-4-yl)ethan-1-amine (42.3 µL, 0.27 mmol). The crude material was purified by FC on Biotage silica-NH gel (from 10% to 100% of EtOAc in c-Hex as eluent), affording title compound (129 mg, 0.16 mmol, 91 % yield) as white solid.

LC-MS (ESI): *m*/*z* (M+2): 397.8 (Method 1)

### Intermediate 91: tert-butyl 6-(5-chloro-2-fluorophenyl)-8-[(2-{[2-(dimethylamino)ethyl]carbamoyl}-1-{[2-(trimethylsilyl)ethoxy]methyl}-1H-pyrrolo[2,3-b]pyridin-4-yl)amino]-2H,3H,4H-pyrido[3,2-b][1,4]oxazine-4-carboxylate

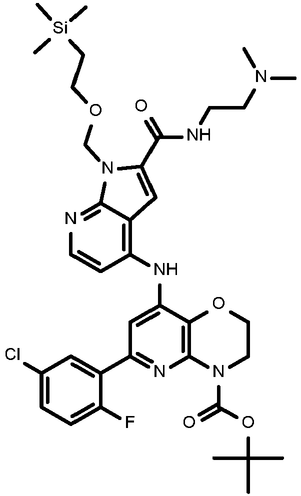

Intermediate 91 was prepared as described for Intermediate 50, starting from Intermediate 89 (120 mg, 0.18 mmol) and N,N-dimethylethylenediamine (29.08 uL, 0.27 mmol). The crude material was purified by FC on Biotage silica-NH gel (from 30% to 100% of EtOAc in c-Hex as eluent) affording the title compound (120 mg, 0.16 mmol, 91% yield) as white solid.

LC-MS (ESI): *m*/*z* (M+2): 370.8 (Method 1)

### INTERMEDIATES FOR COMPARATIVE COMPOUNDS

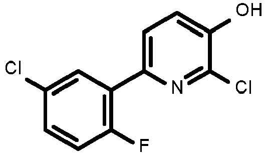

### Intermediate 92: 2-chloro-6-(5-chloro-2-fluorophenyl)pyridin-3-ol

In a suitable vial, charged with 2-chloro-6-iodopyridin-3-ol (500 mg, 1.96 mmol), 5-chloro-2-fluorophenylboronic acid (341 mg, 1.96 mmol), Pd(PPh₃)₄ (113 mg, 0.10 mmol) and NaHCO₃ (333 mg, 3.91 mmol), a mixture of toluene, ethanol and water (16 mL, 6:1:1 ratio) was added. The vessel was sealed, evacuated and backfilled with N₂ (three times), then heated at 90 °C for 6 hrs. The mixture was cooled down and filtered through a Celite^{®} pad, washing with EtOAc. The filtrate was washed with acidified brine (2x), then filtered through a phase separator and evaporated under vacuum. The crude material was purified by FC on Biotage silica gel (c-Hex 100%, then DCM 100% as eluent), to provide the title compound (367 mg, 1.42 mmol, 73% yield) as pale orange solid.

LC-MS (ESI): *m*/*z* (M+1): 258.2 (Method 1)

### Intermediate 93: 2-chloro-6-(5-chloro-2-fluorophenyl)-4-iodopyridin-3-ol

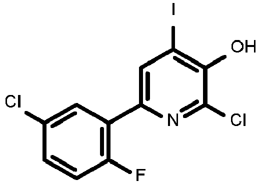

To a solution of Intermediate 92 (1.47 g, 5.68 mmol) and Na₂CO₃ (1.32 g, 12.49 mmol) in water (14.5 mL), iodine (1.44 g, 5.68 mmol) was added. The mixture was stirred at RT for 1 h. Residual iodine was quenched with 10% Na₂S₂O₃ aqueous solution, then the mixture was acidified with 2 N aqueous HCl solution. The resulting precipitate was collected by filtration and washed with cold water, affording Intermediate 93 (2.13 g, 5.55 mmol, 98% yield) as an ivory solid.

LC-MS (ESI): *m*/*z* (M+1): 383.9 (Method 1)

### Intermediate 94: 2-chloro-6-(5-chloro-2-fluorophenyl)-[4,4'-bipyridin]-3-ol

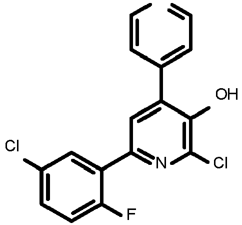

In a suitable vial, Intermediate 93 (1.0 g, 2.6 mmol), 4-pyridinylboronic acid (384 mg, 3.12 mmol). Pd(PPh₃)₄ (150 mg, 0.14 mmol) and NaHCO₃ (442 mg, 5.2 mmol) were dissolved in toluene/ethanol/water mixture (32 mL, 6:1:1 ratio). The vessel was sealed and evacuated and backfilled with N₂ (3x), then vigorously stirred at 90 °C overnight. Water was added and the resulting precipitate was collected by filtration, washed with water and dried under vacuum, affording Intermediate 94 (420 mg, 1.25 mmol, 48% yield), that was used without further purification.

LC-MS (ESI): *m*/*z* (M+1): 335.0 (Method 1)

### Intermediate 95: 2-chloro-6-(5-chloro-2-fluorophenyl)-3-(methoxymethoxy)-4,4'-bipyridine

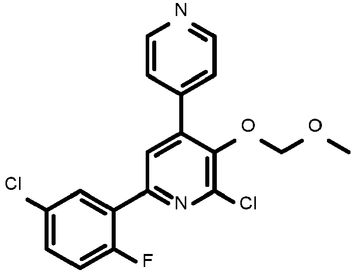

To an ice-cooled suspension of Intermediate 94 (420 mg, 1.25 mmol) in dry DMF (5mL) potassium *tert*-butoxide (352 mg, 3.13 mmol) was added and the mixture was stirred at 0 °C for 15 min. Chloromethyl methyl ether (219 µL, 2.88 mmol) was added dropwise. The mixture was stirred at 0 °C for 30 min, then further potassium *tert*-butoxide (141 mg, 1.25 mmol) and chloromethyl methyl ether (76 µL, 1 mmol) were added. After 30 min, the reaction was quenched with water, diluted with EtOAc and transferred to a separatory funnel. The organic phase was washed with sat. NaHCO₃ (3x) and brine (1x), filtered through a phase separator and evaporated under vacuum. The crude material was purified by FC on Biotage silica gel (from 15% to 45% of EtOAc in c-Hex as eluent) affording the title compound (253 mg, 0.67 mmol, 53% yield).

LC-MS (ESI): *m*/*z* (M+1): 379.0 (Method 1)

### Intermediate 96: 6-(5-chloro-2-fluorophenyl)-N-[(2,4-dimethoxyphenyl)methyl]-3-(methoxymethoxy)-[4,4'-bipyridin]-2-amine

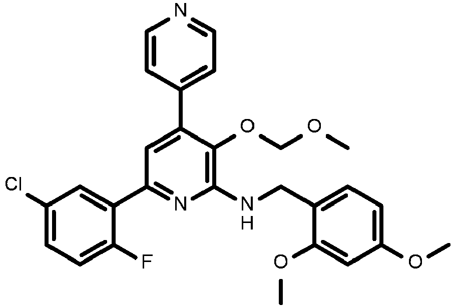

In a suitable vial, a mixture of Intermediate 95 (250 mg, 0.66 mmol), sodium tert-butoxide (79.2 mg, 0.820 mmol), DTBPF (36.9 mg, 0.08 mmol) and Pd₂(dba)₃ (37.91 mg, 0.07 mmol) was suspended in 1,4-dioxane (2.6 mL). The mixture was degassed (vacuum/N₂) then 2,4-dimethoxybenzylamine (198 µL, 1.32 mmol) was added and the mixture heated to 100 °C for 30 min. The mixture was filtered over a pad of Celite^{®}, the cake was washed with EtOAc, then the organic phase was washed with brine (2x), filtered through a phase separator and evaporated under vacuum. The crude material was purified by FC on Biotage silica gel (from 25% to 70% of EtOAc in c-Hex as eluent), affording Intermediate 96 (249 mg, 0.49 mmol, 74% yield) as pale yellow oil.

LC-MS (ESI): *m*/*z* (M+1): 510.2 (Method 1)

### Intermediate 97: 2-amino-6-(5-chloro-2-fluorophenyl)-[4,4'-bipyridin]-3-ol

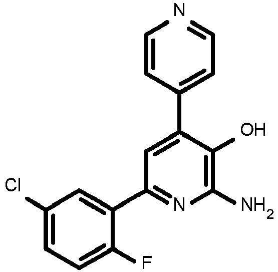

A solution of Intermediate 96 (180 mg, 0.32 mmol) in a DCM/TFA (8 mL, 8:2 ratio) was stirred at RT for 30 min. Toluene (2 mL) was added and the solvents were removed under reduced pressure. The crude material was purified by SCX (5 g, washing with MeOH, and eluting with 1 N ammonia in MeOH), affording the title compound (132 mg, recovery assumed quantitative), that was used for next step without further purification.

LC-MS (ESI): *m*/*z* (M+1): 316.1 (Method 1)

### PREPARATIONS OF EXAMPLES

### Example 1: N-[6-(5-chloro-2-fluorophenyl)-2H,3H,4H-pyrido[3,2-b][1,4]oxazin-8-yl]-pyridine-4-amine

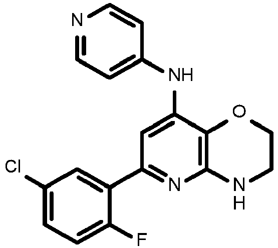

In a suitable vial, a mixture of Intermediate 5 (54 mg, 0.16 mmol), Xantphos (10.9 mg, 0.02 mmol), Pd₂(dba)₃ (8.64 mg, 0.01 mmol) and sodium tert-butoxide (18.9 mg, 0.20mmol) was suspended in 1,4-dioxane (1.25 mL). The mixture was degassed (vacuum/Nz) then pyridin-4-amine (16.3 mg, 0.17 mmol) was added and the reaction heated at 90 °C in a PLS for 30 min. The mixture was diluted with EtOAc and filtered over a pad of Celite^{®}. The filtrate was washed with sat. aqueous NaHCO₃ solution, filtered through a phase separator and concentrated under reduced pressure. The crude material was purified by FC on Biotage silica-NH gel (from 0% to 30% of EtOAc in c-Hex as eluent). Fractions containing the target product were evaporated and further purified by reverse FC on Biotage C18 cartridge (from 5% to 45% of MeCN in water with ammonia-ammonium bicarbonate buffer (pH=10) as eluent). Evaporation of proper fractions provided the title compound (24 mg, 0.07 mmol, 43% yield) as an off-white solid.

LC-MS (ESI): *m*/*z* (M+1): 357.1 (Method 2)

¹H NMR (500 MHz, *DMSO-d₆*) δ ppm 8.56 (s, 1 H), 8.18 - 8.29 (m, 2 H), 7.94 (dd, *J*=6.9, 2.7 Hz, 1 H), 7.38 - 7.50 (m, 1 H), 7.31 (dd, *J*=11.3, 8.8 Hz, 1 H), 7.07 (d, *J*=1.1 Hz, 1 H), 6.98 - 7.03 (m, 2 H), 6.95 (s, 1 H), 4.22 (t, *J*=4.3 Hz, 2 H), 3.41 - 3.53 (m, 2 H).

### Example 2: 4-{[6-(5-chloro-2-fluorophenyl)-2H,3H,4H-pyrido[3,2-b][1,4]oxazin-8-yl]amino}-N-methylpyridine-3-carboxamide

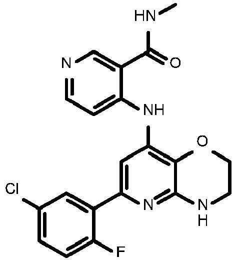

In a suitable vial, Intermediate 6 (30 mg, 0.07 mmol) was dissolved in methylamine, 2 M solution in MeOH (1.45 mL, 2.89 mmol) and the mixture was shaken at 60 °C overnight. Volatiles were removed at reduced pressure and the crude material was purified by reverse FC on Biotage C18 cartridge (from 5% to 40% of MeCN +0.1% HCOOH in water +0.1% HCOOH as eluent). Evaporation of opportune fractions provided title compound (22 mg, 0.05 mmol, 74% yield) as a white solid.

LC-MS (ESI): *m*/*z* (M+1): 414.1 (Method 2)

¹H NMR (400 MHz, *DMSO-d6*) δ ppm 10.38 (s, 1 H), 8.78 (br q, *J*=4.7 Hz, 1 H), 8.70 (s, 1 H), 8.34 (d, *J*=5.9 Hz, 1 H), 7.93 (dd, *J*=6.9, 2.8 Hz, 1 H), 7.44 (ddd, *J*=8.7, 4.1, 3.0 Hz, 1 H), 7.29 - 7.36 (m, 1 H), 7.29 (d, *J*=5.9 Hz, 1 H), 7.16 (s, 1 H), 7.00 (br t, *J*=2.1 Hz, 1 H), 4.26 (t, *J*=4.1 Hz, 2 H), 3.48 (br s, 2 H), 2.80 (d, *J*=4.5 Hz, 3 H).

### Example 3: N4-[6-(5-chloro-2-fluorophenyl)-2H,3H,4H-pyrido[3,2-b][1,4]oxazin-8-yl]pyridine-2,4-diamine

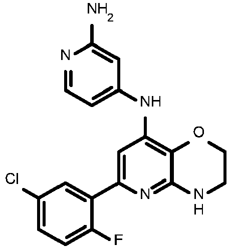

To a mixture of Intermediate 7 (19 mg, 0.05 mmol) and nickel(II) chloride hexahydrate (2.27 mg, 0.01 mmol) in THF/MeOH (0.5 mL, 1:1 ratio) cooled to 0 °C, sodium borohydride (3.58 mg, 0.09 mmol) was slowly added and the mixture stirred for 30 minutes at 0 °C. Few drops of 2 N HCl were added at 0 °C, then the mixture was allowed to reach RT and stirred for 30 minutes. The mixture was partitioned between EtOAc and brine (adjusting pH to ~ 8-9 with 2 N NaOH) then the organic phase was separated, dried over Na₂SO₄, filtered and concentrated at reduced pressure. The crude material was purified by reverse FC on Biotage C18 cartridge (from 5% to 45% of MeCN in water with ammonia-ammonium bicarbonate buffer (pH=10) as eluent). Evaporation of opportune fractions provided the title ompound (9.5 mg, 0.03 mmol, 54% yield) as an off-white solid.

LC-MS (ESI): *m*/*z* (M+1): 372.1 (Method 2)

¹H NMR (500 MHz, *DMSO-d₆*) δ ppm 8.05 (s, 1 H), 7.93 (dd, *J*=6.9, 2.7 Hz, 1 H), 7.65 (d, *J*=5.8 Hz, 1 H), 7.38 - 7.44 (m, 1 H), 7.31 (dd, *J*=11.3, 8.8 Hz, 1 H), 7.06 (d, *J*=0.8 Hz, 1 H), 6.83 (s, 1 H), 6.29 (dd, *J*=5.8, 1.9 Hz, 1 H), 6.12 (d, *J*=1.9 Hz, 1 H), 5.60 (s, 2 H), 4.20 (t, *J*=4.3 Hz, 2 H), 3.46 (br s, 2 H).

### Example 4: N-[6-(4-methyl-1,3-thiazol-2-yl)-2H,3H,4H-pyrido[3,2-b][1,4]oxazin-8-yl]pyridin-4-amine

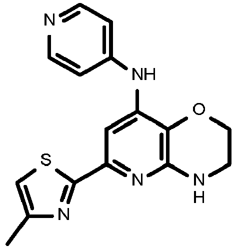

4 N HCl solution in 1,4-dioxane (1.0 mL, 32.91 mmol) was added to a stirred solution of Intermediate 13 (25 mg, 0.06 mmol) in MeOH (1 mL). The reaction was stirred at RT for 3 hrs, then volatiles were removed under vacuum. The residue material was taken up with water and washed with EtOAc that was discarded. Then the aqueous phase was basified adding ammonium hydroxide solution and extracted with DCM. The combined organic layers were filtered through a phase separator and concentrated under vacuum, affording the title compound (16 mg, 0.05 mmol, 84% yield).

LC-MS (ESI): *m*/*z* (M+1): 326.1 (Method 2)

¹H NMR (500 MHz, *DMSO-d₆*) δ ppm 8.55 (s, 1 H), 8.21 - 8.35 (m, 2 H), 7.33 (s, 1 H), 7.22 (d, *J*=0.8 Hz, 1 H), 7.00 - 7.06 (m, 2 H), 6.98 (s, 1 H), 4.21 (t, *J*=4.4 Hz, 2 H), 3.41 - 3.51 (m, 2 H), 2.36 (s, 3 H).

### Example 5: N-[6-(6-methylpyridin-2-yl)-2H,3H,4H-pyrido[3,2-b][1,4]oxazin-8-yl]pyridin-4-amine

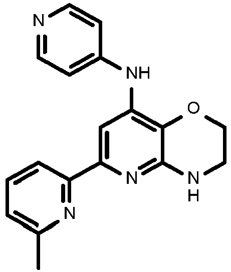

Example 5 (15 mg, 0.05 mmol, 66% yield) was prepared as described for Example 4, starting from Intermediate 20 (30 mg, 0.07 mmol).

LC-MS (ESI): *m*/*z* (M+1): 320.1 (Method 2)

¹H NMR (400 MHz, *DMSO-d₆*) δ ppm 8.53 (s, 1 H), 8.16 - 8.35 (m, 2 H), 7.96 (d, *J*=7.8 Hz, 1 H), 7.72 (t, *J*=7.7 Hz, 1 H), 7.65 (s, 1 H), 7.17 (d, *J*=7.5 Hz, 1 H), 6.88 - 7.05 (m, 2 H), 6.76 (s, 1 H), 4.21 (t, *J*=4.2 Hz, 2 H), 3.47 (br d, *J*=2.3 Hz, 2 H), 2.47 (s, 3 H).

### Example 6: 4-{[6-(5-chloro-2-fluorophenyl)-2H,3H,4H-pyrido[3,2-b][1,4]oxazin-8-yl]amino}-N-methylpyridine-2-carboxamide

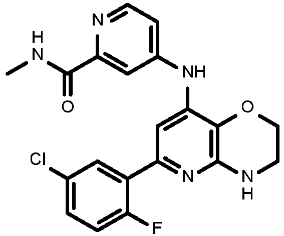

In a suitable vial,.a mixture of Intermediate 5 (70 mg, 0.20 mmol), Pd₂(dba)₃ (11.2 mg, 0.01 mmol), sodium *tert*-butoxide (24.5 mg, .0.26 mmol) and Xantphos (14.2 mg, 0.02 mmol) was suspended in 1,4-dioxane (1.8 mL). The mixture was degassed (vacuum/Nz) then 4-amino-N-methylpyridine-2-carboxamide (Intermediate 21, 40 mg, 0.24 mmol) was added. The vial was sealed and submitted to MW cycle at 150 °C for 30 min. Then further. Pd₂(dba)₃ (11.2 mg, 0.01 mmol), sodium *tert*-butoxide (24.5 mg, .0.26 mmol), Xantphos (14.2 mg, 0.02 mmol) and Intermediate 21 (40 mg, 0.24 mmol) were added the vial submitted again to MW cycle at 150 °C for 30 min. The reaction mixture was diluted with EtOAc and filtered through a pad of Celite^{®}. The organic phase was washed with brine, filtered through a phase separator and concentrated under vacuum. The crude material was purified by FC on Biotage silica-NH gel (from 0% to 3% of MeOH in DCM as eluent). Fractions containing the target product were further purified by reverse FC on Biotage C18 cartridge (from 5% to 60% of MeCN in water with ammonia-ammonium bicarbonate buffer (pH=10) as eluent). Evaporation of opportune fractions provided the title compound (17 mg, 0.04 mmol, 20% yield) as a white solid.

LC-MS (ESI): *m*/*z* (M+1): 414.2 (Method 2)

¹H NMR (400 MHz, *DMSO-d₆*) δ ppm 8.87 (s, 1 H), 8.63 (q, *J*=4.6 Hz, 1 H), 8.26 (d, *J*=5.6 Hz, 1 H), 7.95 (dd, *J*=6.9, 2.8 Hz, 1 H), 7.64 (d, *J*=2.3 Hz, 1 H), 7.40 - 7.46 (m, 1 H), 7.32 (dd, *J*=11.1, 8.8 Hz, 1 H), 7.07 (dd, *J*=5.6, 2.4 Hz, 1 H), 7.05 (d, *J*=1.3 Hz, 1 H), 7.03 (br t, *J*=2.4 Hz, 1 H), 4.20 (t, *J*=4.3 Hz, 2 H), 3.48 (br q, *J*=2.3 Hz, 2 H), 2.79 (d, *J*=4.9 Hz, 3 H).

### Example 7: N4-[6-(5-chloro-2-fluorophenyl)-2H,3H,4H-pyrido[3,2-b][1,4]oxazin-8-yl]pyridine-3,4-diamine

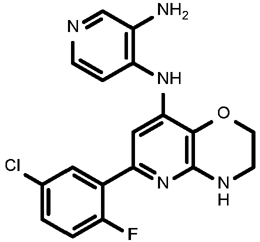

Example 7 was prepared following a similar procedure as described for Example 3, starting from Intermediate 22 (23 mg, 0.06 mmol). The crude material was purified by FC on Biotage silica-NH gel (from 0% to 3% of MeOH in DCM as eluent). Evaporation of proper fractions provided title compound (10 mg, 0.03 mmol, 47% yield) as an off-white solid.

LC-MS (ESI): *m*/*z* (M+1): 372.1 (Method 2)

¹H NMR (400 MHz, *DMSO-d₆*) δ ppm 7.96 (s, 1 H), 7.92 (dd, *J*=6.9, 2.7 Hz, 1 H), 7.70 (d, *J*=5.3 Hz, 1 H), 7.36 - 7.43 (m, 1 H), 7.27 (dd, *J*=11.2, 8.8 Hz, 1 H), 7.13 (s, 1 H), 6.91 (d, *J*=5.3 Hz, 1 H), 6.83 (d, *J*=1.3 Hz, 1 H), 6.80 (s, 1 H), 5.04 (s, 2 H), 4.22 (t, *J*=4.3 Hz, 2 H), 3.47 (br d, *J*=2.6 Hz, 2 H).

### Example 8: N-[6-(2-fluoro-5-methylphenyl)-2H,3H,4H-pyrido[3,2-b][1,4]oxazin-8-yl]pyridin-4-amine

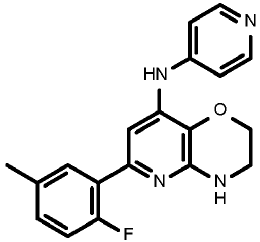

Example 8 (26 mg, 0.077 mmol, 84% yield) was prepared as described for Example 4, starting from Intermediate 24 (40 mg, 0.09 mmol).

LC-MS (ESI): *m*/*z* (M+1): 337.1 (Method 2)

¹H NMR (400 MHz, *DMSO-d₆*) δ 8.51 (s, 1 H), 8.15 - 8.31 (m, 2 H), 7.66 (dd, *J*=7.9, 2.2 Hz, 1 H), 7.05 - 7.20 (m, 2 H), 6.94 - 7.02 (m, 3 H), 6.83 (s, 1 H), 4.20 (t, *J*=4.2 Hz, 2 H), 3.37 - 3.57 (m, 2 H), 2.31 (s, 3 H).

### Example 9: 4-{[6-(5-chloro-2-fluorophenyl)-2H,3H,4H-pyrido[3,2-b][1,4]oxazin-8-yl]amino}-N-[2-(morpholin-4-yl)ethyl]pyridine-3-carboxamide

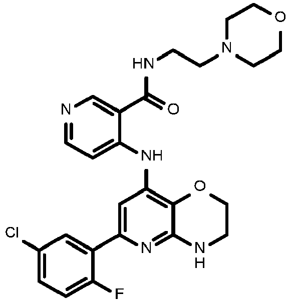

To a suspension of Intermediate 25 (50 mg, 0.12 mmol) in dry DMF (2 mL), TEA (0.05 mL, 0.37 mmol) and HATU (56.9 mg, 0.15 mmol) were added and the mixture stirred at RT for 15 min. 2-(morpholin-4-yl)ethan-1-amine (0.02 mL, 0.19 mmol) was added and the reaction was stirred for 1.5 hrs. The mixture was diluted with EtOAc and washed with sat. aqueous NaHCO₃ solution (3x). The organic phase was separated, filtered through a phase separator and concentrated under reduced pressure. The crude material was purified by reverse FC on Biotage C18 cartridge (from 5% to 60% of MeCN in water with ammonia-ammonium bicarbonate buffer (pH=10) as eluent). Fractions containing the target product were concentrated and further purified by FC on Biotage silica gel (from 0% to 10% of MeOH in DCM as eluent). Evaporation of opportune fractions provided title compound (21 mg, 0.04 mmol, 33% yield) as a pale yellow solid.

LC-MS (ESI): *m*/*z* (M+1): 513.2 (Method 2)

¹H NMR (500 MHz, *DMSO-d₆*) δ ppm 10.25 (s, 1 H), 8.75 (t, *J*=5.6 Hz, 1 H), 8.71 (s, 1 H), 8.34 (d, *J*=5.8 Hz, 1 H), 7.93 (dd, *J*=6.9, 2.7 Hz, 1 H), 7.41 - 7.47 (m, 1 H), 7.33 (dd, *J*=11.3, 8.8 Hz, 1 H), 7.28 (d, *J*=5.8 Hz, 1 H), 7.15 (s, 1 H), 7.01 (s, 1 H), 4.25 (t, *J*=4.3 Hz, 2 H), 3.56 (t, *J*=4.5 Hz, 4 H), 3.47 (br s, 2 H), 3.40 (q, *J*=6.6 Hz, 2 H), 2.46 - 2.57 (m, 2 H), 2.42 (br s, 4 H).

### Example 10: 4-{[6-(5-chloro-2-fluorophenyl)-2H,3H,4H-pyrido[3,2-b][1,4]oxazin-8-yl]amino}-N-[2-(dimethylamino)ethyl]pyridine-3-carboxamide

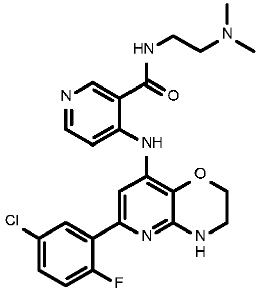

Example 10 was prepared as described for Example 9, starting from Intermediate 25 (50 mg, 0.12 mmol) and (2-aminoethyl)dimethylamine (37.8 mg, 0.37mmol). After purification by reverse FC on Biotage C18 cartridge (from 5% to 90% of MeCN in water with ammonia-ammonium bicarbonate buffer (pH=10) as eluent), title compound (15.4 mg, 0.03 mmol, 26% yield) was obtained as pale orange solid.

LC-MS (ESI): *m*/*z* (M+1): 471.2 (Method 2)

¹H NMR (400 MHz, *DMSO-d₆*) δ ppm 10.29 (s, 1 H), 8.73 (br t, *J*=5.7 Hz, 1 H), 8.71 (s, 1 H), 8.33 (d, *J*=5.7 Hz, 1 H), 7.94 (dd, *J*=6.9, 2.7 Hz, 1 H), 7.44 (dt, *J*=8.3, 3.6 Hz, 1 H), 7.32 (dd, *J*=11.1, 8.9 Hz, 1 H), 7.27 (d, *J*=5.9 Hz, 1 H), 7.15 (s, 1 H), 7.01 (s, 1 H), 4.25 (t, *J*=3.9 Hz, 2 H), 3.42 - 3.56 (m, 2 H), 3.34 - 3.41 (m, 2 H), 2.42 (t, *J*=6.8 Hz, 2 H), 2.18 (s, 6 H).

### Example 11: N-[6-(2-fluorophenyl)-2H,3H,4H-pyrido[3,2-b][1,4]oxazin-8-yl]pyridin-4-amine

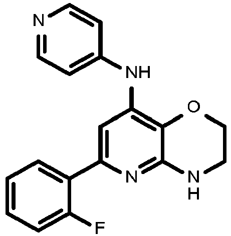

Example 11 (24 mg, 0.07 mmol, 79% yield) was prepared as described for Example 4, starting *tert*-butyl 6-(2-fluorophenyl)-8-[(pyridin-4-yl)amino]-2H,3H,4H-pyrido[3,2-b][1,4]oxazine-4-carboxylate (Intermediate 26, 40 mg, 0.09 mmol).

LC-MS (ESI): *m*/*z* (M+1): 323.1 (Method 2)

¹H NMR (500 MHz, *DMSO-d₆*) δ ppm 8.53 (s, 1 H), 8.23 (d, *J*=6.0 Hz, 2 H), 7.86 (td, *J*=8.0, 1.8 Hz, 1 H), 7.33 - 7.41 (m, 1 H), 7.17 - 7.29 (m, 2 H), 6.96 - 7.05 (m, 3 H), 6.82 (s, 1 H), 4.21 (t, *J*=4.4 Hz, 2 H), 3.42 - 3.52 (m, 2 H).

### Example 12: 4-{[6-(5-chloro-2-fluorophenyl)-2H,3H,4H-pyrido[3,2-b][1,4]oxazin-8-yl]amino}-N-[2-(piperazin-1-yl)ethyl]pyridine-3-carboxamide

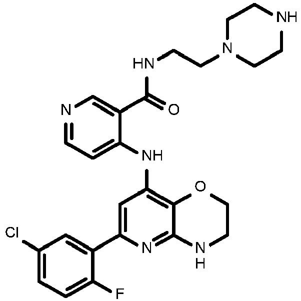

To a solution of Intermediate 27 (22 mg, 0.04 mmol) in DCM (0.4 mL), TFA (0.11 mL, 1.44 mmol) was added and the reaction was stirred at RT for 1 h. The mixture was concentrated under reduced pressure and the crude material was purified by FC on Biotage silica-NH gel (from 0% to 10% of MeOH in DCM as eluent). Evaporation of opportune fraction provided title compound (13 mg, 0.03 mmol, 71% yield) as an off-white solid.

LC-MS (ESI): *m*/*z* (M+1): 512.2 (Method 2)

¹H NMR (400 MHz, *DMSO-d₆*) δ ppm 10.22 - 10.33 (m, 1 H), 8.64 - 8.78 (m, 2 H), 8.34 (d, *J*=5.9 Hz, 1 H), 7.94 (dd, *J*=6.8, 2.6 Hz, 1 H), 7.44 (ddd, *J*=8.7, 4.1, 2.9 Hz, 1 H), 7.32 (dd, *J*=11.0, 8.8 Hz, 1 H), 7.28 (d, *J*=5.9 Hz, 1 H), 7.15 (s, 1 H), 7.01 (s, 1 H), 4.25 (br t, *J*=3.9 Hz, 2 H), 3.47 (br s, 2 H), 3.35 - 3.42 (m, 2 H), 2.69 (t, *J*=4.7 Hz, 4 H), 2.45 (t, *J*=6.9 Hz, 2 H), 2.36 (br s, 4 H).

### Example 13: N-[6-(6-chloropyridin-2-yl)-2H,3H,4H-pyrido[3,2-b][1,4]oxazin-8-yl]pyridin-4-amine

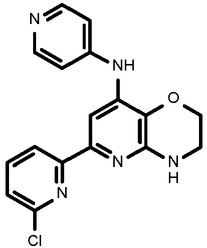

Example 13 (20 mg, 0.06 mmol, 29% yield) was prepared as described for Example 4, starting Intermediate 28 (90 mg, 0.20 mmol).

LC-MS (ESI): *m*/*z* (M+1): 340.0 (Method 2)

¹H NMR (400 MHz, *DMSO-d₆*) δ ppm 8.58 (s, 1 H), 8.23 - 8.30 (m, 2 H), 8.12 (d, *J*=7.2 Hz, 1 H), 7.92 (t, *J*=7.8 Hz, 1 H), 7.54 (s, 1 H), 7.43 (d, *J*=7.9 Hz, 1 H), 6.95 - 7.00 (m, 2 H), 6.85 - 6.91 (m, 1 H), 4.22 (t, *J*=4.2 Hz, 2 H), 3.44 - 3.52 (m, 2 H).

### Example 14: N-[6-(2,5-difluorophenyl)-2H,3H,4H-pyrido[3,2-b][1,4]oxazin-8-yl]pyridin-4-amine

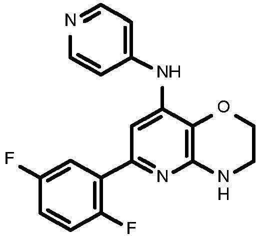

Example 14 (30 mg, 0.09 mmol, 65% yield) was prepared as described for Example 4, starting from Intermediate 29 (60 mg, 0.14 mmol).

LC-MS (ESI): *m*/*z* (M+1): 341.1 (Method 2)

¹H NMR (400 MHz, *DMSO-d₆*) δ ppm 8.55 (s, 1 H), 8.24 (d, *J*=6.2 Hz, 2 H), 7.67 (ddd, *J*=9.8, 6.3, 3.3 Hz, 1 H), 7.26 - 7.35 (m, 1 H), 7.17 - 7.25 (m, 1 H), 7.08 (d, *J*=1.0 Hz, 1 H), 7.00 (d, *J*=6.4 Hz, 2 H), 6.89 (br t, *J*=2.5 Hz, 1 H), 4.22 (t, *J*=4.2 Hz, 2 H), 3.47 (br q, *J*=2.5 Hz, 2 H).

### Example 15: N-[6-(3-chlorophenyl)-2H,3H,4H-pyrido[3,2-b][1,4]oxazin-8-yl]pyridin-4-amine

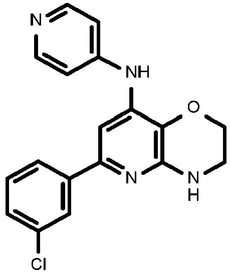

Example 15 was prepared as described for Example 4 starting from Intermediate 30 (50 mg, 0.110mmol).The crude material was purified by FC on Biotage silica-NH gel (from 0% to 2% of MeOH in DCM as eluent). Evaporation of opportune fractions provided the title compound (30 mg, 0.09 mmol, 78% yield).

LC-MS (ESI): *m*/*z* (M+1): 339.1 (Method 2)

¹H NMR (400 MHz, *DMSO-d₆*) δ ppm 8.48 (s, 1 H), 8.24 (d, *J*=6.4 Hz, 2 H), 7.95 (t, *J*=1.7 Hz, 1 H), 7.81 (d, *J*=7.7 Hz, 1 H), 7.42 (t, *J*=7.9 Hz, 1 H), 7.35 -7.39 (m, 1 H), 7.09 (s, 1 H), 7.02 (d, *J*=6.2 Hz, 2 H), 6.86 (br t, *J*=2.5 Hz, 1 H), 4.20 (t, *J*=4.2 Hz, 2 H), 3.47 (br q, *J*=2.5 Hz, 2 H).

### Example 16: lithium 4-{[6-(5-chloro-2-fluorophenyl)-2H,3H,4H-pyrido[3,2-b][1,4]oxazin-8-yl]amino}pyridine-3-carboxylate

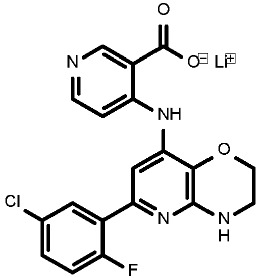

Title compound was also described as Intermediate 25. Therefore, for detailed information about the synthesis of Example 16, see preparation of Intermediate 25.

¹H NMR (400 MHz, *DMSO-d₆*) δ ppm 12.68 (br s, 1 H), 8.81 (s, 1 H), 8.13 (d, *J*=5.7 Hz, 1 H), 7.95 (dd, *J*=6.9, 2.7 Hz, 1 H), 7.41 (ddd, *J*=8.8, 4.2, 3.1 Hz, 1 H), 7.32 (dd, *J*=11.2, 8.8 Hz, 1 H), 7.20 (d, *J*=0.9 Hz, 1 H), 7.10 (d, *J*=5.9 Hz, 1 H), 6.81 (br s, 1 H), 4.21 (t, *J*=4.2 Hz, 2 H), 3.42 - 3.49 (m, 2 H).

### Example 17: methyl 4-{[6-(5-chloro-2-fluorophenyl)-2H,3H,4H-pyrido[3,2-b][1,4]oxazin-8-yl]amino}pyridine-3-carboxylate

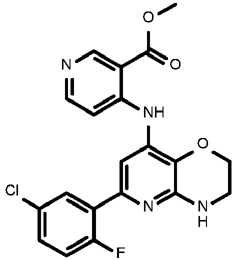

Title compound was also described as Intermediate 6. Therefore, for detailed information about the synthesis of Example 17, see preparation of Intermediate 6.

¹H NMR (400 MHz, *DMSO-d₆*) δ ppm 9.84 (s, 1 H), 8.92 (s, 1 H), 8.40 (d, *J*=5.9 Hz, 1 H), 7.94 (dd, *J*=6.8, 2.9 Hz, 1 H), 7.41 - 7.50 (m, 1 H), 7.33 (dd, *J*=11.1, 8.7 Hz, 1 H), 7.27 (d, *J*=6.2 Hz, 1 H), 7.19 (d, *J*=0.9 Hz, 1 H), 7.11 (s, 1 H), 4.27 (t, *J*=4.3 Hz, 2 H), 3.91 (s, 3 H), 3.43 - 3.59 (m, 2 H).

### Example 18: N-[6-(3-methylphenyl)-2H,3H,4H-pyrido[3,2-b][1,4]oxazin-8-yl]pyridin-4-amine

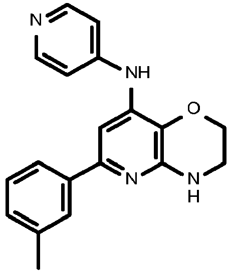

Example 18 was prepared as described for Example 4 starting from Intermediate 31 (40 mg, 0.10 mmol).The crude material was purified by FC on Biotage silica-NH gel (from 0% to 2% of MeOH in DCM as eluent). Evaporation of opportune fractions provided the title compound (20 mg, 0.06 mmol, 66% yield).

LC-MS (ESI): *m*/*z* (M+1): 319.2 (Method 2)

¹H NMR (400 MHz, *DMSO-d₆*) δ ppm 8.45 (s, 1 H), 8.24 (d, *J*=6.4 Hz, 2 H), 7.69 (s, 1 H), 7.63 (d, *J*=7.9 Hz, 1 H), 7.27 (t, *J*=7.6 Hz, 1 H), 7.13 (d, *J*=7.5 Hz, 1 H), 7.01 (s, 1 H), 6.98 - 7.03 (m, 2 H), 6.77 (s, 1 H), 4.19 (t, *J*=4.2 Hz, 2 H), 3.42 - 3.49 (m, 2 H), 2.34 (s, 3 H).

### Example 19: N-[6-(3-chloro-4-fluorophenyl)-2H,3H,4H-pyrido[3,2-b][1,4]oxazin-8-yl]pyridin-4-amine

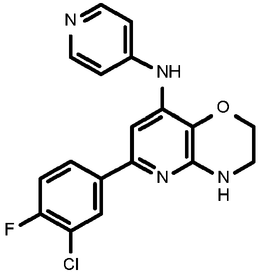

Example 19 was prepared as described for Example 4 starting from Intermediate 32 (50 mg, 0.11 mmol). The crude material was purified by FC on Biotage silica-NH gel (from 0% to 2% of MeOH in DCM as eluent). Evaporation of opportune fractions provided the title compound (30 mg, 0.08 mmol, 77% yield).

LC-MS (ESI): *m*/*z* (M+1): 357.1 (Method 2)

¹H NMR (400 MHz, *DMSO-d₆*) δ ppm 8.47 (s, 1 H), 8.18 - 8.30 (m, 2 H), 8.08 (dd, *J*=7.5, 2.2 Hz, 1 H), 7.86 (ddd, *J*=8.7, 4.8, 2.3 Hz, 1 H), 7.42 (t, *J*=9.0 Hz, 1 H), 7.09 (s, 1 H), 6.97 - 7.07 (m, 2 H), 6.86 (s, 1 H), 4.20 (t, *J*=4.2 Hz, 2 H), 3.40 - 3.54 (m, 2 H).

### Example 20: N-(4-{[6-(5-chloro-2-fluorophenyl)-2H,3H,4H-pyrido[3,2-b][1,4]oxazin-8-yl]amino)pyridin-2-yl)-3-(morpholin-4-yl)propanamide

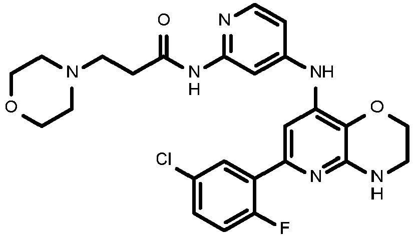

To a solution of Intermediate 37, 35 mg, 0.06 mmol) in DCM (0.8 mL), TFA (0.17 mL, 2.28 mmol) was added and the mixture was stirred at RT overnight. Volatiles were removed under vacuum. The crude material was purified by FC on Biotage silica-NH gel (from 0% to 75% of EtOAc in c-Hex as eluent). Evaporation of opportune fractions provided title compound (8.5 mg, 0.02 mmol, 29% yield) as a white solid.

LC-MS (ESI): *m*/*z* (M+1): 513.2 (Method 2)

¹H NMR (500 MHz, *DMSO-d₆*) δ ppm 10.40 (s, 1 H), 8.60 (s, 1 H), 7.88 - 8.03 (m, 2 H), 7.81 (s, 1 H), 7.35 - 7.51 (m, 1 H), 7.29 (dd, *J*=11.1, 8.8 Hz, 1 H), 7.03 (d, *J*=0.7 Hz, 1 H), 6.94 (s, 1 H), 6.68 (dd, *J*=5.8, 2.2 Hz, 1 H), 4.20 (t, *J*=4.3 Hz, 2 H), 3.56 (t, J=4.5 Hz, 4 H), 3.46 (br s, 2 H), 2.56 - 2.62 (m, 2 H), 2.46 - 2.54 (m, 2 H), 2.36 - 2.44 (m, 4 H).

### Example 21: 4-{[6-(5-chloro-2-fluorophenyl)-2H,3H,4H-pyrido[3,2-b][1,4]oxazin-8-yl]amino}-N-[2-(methylamino)ethyl]pyridine-3-carboxamide

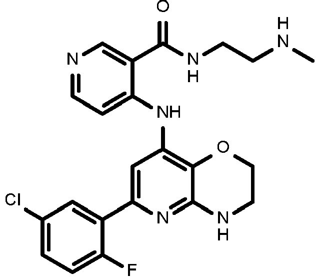

Example 21 was prepared as described for Example 12 starting from Intermediate 38 (33 mg, 0.06 mmol).The crude material was purified by FC on Biotage silica-NH gel (from 0% to 10% of MeOH in DCM as eluent). Evaporation of opportune fractions provided title compound (28 mg, 0.06 mmol, 97% yield) as a pale yellow solid.

LC-MS (ESI): *m*/*z* (M+1): 457.1 (Method 2)

¹H NMR (500 MHz, *DMSO-d₆*) δ ppm 10.32 (br s, 1 H), 8.68 - 8.79 (m, 2 H), 8.34 (d, *J*=5.9 Hz, 1 H), 7.93 (dd, *J*=6.8, 2.8 Hz, 1 H), 7.39 - 7.46 (m, 1 H), 7.32 (dd, *J*=11.0, 8.8 Hz, 1 H), 7.28 (d, *J*=5.9 Hz, 1 H), 7.15 (s, 1 H), 7.01 (s, 1 H), 4.25 (t, *J*=4.3 Hz, 2 H), 3.45 - 3.50 (m, 2 H), 3.33 - 3.38 (m, 2 H), 2.64 (t, *J*=6.4 Hz, 2 H), 2.29 (s, 3 H), 1.90 (br s, 1 H).

### Example 22: 4-{[6-(5-chloro-2-fluorophenyl)-2H,3H,4H-pyrido[3,2-b][1,4]oxazin-8-yl]amino}-N-(propan-2-yl)pyridine-3-carboxamide

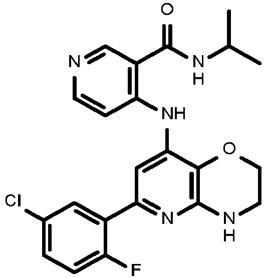

To a solution of Intermediate 41 (29.5 mg, 0.05mmol) in MeOH (1 mL), ~1.25 M HCl solution in MeOH (1.09 mL, 1.36 mmol) was added and the mixture was stirred at 40 °C for 30 minutes. Volatiles were removed under vacuum and the residue was purified by FC on Biotage silica-NH gel (from 0% to 20% of EtOAc in c-Hex as eluent). Evaporation of opportune fraction provided title compound (6.6 mg, 0.01 mmol, 27% yield) as a white solid.

LC-MS (ESI): *m*/*z* (M+2): 221.8 (Method 1)

¹H NMR (400 MHz, *DMSO-d₆*) δ ppm 10.30 (s, 1 H), 8.72 (s, 1 H), 8.59 (d, *J*=7.6 Hz, 1 H), 8.34 (d, *J*=5.8 Hz, 1 H), 7.93 (dd, *J*=6.9, 2.8 Hz, 1 H), 7.40 - 7.50 (m, 1 H), 7.32 (dd, *J*=11.1, 8.8 Hz, 1 H), 7.28 (d, *J*=5.9 Hz, 1 H), 7.15 (s, 1 H), 7.00 (s, 1 H), 4.25 (t, *J*=4.1 Hz, 2 H), 4.12 (ddt, *J*=13.6, 6.7, 6.6, 6.6 Hz, 1 H), 3.47 (br d, *J*=1.6 Hz, 2 H), 1.18 (d, *J*=6.6 Hz, 6 H).

### Example 23: 4-{[6-(5-chloro-2-fluorophenyl)-2H,3H,4H-pyrido[3,2-b][1,4]oxazin-8-yl]amino}-N-cyclopropylpyridine-3-carboxamide

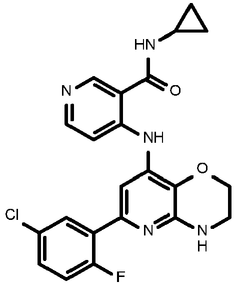

Example 23 was prepared as described for Example 22, starting from Intermediate 42 (18.6 mg, 0. mmol). The crude material was purified by FC on Biotage silica-NH gel (from 0% to 50% of EtOAc in c-Hex as eluent). Evaporation of opportune fraction provided title compound (9 mg, 0.02 mmol, 59% yield) as an off-white solid.

LC-MS (ESI): *m*/*z* (M+1): 440.3 (Method 2)

¹H NMR (500 MHz, *DMSO-d₆*) δ ppm 10.35 (s, 1 H), 8.78 (d, *J*=4.1 Hz, 1 H), 8.67 (s, 1 H), 8.33 (d, *J*=6.0 Hz, 1 H), 7.93 (dd, *J*=6.9, 2.7 Hz, 1 H), 7.39 - 7.47 (m, 1 H), 7.33 (dd, *J*=11.0, 8.8 Hz, 1 H), 7.29 (d, *J*=5.8 Hz, 1 H), 7.16 (s, 1 H), 7.01 (s, 1 H), 4.27 (t, *J*=4.3 Hz, 2 H), 3.48 (br d, *J*=2.5 Hz, 2 H), 2.86 (td, *J*=7.3, 3.8 Hz, 1 H), 0.55 - 0.82 (m, 4 H).

### Example 24: 4-{[6-(5-chloro-2-fluorophenyl)-2H,3H,4H-pyrido[3,2-b][1,4]oxazin-8-yl]amino}-N-(piperidin-4-yl)pyridine-3-carboxamide

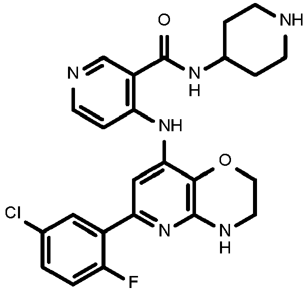

Example 24 was prepared as described for Example 22, starting from Intermediate 43 (37 mg, 0.05 mmol). The crude material was purified by reverse FC on Biotage C18 cartridge (from 5% to 75% of MeCN in water with ammonia-ammonium bicarbonate buffer (pH=10) as eluent). Evaporation of opportune fractions provided title compound (2.5 mg, 0.005 mmol, 9.6 % yield) as a white solid.

LC-MS (ESI): *m*/*z* (M+2): 242.1 (Method 1)

¹H NMR (500 MHz, *DMSO-d₆)* δ ppm 10.28 (s, 1 H), 8.72 (s, 1 H), 8.62 (d, *J*=7.7 Hz, 1 H), 8.34 (d, *J*=5.8 Hz, 1 H), 7.93 (dd, *J*=6.7, 2.9 Hz, 1 H), 7.38 - 7.50 (m, 1 H), 7.33 (dd, *J*=11.3*,* 8.8 Hz, 1 H), 7.29 (d, *J*=6.0 Hz, 1 H), 7.15 (s, 1 H), 7.00 (s, 1 H), 4.25 (t, *J*=4.3 Hz, 2 H), 3.77 - 3.90 (m, 1 H), 3.47 (br s, 2 H), 2.95 (br d, *J*=12.4 Hz, 2 H), 2.44 - 2.56 (m, 2 H), 1.74 (br d, *J*=9.6 Hz, 2 H), 1.41 (qd, *J*=11.8*,* 4.0 Hz, 2 H).

### Example 25: 6-(5-chloro-2-fluorophenyl)-N-{1H-pyrazolo[3,4-b]pyridin-4-yl}-2H,3H,4H -pyrido [3,2-b] [1,4] oxazin-8-amine

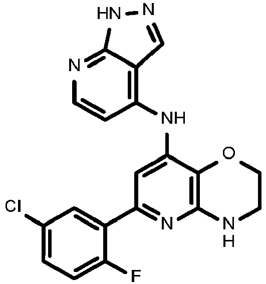

A solution of Intermediate 47 (104 mg, 0.17 mmol) was dissolved in a mixture of DCM (4 mL) and TFA (1.02 mL, 13.27 mmol) was stirred at RT for 48 hrs. Volatiles were removed under vacuum and the crude material was purified by reverse FC on Biotage C18 cartridge (from 5% to 100% of MeCN in water with ammonia-ammonium bicarbonate buffer (pH=10) as eluent). Evaporation of opportune fractions provided the title compound (9 mg, 0.02 mmol, 14% yield) as a white solid.

LC-MS (ESI): *m*/*z* (M+1): 397.2 (Method 2)

¹H NMR (400 MHz, *DMSO-d₆)* δ ppm 13.26 (s, 1 H), 9.00 (s, 1 H), 8.20 (s, 1 H), 8.12 (d, *J*=*5.5* Hz, 1 H), 7.97 (dd, *J*=6.9, 2.8 Hz, 1 H), 7.38 - 7.47 (m, 1 H), 7.31 (dd, *J*=11.2, 8.8 Hz, 1 H), 7.06 - 7.11 (m, 2 H), 6.51 (d, *J*=*5.5* Hz, 1 H), 4.21 (t, *J*=4.1 Hz, 2 H), 3.49 (br q, *J*=4.0 Hz, 2 H).

### Example 26: N-[6-(5-fluoropyridin-2-yl)-2H,3H,4H-pyrido[3,2-b][1,4]oxazin-8-yl]pyridin-4-amine

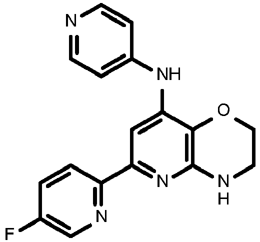

Example 26 was prepared as described for Example 4 starting from Intermediate 48 (70 mg, 0.14 mmol). The crude material was purified by FC on Biotage silica gel (from 0% to 3% of MeOH in DCM as eluent, 0.3% of aqueous ammonia as additive). Evaporation of opportune fractions provided title compound (40 mg, 0.12 mmol, 89% yield).

LC-MS (ESI): *m*/*z* (M+1): 324.1 (Method 2)

¹H NMR (500 MHz, *DMSO-d₆)* δ ppm 8.55 (d, *J*=3.0 Hz, 1 H), 8.52 (s, 1 H), 8.25 - 8.29 (m, 2 H), 8.21 (dd, *J*=8.9, 4.8 Hz, 1 H), 7.78 (td, *J*=8.8, 3.0 Hz, 1 H), 7.61 (s, 1 H), 7.00 - 7.07 (m, 2 H), 6.80 (s, 1 H), 4.22 (t, *J*=4.4 Hz, 2 H), 3.44 - 3.54 (m, 2 H).

### Example 27: 2-(dimethylamino)ethyl 4-{[6-(5-chloro-2-fluorophenyl)-2H,3H,4H-pyrido[3,2-b][1,4]oxazin-8-yl]amino)pyridine-3-carboxylate

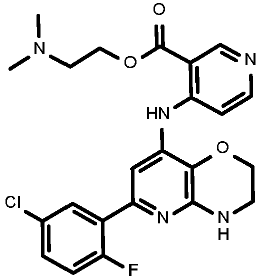

To a solution of Intermediate 49 (61 mg, 0.14 mmol) in dry DMF (3.2 mL), HATU (63.7 mg, 0.17 mmol) and DIPEA (73 µL, 0.42 mmol) were added, followed by 2-dimethylaminoethanol (28 µL, 0.28 mmol). The reaction was stirred at RT for 5 hrs. The mixture was diluted with EtOAc and washed with sat. aqueous NaHCO₃ solution (3x) and brine (1x). The organic phase was filtered through a phase separator and concentrated under vacuum. The crude material was purified by FC on Biotage silica-NH gel (from 20% to 50% of EtOAc in c-Hex as eluent), affording title compound (15 mg, 0.03 mmol, 23% yield) as pale yellow solid.

LC-MS (ESI): *m*/*z* (M+2): 236.8 (Method 1)

¹H NMR (500 MHz, *DMSO-d₆)* δ ppm 9.77 (s, 1 H), 8.90 (s, 1 H), 8.39 (d, *J*=6.0 Hz, 1 H), 7.94 (dd, *J*=6.9, 2.7 Hz, 1 H), 7.45 (ddd, *J*=8.7, 4.0, 2.9 Hz, 1 H), 7.34 (dd, *J*=*11.0,* 8.8 Hz, 1 H), 7.23 (d, *J*=6.0 Hz, 1 H), 7.17 (d, *J*=0.8 Hz, 1 H), 7.12 (s, 1 H), 4.41 (t, *J*=5.8 Hz, 2 H), 4.26 (t, *J*=4.3 Hz, 2 H), 3.48 (br d, *J*=2.5 Hz, 2 H), 2.60 - 2.71 (m, 2 H), 2.22 (s, 6 H).

### Example 28: 4-{[6-(5-chloro-2-fluorophenyl)-2H,3H,4H-pyrido[3,2-b][1,4]oxazin-8-yl]amino}-N-[2-(4-methylpiperazin-1-yl)ethyl]pyridine-3-carboxamide

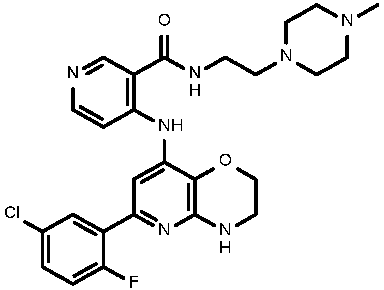

Intermediate 50 (79 mg, 0.13 mmol) was dissolved in a mixture of DCM (1.6 mL) and TFA (0.39 mL, 5.05 mmol) and the solution was shaken at RT overnight. Volatiles were removed under reduced pressure and the residue material was purified by FC on Biotage silica-NH gel (from 0% to 10% of MeOH in DCM as eluent). Evaporation of opportune fractions provided title compound (31 mg, 0.06 mmol, 47% yield) as a white solid.

LC-MS (ESI): *m*/*z* (M+2): 263.7 (Method 1)

¹H NMR (500 MHz, *DMSO-d₆)* δ ppm 10.22 (s, 1 H), 8.72 (br t, *J*=5.5 Hz, 1 H), 8.69 (s, 1 H), 8.34 (d, *J*=6.0 Hz, 1 H), 7.93 (dd, *J*=6.9, 2.7 Hz, 1 H), 7.43 (ddd, *J*=8.8, 4.1, 3.0 Hz, 1 H), 7.32 (dd, *J*=11.0*,* 8.8 Hz, 1 H), 7.28 (d, *J*=6.0 Hz, 1 H), 7.15 (s, 1 H), 7.01 (s, 1 H), 4.25 (t, *J*=4.3 Hz, 2 H), 3.47 (br s, 2 H), 3.38 (q, *J*=6.6 Hz, 2 H), 2.47 (br t, *J*=6.9 Hz, 2 H), 2.15 - 2.59 (m, 8 H), 2.11 (s, 3 H).

### Example 29: 2-(4-methylpiperazin-1-yl)ethyl 4-{[6-(5-chloro-2-fluorophenyl)-2H,3H,4H -pyrido [3,2-b] [1,4] oxazin-8-yl] amino} pyridine-3-carboxylate

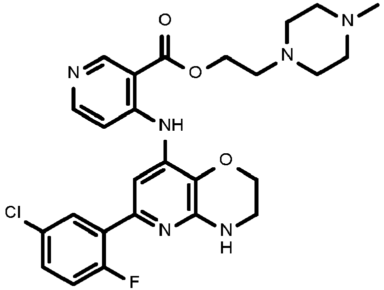

Example 29 was prepared as described for Example 27 starting from Intermediate 49 (65 mg, 0.15 mmol) and 1-(2-hydroxyethyl)-4-methylpiperazine (64.3 µL, 0.45 mmol). The crude material was purified by FC on Biotage silica-NH gel (from 10% to 100% of EtOAc in c-Hex as eluent), affording title compound (18 mg, 0.03 mmol, 23% yield) as pale yellow solid.

LC-MS (ESI): *m*/*z* (M+2): 264.3 (Method 1)

¹H NMR (500 MHz, *DMSO-d₆)* δ ppm 9.71 (s, 1 H), 8.89 (s, 1 H), 8.38 (d, *J***=**6.0 Hz, 1 H), 7.94 (dd, *J*=6.7, 2.9 Hz, 1 H), 7.42 - 7.48 (m, 1 H), 7.33 (dd, *J*=11.0, 8.8 Hz, 1 H), 7.22 (d, *J*=6.0 Hz, 1 H), 7.17 (s, 1 H), 7.13 (s, 1 H), 4.42 (t, *J*=5.6 Hz, 2 H), 4.26 (t, *J*=4.1 Hz, 2 H), 3.44 - 3.52 (m, 2 H), 2.71 (t, *J*=5.6 Hz, 2 H), 2.13 - 2.58 (m, 8 H), 2.09 (s, 3 H).

### Example 30: methyl 4-{[6-(6-methylpyridin-2-yl)-2H,3H,4H-pyrido[3,2-b][1,4]oxazin-8-yl]amino)pyridine-3-carboxylate

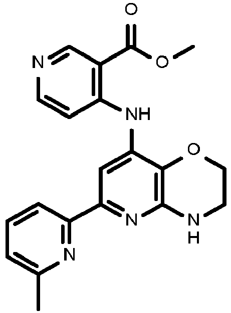

Example 30 was prepared as described for Example 4 starting from Intermediate 51 (35 mg, 0.07 mmol). The crude material was purified by FC on Biotage silica gel (from 0% to 3% of MeOH in DCM as eluent, 0.3% of aqueous ammonia as additive). Evaporation of opportune fractions provided title compound (19 mg, 0.05 mmol, 69% yield).

LC-MS (ESI): *m*/*z* (M+1): 378.2 (Method 2)

¹H NMR (500 MHz, *DMSO-d₆)* δ ppm 9.81 (s, 1 H), 8.92 (s, 1 H), 8.42 (d, *J*=6.0 Hz, 1 H), 7.98 (d, *J*=8.0 Hz, 1 H), 7.68 - 7.80 (m, 2 H), 7.23 (d, *J*=6.0 Hz, 1 H), 7.19 (d, *J*=7.7 Hz, 1 H), 6.95 (s, 1 H), 4.26 (t, *J*=4.3 Hz, 2 H), 3.91 (s, 3 H), 3.44 - 3.54 (m, 2 H), 2.50 (s, 3 H).

### Example 31: 6-(5-chloro-2-fluorophenyl)-N-11H-pyrrolo[2,3-b]pyridin-4-yl}-2H,3H,4H-pyrido[3,2-b][1,4]oxazin-8-amine

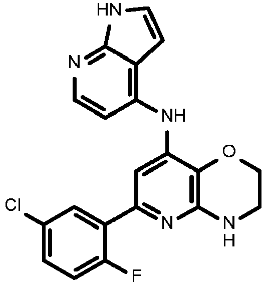

A solution of Intermediate 54 (114 mg, 0.18 mmol) in a mixture of DCM (4 mL) and TFA (1.11 mL, 14.56 mmol) was stirred overnight at RT. Then volatiles were removed under vacuum and the residue was dissolved in 7 N ammonia in MeOH and stirred at 50 °C for 1 h. Volatiles were removed again under vacuum. The residue was triturated with DCM, affording title compound (51 mg, 0.13 mmol, 71% yield) as a white solid.

LC-MS (ESI): *m*/*z* (M+1): 396.2 (Method 1)

¹H NMR (500 MHz, *DMSO-d₆)* δ ppm 11.40 (br s, 1 H), 8.20 (s, 1 H), 7.93 - 7.98 (m, 2 H), 7.38 - 7.44 (m, 1 H), 7.28 (dd, *J*=11.0*,* 8.8 Hz, 1 H), 7.21 - 7.25 (m, 1 H), 7.05 (s, 1 H), 6.91 (s, 1 H), 6.63 (d, *J*=5.5 Hz, 1 H), 6.58 (dd, *J*=3.3, 1.9 Hz, 1 H), 4.22 (t, *J*=4.3 Hz, 2 H), 3.48 (br d, *J*=2.5 Hz, 2 H).

### Example 32: 4-{[6-(5-chloro-2-fluorophenyl)-2H,3H,4H-pyrido[3,2-b][1,4]oxazin-8-yl]amino}-N-(1-methylpiperidin-4-yl)pyridine-3-carboxamide

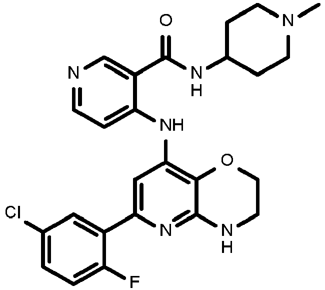

Example 32 was prepared as described for Example 28, starting from Intermediate 55 (87 mg, 0.15 mmol). The crude material was purified by FC on Biotage silica-NH gel (from 0% to 100% of MeOH in DCM as eluent)). Evaporation of opportune fractions provided the title compound (62 mg, 0.12 mmol, 86% yield) as a white solid.

LC-MS (ESI): *m*/*z* (M+1): 497.2 (Method 2)

¹H NMR (500 MHz, *DMSO-d₆)* δ ppm 10.26 (s, 1 H), 8.72 (s, 1 H), 8.61 (d, *J*=7.7 Hz, 1 H), 8.34 (d, *J*=6.0 Hz, 1 H), 7.93 (dd, *J*=6.9, 2.7 Hz, 1 H), 7.40 - 7.48 (m, 1 H), 7.27 - 7.37 (m, 2 H), 7.15 (d, *J*=0.8 Hz, 1 H), 7.01 (s, 1 H), 4.25 (t, *J*=4.4 Hz, 2 H), 3.65 - 3.85 (m, 1 H), 3.44 - 3.55 (m, 2 H), 2.77 (br d, *J*=11.5 Hz, 2 H), 2.16 (s, 3 H), 1.94 (td, *J*=11.7, 1.6 Hz, 2 H), 1.71 - 1.84 (m, 2 H), 1.59 (qd, *J*=12.0, 3.8 Hz, 2 H).

### Example 33: N-(4-{[6-(5-chloro-2-fluorophenyl)-2H,3H,4H-pyrido[3,2-b][1,4]oxazin-8-yl]amino}pyridin-2-yl)-3-(4-methylpiperazin-1-yl)propanamide

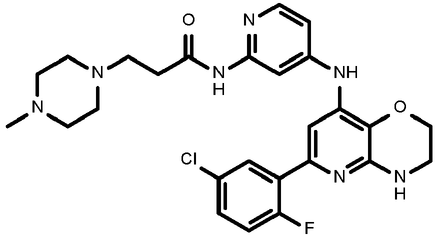

Example 33 was prepared as described for Example 28, starting from Intermediate 58 (68 mg, 0.11 mmol). The crude material was purified by FC on Biotage silica-NH gel (from 0% to 100% of EtOAc in c-Hex, followed by 20% of MeOH in EtOAc as eluent). Fractions containing the target compound were concentrated at reduced pressure and further purified reverse FC on Biotage C18 cartridge (from 5% to 60% of MeCN in water with ammonia-ammonium bicarbonate buffer (pH=10) as eluent). Evaporation of opportune fractions provided title compound (34 mg, 0.06 mmol, 60% yield) as a white solid.

LC-MS (ESI): *m*/*z* (M+2): 263.7 (Method 2)

¹H NMR (400 MHz, *DMSO-d₆)* δ ppm 10.41 (s, 1 H), 8.59 (s, 1 H), 7.92 - 8.00 (m, 2 H), 7.81 (d, *J*=1.3 Hz, 1 H), 7.42 (ddd, *J*=8.8, 4.0, 2.8 Hz, 1 H), 7.29 (dd, *J*=11.2*,* 8.8 Hz, 1 H), 7.03 (s, 1 H), 6.94 (s, 1 H), 6.68 (dd, *J*=5.7, 2.0 Hz, 1 H), 4.20 (t, *J*=4.2 Hz, 2 H), 3.43 - 3.52 (m, 2 H), 2.58 (t, *J*=7.2 Hz, 2 H), 2.45 - 2.49 (m, 2 H), 2.19 - 2.45 (m, 8 H), 2.13 (s, 3 H).

### Example 34: N-(4-{[6-(5-chloro-2-fluorophenyl)-2H,3H,4H-pyrido[3,2-b][1,4]oxazin-8-yl]amino}pyridin-2-yl)-3-(dimethylamino)propanamide

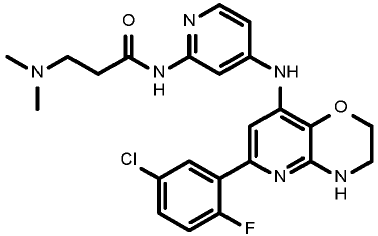

Example 34 was prepared as described for Example 28, starting from Intermediate 61 (58 mg, 0.10 mmol). The crude material was purified by FC on Biotage silica-NH gel (from 10% to 100% of EtOAc in c-Hex, followed by 10% of MeOH in EtOAc as eluent). Fractions containing the target compound were concentrated at reduced pressure and further purified by reverse FC on Biotage C18 cartridge (from 5% to 70% of MeCN in water with ammonia-ammonium bicarbonate buffer (pH=10) as eluent). Evaporation of opportune fractions provided title compound (27 mg, 0.06 mmol, 56% yield) as an off-white solid.

LC-MS (ESI): *m*/*z* (M+1): 471.1 (Method 2)

¹H NMR (400 MHz, *DMSO-d₆)* δ ppm 10.40 (s, 1 H), 8.58 (s, 1 H), 7.92 - 8.01 (m, 2 H), 7.81 (d, *J*=1.5 Hz, 1 H), 7.42 (ddd, *J*=8.8, 4.2, 2.8 Hz, 1 H), 7.29 (dd, *J*=11.2, 8.8 Hz, 1 H), 7.03 (d, *J*=0.7 Hz, 1 H), 6.93 (s, 1 H), 6.68 (dd, *J*=5.7, 2.2 Hz, 1 H), 4.20 (t, *J*=4.2 Hz, 2 H), 3.42 - 3.50 (m, 2 H), 2.42 - 2.55 (m, 4 H), 2.15 (s, 6 H).

### Example 35: 6-(5-chloro-2-fluorophenyl)-N-{3H-imidazo[4,5-b]pyridin-7-yl}-2H,3H,4H-pyrido[3,2-b][1,4]oxazin-8-amine

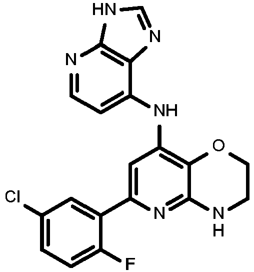

Intermediate 66 (85 mg, 0.14 mmol) was suspended in 4 N HCl solution in 1,4-dioxane (1.4 mL) and MeOH (0.8 mL) and stirred at RT for 2 hrs. Volatiles were removed under vacuum. The residue was taken up with 4 N ammonia in MeOH (2 mL) and volatiles were removed again (3x). The solid was triturated with MeOH and water, affording 6-(5-chloro-2-fluorophenyl)-N-{3H-imidazo[4,5-b]pyridin-7-yl}-2H,3H,4H-pyrido[3,2-b][1,4]oxazin-8-amine (18 mg, 0.04 mmol, 33 % yield) as white solid.

LC-MS (ESI): *m*/*z* (M+1): 397.1 (Method 1)

¹H NMR (400 MHz, *DMSO-d₆)* δ ppm 12.20 - 13.74 (m, 1 H), 8.22 (s, 1 H), 8.09 (d, *J*=5.5 Hz, 1 H), 7.98 - 8.16 (m, 1 H), 7.94 (dd, *J*=6.7, 2.5 Hz, 1 H), 7.38 - 7.47 (m, 1 H), 7.27 - 7.37 (m, 1 H), 7.23 (s, 1 H), 6.85 - 6.99 (m, 2 H), 4.25 (br s, 2 H), 3.48 (br s, 2 H).

### Example 36: methyl 4-{[6-(5-chloro-2-fluorophenyl)-2H,3H,4H-pyrido[3,2-b][1,4]oxazin-8-yl]amino}-1H-pyrrolo[2,3-b]pyridine-3-carboxylate

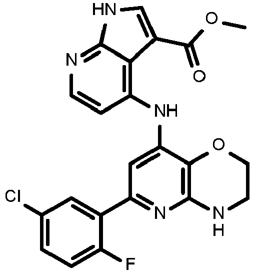

To a solution of Intermediate 68 (71 mg, 0.10 mmol) in DCM (2.0 mL), TFA (0.32 mL, 4.15 mmol) was added. The mixture was stirred at RT overnight. Toluene (2 mL) was added, the volatiles were removed under vacuum. The crude material was purified reverse FC on Biotage C18 cartridge (from 5% to 50% of MeCN +0.1% HCOOH in water +0.1% HCOOH as eluent) affording title compound (5 mg, 0.01 mmol, 11% yield) as white solid.

LC-MS (ESI): *m*/*z* (M+1): 454.2 (Method 1)

¹H NMR (400 MHz, *DMSO-d₆)* δ ppm 12.39 (br s, 1 H), 10.12 (s, 1 H), 8.12 (s, 1 H), 8.06 (d, *J*=5.7 Hz, 1 H), 7.96 (dd, *J*=6.6, 2.6 Hz, 1 H), 7.42 (br dd, *J*=7.2, 4.2 Hz, 1 H), 7.26 - 7.37 (m, 2 H), 6.90 - 7.02 (m, 2 H), 4.27 (br s, 2 H), 3.85 (s, 3 H), 3.50 (br s, 2 H).

### Example 37: N-{2-[bis(2-hydroxyethyl)amino]ethyl}-4-{[6-(5-chloro-2-fluorophenyl)-2H,3H,4H-pyrido[3,2-b][1,4]oxazin-8-yl]amino}pyridine-3-carboxamide

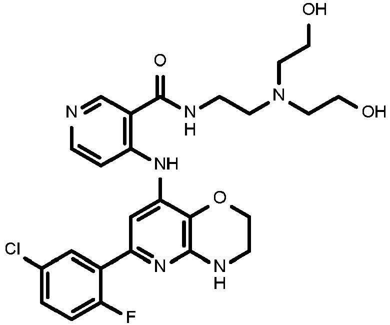

Example 37 was prepared as described for Example 28, starting from Intermediate 69 (58 mg, 0.09 mmol). The crude material was purified reverse FC on Biotage C18 cartridge (from 5% to 65% of MeCN in water with ammonia-ammonium bicarbonate buffer (pH=10) as eluent). Evaporation of opportune fractions provided title compound (33 mg, 0.06 mmol, 68% yield) as a white solid.

LC-MS (ESI): *m*/*z* (M+1): 531.1 (Method 1)

¹H NMR (400 MHz, *DMSO-d₆)* δ ppm 10.27 (s, 1 H), 8.70 (s, 1 H), 8.68 (br t, *J*=5.6 Hz, 1 H), 8.34 (d, *J*=5.9 Hz, 1 H), 7.94 (dd, *J*=6.8, 2.8 Hz, 1 H), 7.44 (ddd, *J*=8.8, 4.1, 2.8 Hz, 1 H), 7.32 (dd, *J*=11.1, 8.8 Hz, 1 H), 7.28 (d, *J*=5.8 Hz, 1 H), 7.16 (s, 1 H), 7.01 (s, 1 H), 4.37 (t, *J*=5.5 Hz, 2 H), 4.26 (t, *J*=4.0 Hz, 2 H), 3.40 - 3.50 (m, 6 H), 3.30 - 3.38 (m, 2 H), 2.68 (t, *J*=6.5 Hz, 2 H), 2.59 (t, *J*=6.2 Hz, 4 H).

### Example 38: methyl 4-{[6-(5-chloro-2-fluorophenyl)-2H,3H,4H-pyrido[3,2-b][1,4]oxazin-8-yl]amino}-1H-pyrrolo[2,3-b]pyridine-2-carboxylate

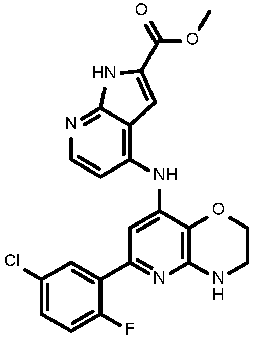

To a solution of Intermediate 71 (95 mg, 0.14 mmol) in DCM (2.8 mL), TFA (0.43 mL, 5.55 mmol) was added dropwise. The mixture was stirred at RT for 5 hrs. The mixture was filtered using a PL-HCO₃ SPE cartridge (5g), washing with DCM and the filtrate was concentrated under vacuum. The residue material was taken up with DMSO/water mixture and the resulting precipitate was filtered and thoroughly washed with water, title compound (35 mg, 0.08 mmol, 56% yield) as white solid.

LC-MS (ESI): *m*/*z* (M+1): 454.1 (Method 1)

¹H NMR (400 MHz, *DMSO-d₆)* δ ppm 12.08 - 12.31 (m, 1 H), 8.67 (s, 1 H), 8.08 (d, *J*=5.3 Hz, 1 H), 7.97 (dd, *J*=6.8, 2.9 Hz, 1 H), 7.52 (s, 1 H), 7.43 (ddd, *J*=8.6, 4.0, 3.0 Hz, 1 H), 7.31 (dd, *J*=11.1*,* 8.7 Hz, 1 H), 7.08 (d, *J*=1.1 Hz, 1 H), 7.03 (s, 1 H), 6.54 (d, *J*=5.5 Hz, 1 H), 4.21 (t, *J*=4.2 Hz, 2 H), 3.86 (s, 3 H), 3.47 - 3.53 (m, 2 H).

### Example 39: N-(4-{[6-(5-chloro-2-fluorophenyl)-2H,3H,4H-pyrido[3,2-b][1,4]oxazin-8-yl]amino}pyridin-3-yl)-3-(dimethylamino)propanamide

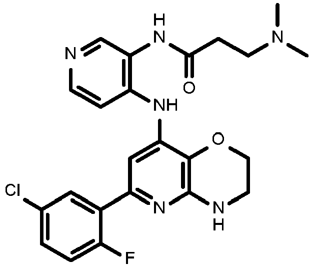

Example 39 was prepared as described for Example 28, starting from Intermediate 74 (61 mg, 0.11 mmol) The crude material was purified by FC on Biotage silica-NH gel (from 0% to 10% of MeOH in DCM as eluent), then by prep-HPLC. Evaporation of proper fraction provided title compound (12 mg, 0.02 mmol, 24% yield) as a white solid.

LC-MS (ESI): *m*/*z* (M+1): 471.1 (Method 1)

¹H NMR (400 MHz, *DMSO-d₆)* δ ppm 10.04 (br s, 1 H), 8.38 (s, 1 H), 8.17 (d, *J*=5.5 Hz, 1 H), 7.93 (dd, *J*=6.9, 2.8 Hz, 1 H), 7.71 (br s, 1 H), 7.36 - 7.46 (m, 1 H), 7.29 (dd, *J*=11.1*,* 8.8 Hz, 1 H), 7.09 (d, *J*=*5.5* Hz, 1 H), 6.93 (br s, 1 H), 6.88 (s, 1 H), 4.20 (t, *J*=4.1 Hz, 2 H), 3.46 (br t, *J*=4.2 Hz, 2 H), 2.53 - 2.61 (m, 2 H), 2.45 - 2.52 (m, 2 H), 2.16 (s, 6 H)

### Example 40: N-{3H-imidazo[4,5-b]pyridin-7-yl}-6-(6-methylpyridin-2-yl)-2H,3H,4H-pyrido[3,2-b][1,4]oxazin-8-amine

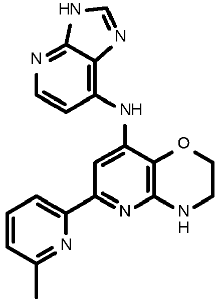

Intermediate 77 (100 mg, 0.17 mmol) was suspended in 4 N HCl solution in 1,4-dioxane (1.7 mL, 6.78 mmol) and MeOH (0.94 mL) and the mixture was stirred at 40 °C for 2 hrs. The solvents were removed under vacuum. 4 N ammonia solution in MeOH (2 mL) was added and the volatiles were removed under vacuum. The crude material was purified by FC on Biotage silica-NH gel (from 0% to 5% of MeOH in DCM as eluent), affording title compound (40 mg, 0.11 mmol, 66% yield) as white solid.

LC-MS (ESI): *m*/*z* (M+2): 180.6 (Method 1)

¹H NMR (500 MHz, *DMSO-d₆)* δ ppm 12.04 - 13.54 (m, 1 H), 8.25 (s, 1 H), 8.15 (d, *J*=*5.5* Hz, 1 H), 7.93 - 8.10 (m, 2 H), 7.84 (br s, 1 H), 7.74 (t, *J*=7.7 Hz, 1 H), 7.18 (d, J=7.5 Hz, 1 H), 6.93 (d, *J*=5.5 Hz, 1 H), 6.79 (s, 1 H), 4.26 (t, *J*=4.3 Hz, 2 H), 3.43 - 3.56 (m, 2 H), 2.42 - 2.55 (m, 3 H).

### Example 41: 6-(6-methylpyridin-2-yl)-N-11H-pyrazolo[3,4-b]pyridin-4-yl}-2H,3H,4H-pyrido[3,2-b][1,4]oxazin-8-amine

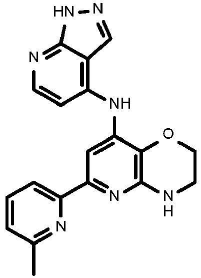

Example 41 was prepared following the procedure described for Example 40, starting from Intermediate 78 (100 mg, 0.17 mmol). The crude material was purified by FC on Biotage silica-NH gel (from 0% to 2% of MeOH in DCM as eluent), affording title compound (54 mg, 0.15 mmol, 89% yield) as white solid.

LC-MS (ESI): *m*/*z* (M+2): 180.6 (Method 1)

¹H NMR (500 MHz, *DMSO-d₆)* δ ppm 13.24 (s, 1 H), 9.03 (s, 1 H), 8.19 (s, 1 H), 8.12 (d, *J*=5.5 Hz, 1 H), 7.99 (d, *J*=7.7 Hz, 1 H), 7.74 (t, *J*=7.7 Hz, 1 H), 7.65 (s, 1 H), 7.17 (d, *J*=7.7 Hz, 1 H), 6.90 (s, 1 H), 6.43 (d, *J*=5.5 Hz, 1 H), 4.20 (t, *J*=3.9 Hz, 2 H), 3.43 - 3.53 (m, 2 H), 2.46 (s, 3 H).

### Example 42: N-(1-methylpiperidin-4-yl)-4-{[6-(6-methylpyridin-2-yl)-2H,3H,4H-pyrido[3,2-b][1,4]oxazin-8-yl]amino)pyridine-3-carboxamide

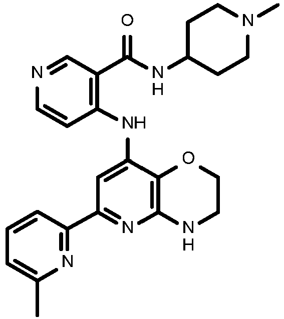

Example 42 was prepared as described for Example 28, starting from Intermediate 80 (68 mg, 0.12 mmol). The crude material was purified by SCX (1g, washing with MeOH, eluting with 1 N ammonia in MeOH), affording title compound (48.2 mg, 0.10 mmol, 86% yield) as white solid.

LC-MS (ESI): *m*/*z* (M+2): 230.6 (Method 2)

¹H NMR (400 MHz, *DMSO-d₆)* δ ppm 10.22 (s, 1 H), 8.74 (s, 1 H), 8.61 (br d, *J*=7.5 Hz, 1 H), 8.37 (d, *J*=5.9 Hz, 1 H), 7.97 (d, *J*=7.7 Hz, 1 H), 7.66 - 7.78 (m, 2 H), 7.27 (d, *J*=5.7 Hz, 1 H), 7.18 (d, *J*=7.2 Hz, 1 H), 6.83 (br s, 1 H), 4.25 (br s, 2 H), 3.67 - 3.85 (m, 1 H), 3.47 (br d, *J*=1.1 Hz, 2 H), 2.77 (br d, *J*=11.8 Hz, 2 H), 2.43 - 2.57 (m, 3 H), 2.16 (s, 3 H), 1.95 (t, *J*=11.9 Hz, 2 H), 1.71 - 1.85 (m, 2 H), 1.50 - 1.67 (m, 2 H).

### Example 43: 4-{[6-(5-chloro-2-fluorophenyl)-2H,3H,4H-pyrido[3,2-b][1,4]oxazin-8-yl]amino}-N-{2-[methyl(oxetan-3-yl)amino]ethyl}pyridine-3-carboxamide

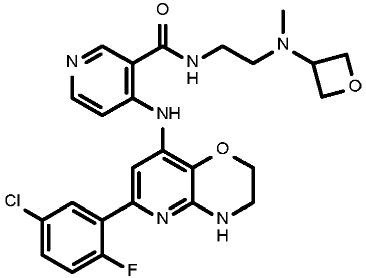

Example 43 was prepared as described for Example 28, starting from Intermediate 82 (53 mg, 0.09 mmol). The crude material was purified by FC on Biotage silica-NH gel (from 0% to 5% of MeOH in DCM as eluent). Evaporation of opportune fractions provided title compound (32 mg, 0.06 mmol, 72% yield) as a white solid.

LC-MS (ESI): *m*/*z* (M+1): 513.2 (Method 2)

¹H NMR (500 MHz, *DMSO-d₆)* δ ppm 10.25 (s, 1 H), 8.78 (t, *J*=5.6 Hz, 1 H), 8.71 (s, 1 H), 8.34 (d, *J*=5.8 Hz, 1 H), 7.93 (dd, *J*=6.9, 2.9 Hz, 1 H), 7.39 - 7.48 (m, 1 H), 7.33 (dd, *J*=11.1, 8.8 Hz, 1 H), 7.28 (d, *J*=5.9 Hz, 1 H), 7.15 (d, *J*=0.7 Hz, 1 H), 7.02 (s, 1 H), 4.36 - 4.58 (m, 4 H), 4.25 (t, *J*=4.3 Hz, 2 H), 3.57 (quin, *J*=6.4 Hz, 1 H), 3.43 - 3.51 (m, 2 H), 3.34 - 3.39 (m, 2 H), 2.41 (t, *J*=6.7 Hz, 2 H), 2.13 (s, 3 H).

### Example 44: methyl 2-({2-[(4-{[6-(5-chloro-2-fluorophenyl)-2H,3H,4H-pyrido[3,2-b][1,4]oxazin-8-yl]amino}pyridin-3-yl)formamido]ethyl}(methyl)amino)acetate

Example 44 was prepared as described for Example 28, starting from 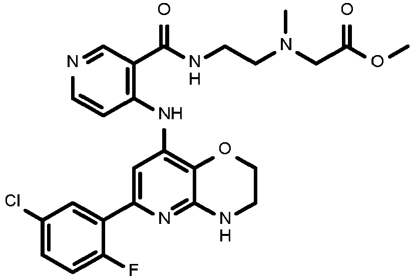 Intermediate 84 (80 mg, 0.13 mmol). The crude material was purified by FC on Biotage silica-NH gel (from 0% to 5% of MeOH in DCM as eluent). Evaporation of opportune fractions provided title compound (57 mg, 0.11 mmol, 85% yield) as a white solid.

LC-MS (ESI): *m*/*z* (M+1): 529.1 (Method 2)

¹H NMR (500 MHz, *DMSO-d₆)* δ ppm 10.29 (s, 1 H), 8.66 - 8.79 (m, 2 H), 8.34 (d, *J*=5.7 Hz, 1 H), 7.93 (dd, *J*=6.9, 2.7 Hz, 1 H), 7.44 (ddd, *J*=8.8, 4.2, 3.1 Hz, 1 H), 7.32 (dd, *J*=11.2, 8.8 Hz, 1 H), 7.28 (d, *J*=5.7 Hz, 1 H), 7.15 (s, 1 H), 6.98 - 7.04 (m, 1 H), 4.25 (t, *J*=4.1 Hz, 2 H), 3.60 (s, 3 H), 3.43 - 3.51 (m, 2 H), 3.33 - 3.41 (m, 4 H), 2.68 (t, *J*=6.6 Hz, 2 H), 2.35 (s, 3 H)

### Example 45: 6-(6-methylpyridin-2-yl)-N-11H-pyrrolo[2,3-b]pyridin-4-yll-2H,3H,4H-pyrido[3,2-b][1,4]oxazin-8-amine

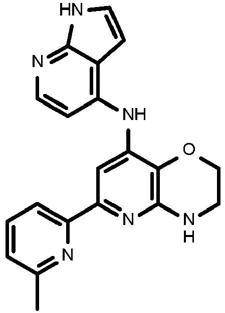

Example 45 was prepared following the procedure described for Example 40, starting from Intermediate 86 (73 mg, 0.12 mmol). The crude material was purified by FC on Biotage silica-NH gel (from 0% to 10% of MeOH in DCM as eluent), affording title compound (17.8 mg, 0.05 mmol, 40% yield) as white solid.

LC-MS (ESI): *m*/*z* (M+1): 359.1 (Method 2)

¹H NMR (500 MHz, *DMSO-d₆)* δ ppm 11.38 (br s, 1 H), 8.22 (s, 1 H), 7.92 - 8.02 (m, 2 H), 7.72 (t, *J*=7.7 Hz, 1 H), 7.64 (s, 1 H), 7.20 - 7.26 (m, 1 H), 7.15 (d, *J*=7.5 Hz, 1 H), 6.74 (br s, 1 H), 6.58 - 6.64 (m, 1 H), 6.55 (d, *J*=5.4 Hz, 1 H), 4.20 (br t, *J*=4.1 Hz, 2 H), 3.48 (br s, 2 H), 2.44 (s, 3 H).

### Example 46: 4-{[6-(5-chloro-2-fluorophenyl)-2H,3H,4H-pyrido[3,2-b][1,4]oxazin-8-yl]amino}-N-[2-(4-methanesulfonylpiperazin-1-yl)ethyl]pyridine-3-carboxamide

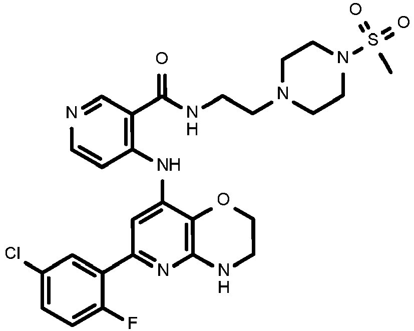

Example 46 was prepared as described for Example 28, starting from Intermediate 87 (95 mg, 0.14 mmol).The crude material was purified by SCX (1g, washing with MeOH, eluting with 1 N ammonia in MeOH) affording title compound (67 mg, 0.11 mmol, 83% yield) as white solid.

LC-MS (ESI): *m*/*z* (M+2): 295.7 (Method 1)

¹H NMR (400 MHz, *DMSO-d₆)* δ ppm 10.25 (s, 1 H), 8.74 (br t, *J*=5.5 Hz, 1 H), 8.71 (s, 1 H), 8.34 (d, *J*=5.8 Hz, 1 H), 7.93 (dd, *J*=6.8, 2.7 Hz, 1 H), 7.39 - 7.47 (m, 1 H), 7.32 (dd, *J*=11.1, 8.9 Hz, 1 H), 7.28 (d, *J*=5.9 Hz, 1 H), 7.15 (s, 1 H), 7.01 (s, 1 H), 4.25 (br t, *J*=3.8 Hz, 2 H), 3.47 (br s, 2 H), 3.41 (q, *J*=6.3 Hz, 2 H), 3.05 - 3.13 (m, 4 H), 2.82 (s, 3 H), 2.52 - 2.59 (m, 6 H).

### Example 47: N-[2-(4-acetylpiperazin-1-yl)ethyl]-4-{[6-(5-chloro-2-fluorophenyl)-2H,3H,4H-pyrido[3,2-b][1,4]oxazin-8-yl]amino}pyridine-3-carboxamide

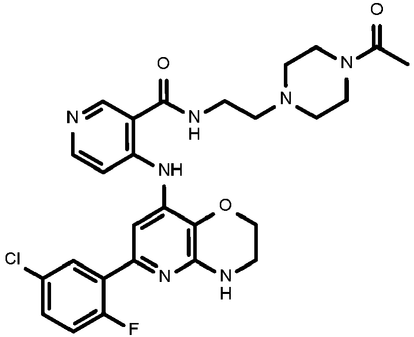

Example 47 was prepared as described for Example 28, starting from Intermediate 88 (74 mg, 0.11 mmol). The crude material was purified by SCX (1g, washing with MeOH, eluting with 1 N ammonia in MeOH) affording the title compound (52 mg, 0.09 mmol, 83% yield) as white solid.

LC-MS (ESI): *m*/*z* (M+2): 277.7 (Method 1)

¹H NMR (400 MHz, *DMSO-d₆)* δ ppm 10.24 (s, 1 H), 8.75 (t, *J*=5.5 Hz, 1 H), 8.71 (s, 1 H), 8.34 (d, *J*=5.9 Hz, 1 H), 7.93 (dd, *J*=6.9, 2.7 Hz, 1 H), 7.40 - 7.47 (m, 1 H), 7.24 - 7.36 (m, 2 H), 7.15 (s, 1 H), 7.01 (s, 1 H), 4.25 (t, *J*=3.9 Hz, 2 H), 3.35 - 3.51 (m, 8 H), 2.50 - 2.58 (m, 2 H), 2.33 - 2.47 (m, 4 H), 1.97 (s, 3 H).

### Example 48: 4-{[6-(5-chloro-2-fluorophenyl)-2H,3H,4H-pyrido[3,2-b][1,4]oxazin-8-yl]amino}-N-[2-(1-methylpiperidin-4-yl)ethyl]-1H-pyrrolo[2,3-b]pyridine-2-carboxamide

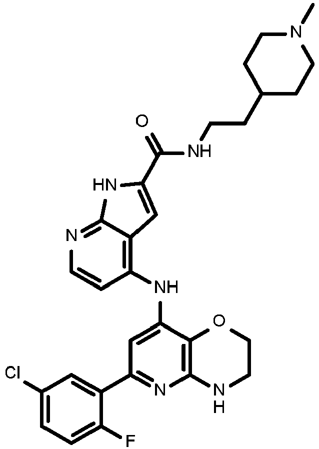

Intermediate 90 (129 mg, 0.16 mmol) was suspended in 4 N HCl solution in 1,4-dioxane (1.62 mL, 6.5 mmol) and MeOH (1.2 mL) and the mixture was stirred at 35 °C for 6 hrs. The solvents were removed under vacuum. The crude material was purified by SCX (1g, washing with MeOH, eluting with 1 N ammonia in MeOH), affording title compound (64 mg, 0.11 mmol, 70% yield) as white solid.

LC-MS (ESI): *m*/*z* (M+2): 282.7 (Method 2)

¹H NMR (400 MHz, *DMSO-d₆)* δ ppm 11.78 (br s, 1 H), 8.41 (s, 1 H), 8.24 (br t, *J*=5.4 Hz, 1 H), 8.05 (d, *J*=5.4 Hz, 1 H), 7.94 (dd, *J*=6.8, 2.8 Hz, 1 H), 7.40 (dt, *J*=8.4, 3.5 Hz, 1 H), 7.27 (dd, *J*=10.9, 8.9 Hz, 1 H), 7.18 (d, *J*=1.0 Hz, 1 H), 7.01 (s, 1 H), 6.94 (br s, 1 H), 6.65 (d, *J*=5.4 Hz, 1 H), 4.22 (br t, *J*=4.0 Hz, 2 H), 3.49 (br s, 2 H), 3.24 - 3.31 (m, 2 H), 2.66 - 2.86 (m, 2 H), 2.12 (br s, 3 H), 1.79 (br d, *J*=4.5 Hz, 2H), 1.58 - 1.69 (m, 2 H), 1.37 - 1.52 (m, 2 H), 1.21 - 1.34 (m, 1 H), 1.14 (qd, *J*=11.9, 3.3 Hz, 2 H).

### Example 49: 4-{[6-(5-chloro-2-fluorophenyl)-2H,3H,4H-pyrido[3,2-b][1,4]oxazin-8-yl]amino}-N-[2-(dimethylamino)ethyl]-1H-pyrrolo[2,3-b]pyridine-2-carboxamide

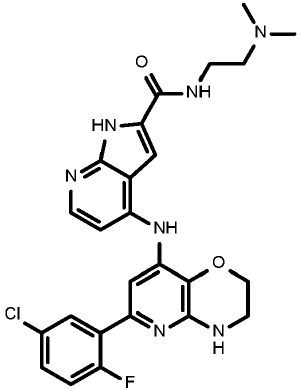

Example 49 was prepared following the procedure described for Example 48, starting from Intermediate 91 (120 mg, 0.16 mmol). Title compound (50 mg, 0.10mmol, 61% yield) was obtained as white solid.

LC-MS (ESI): *m*/*z* (M+2): 255.6 (Method 1)

¹H NMR (400 MHz, *DMSO-d₆)* δ ppm 11.82 (s, 1 H), 8.42 (s, 1 H), 8.17 (t, *J*=5.5 Hz, 1 H), 8.05 (d, *J*=5.4 Hz, 1 H), 7.94 (dd, *J*=6.9, 2.8 Hz, 1 H), 7.35 - 7.48 (m, 1 H), 7.27 (dd, *J*=11.1, 8.8 Hz, 1 H), 7.20 (s, 1 H), 7.02 (s, 1 H), 6.95 (s, 1 H), 6.64 (d, *J*=5.5 Hz, 1 H), 4.22 (t, *J*=4.1 Hz, 2 H), 3.49 (br s, 2 H), 3.36 (q, *J*=6.5 Hz, 2 H), 2.39 (t, *J*=6.6 Hz, 2 H), 2.17 (s, 6 H).

### Comparative newly synthesised compounds lacking an amino group as linker between the pyrido oxazine ring and the pyridine or phenyl ring

### Example C1: 4-[6-(5-chloro-2-fluorophenyl)-2H,3H,4H-pyrido[3,2-b][1,4]oxazin-8-yl]pyridine

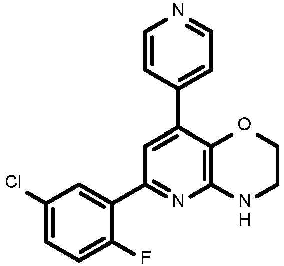

To a stirred suspension of 2-amino-6-(5-chloro-2-fluorophenyl)-[4,4'-bipyridin]-3-ol (Intermediate 97, 103 mg, 0.33 mmol), and K₂CO₃ (359 mg, 2.6 mmol) in dry DMF (6.5 mL), 1,2-dibromoethane (84 µL, 0.980 mmol) was added and the mixture was heated at 80 °C, in a PLS, for 5 hrs. The mixture was diluted with EtOAc and washed with sat. aqueous NaHCO₃ solution (3x) and brine (1x). The organic phase was filtered through a phase separator and concentrated under vacuum. The crude material was purified by FC on Biotage silica gel (from 20% to 50% of EtOAc in c-Hex as eluent), and then by reverse FC on Biotage C18 cartridge (from 5% to 40% of MeCN +0.1% HCOOH in water +0.1% HCOOH as eluent) to give 4-[6-(5-chloro-2-fluorophenyl)-2H,3H,4H-pyrido[3,2-b][1,4]oxazin-8-yl]pyridine (23.3 mg, 0.07 mmol, 21% yield) as pale yellow solid.

LC-MS (ESI): *m*/*z* (M+1): 342.1 (Method 1)

¹H NMR (400 MHz, *DMSO-d₆)* δ ppm 8.59 - 8.73 (m, 2 H), 7.95 (dd, *J*=6.8, 2.9 Hz, 1 H), 7.56 - 7.65 (m, 2 H), 7.40 - 7.50 (m, 1 H), 7.34 (dd, *J*=11.0*,* 8.8 Hz, 1 H), 7.28 (s, 1 H), 7.06 (d, *J*=1.5 Hz, 1 H), 4.21 (t, *J*=4.4 Hz, 2 H), 3.50 (br d, *J*=2.9 Hz, 2 H).

### Example C2: 4-[6-(5-chloro-2-fluorophenyl)-2H,3H,4H-pyrido[3,2-b][1,4]oxazin-8-yl]phenol

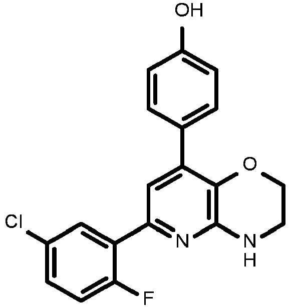

In a suitable vial, a mixture 8-bromo-6-(5-chloro-2-fluorophenyl)-2H,3H,4H-pyrido[3,2-b][1,4]oxazine (Intermediate 5, 60 mg, 0.17 mmol), 4-hydroxyphenylboronic acid (28.9 mg, 0.21 mmol), Na₂CO₃ (37 mg, 0.35 mmol) and Pd(dppf)Cl₂ DCM (7.15 mg, 0.01 mmol) was suspended in 1,4-dioxane/water (2.2 mL, 3:1 ratio). The vial was sealed, evacuated and backfilled with N₂ three times, then heated at 80 °C for 45 min. The mixture was diluted with EtOAc, filtered over a pad of Celite^{®}. The filtrate was washed with brine (1x), the organic phase was separated, filtered through a phase separator and concentrated under vacuum. The crude material was purified by FC on Biotage silica gel (from 0% to 20% of EtOAc in c-Hex as eluent), then charged on a SCX cartridge, washed with MeOH and eluted with 1 N ammonia in MeOH. Basic fractions were evaporated to provide 4-[6-(5-chloro-2-fluorophenyl)-2H,3H,4H-pyrido[3,2-b][1,4]oxazin-8-yl]phenol (50 mg, 0.14 mmol, 80% yield) as white solid.

LC-MS (ESI): *m*/*z* (M+1): 357.2 (Method 1)

¹H NMR (500 MHz, *DMSO-d₆)* δ ppm 9.66 (br s, 1 H), 7.94 (dd, *J*=6.9, 2.7 Hz, 1 H), 7.38 - 7.51 (m, 3 H), 7.32 (dd, *J*=11.1, 8.9 Hz, 1 H), 7.04 (br s, 1 H), 6.99 (d, *J*=1.4 Hz, 1 H), 6.83 (d, *J*=8.5 Hz, 2 H), 4.16 (br t, *J*=4.1 Hz, 2 H), 3.48 (br s, 2 H).

### Example C3: 6-(5-chloro-2-fluorophenyl)-8-(4-methoxyphenyl)-2H,3H,4H-pyrido[3,2-b][1,4]oxazine

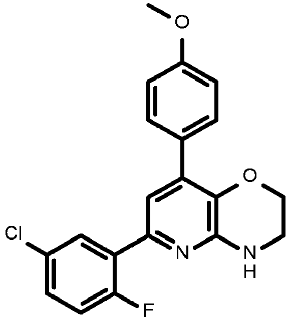

Prepared from 8-bromo-6-(5-chloro-2-fluorophenyl)-2H,3H,4H-pyrido[3,2-b][1,4]oxazine (Intermediate 5, 50 mg, 0.15 mmol) and 4-methoxyphenylboronic acid (26.6 mg, 0.17 mmol), following similar procedure as for Example C2. The crude material was purified by FC on Biotage silica gel (from 5% to 25% of EtOAc in c-Hex as eluent), affording 6-(5-chloro-2-fluorophenyl)-8-(4-methoxyphenyl)-2H,3H,4H-pyrido[3,2-b][1,4]oxazine (40 mg, 0.11 mmol, 74% yield) as a white solid.

LC-MS (ESI): *m*/*z* (M+1): 371.2 (Method 1)

¹H NMR (500 MHz, *DMSO-d₆)* δ ppm 7.92 - 7.97 (m, 1 H), 7.53 - 7.57 (m, 2 H), 7.40 - 7.46 (m, 1 H), 7.32 (dd, *J*=11.3*,* 8.8 Hz, 1 H), 7.08 (s, 1 H), 6.99 - 7.04 (m, 3 H), 4.17 (t, *J*=4.5 Hz, 2 H), 3.80 (s, 3 H), 3.44 - 3.54 (m, 2 H).

### PHARMACOLOGICAL ACTIVITY OF THE COMPOUNDS OF THE INVENTION

### In vitro Assay

The enzymatic activity of compounds of the present invention was monitored measuring the formation of ADP using the ADP-GLO Kinases assay. Following the incubation of the purified enzyme, a substrate and ATP, the produced ADP was converted into ATP, which in turn was converted into light by Ultra-Glo Luciferase. The luminescent signal positively correlated with ADP amount and kinase activity. Briefly, the kinase reaction was performed by incubating 2.6nM of the purified, commercially available human ALK5 (recombinant TGF β1 N-term GST-tagged, 80-end), a final concentration of TGFβ1 peptide 94.5µM (Promega, T36-58) and ultra-pure ATP (Promega V915B). The ATP concentration was set at the Km value (concentration of substrate which permits the enzyme to achieve half maximal velocity (Vmax)) of ALK5 (5µM). All reactions/incubations were performed at 25°C. Compound and ALK5 kinase were mixed and incubated for 15 mins. Reactions were initiated by addition of ATP at a final concentration in the assay of 0.83µM. After an incubation of 150 min, the reaction was stopped, and ADP production detected with ADP-Glo kit according to manufacturer's indications. The assay was performed in 384-well format and was validated using a selection of reference compounds that was tested in 11 point concentration-response curve.

The results for individual compounds are provided below in Table 10 wherein the compounds are classified in term of potency (nM) with respect to their inhibitory activity on ALK5 receptor

**Table 10**

| **Example No** | **ALK5 IC50 (nM)** |
|---|---|
| Compounds of formula (I) wherein A corresponds to A1: 1, 2, 8, 10, 12, 16, 17, 20, 21, 23, 24, 29, 30, 32, 33, 34, 37 | < 1 |
| Compounds of formula (I) wherein A corresponds to A2: 25, 31, 35, 36, 38, 40, 41, 45, 48, 49 | |
| 3, 7, 9, 11, 13-15, 18, 22, 27, 28, 42, 43, 46, 47 | 1 - 5 |
| 4-6, 19, 26, 39 | 5 - 10 |

As it can be appreciated, all the compounds of Table 10 show a good activity as antagonists of ALK5 receptor.

### Comparative Examples

Compounds of the examples C1, C2 and C3 were tested in the same *in vitro* assay described above.

**Table 11**

| **Example No** | **ALK5 IC50 (nM)** |
|---|---|
| C1 | 6500 |
| C2 | 1700 |
| C3 | 10000 |

The compounds of the present invention, as shown in Table 10, have a potency even lower than 1 nM, whereas comparative examples C1-C3 have a potency higher than 1700 nM and even largely higher.

These data, not even comparable, demonstrate that, conversely to the compounds C1, C2 and C3 characterized by a pyrido oxazine ring directly fused to a pyridine ring, in the compounds of the present invention, characterized by an amino group as linker between the pyrido oxazine ring and the pyridine or pyridine condensed to a 5-membered heterocyclic ring, the presence of the amino group as linker between these two groups unexpectedly and remarkably determines a relevant increase in the inhibitory activity on the ALK5 receptor.

### In vivo Assay

Some compounds of this invention were intranasally instilled at a concentration of 0.5 mg/kg (30µL/mouse, 0.2%Tween80 as vehicle) in C57BL6/J female mice.

Lung-to-plasma ratio (L/P) was calculated as ratio between lung and plasma (i.e. systemic) maximum concentrations obtained after the instillation.
L/P represents the extent of compound repartitioning among lung and plasma;
L/P ratio of 1 corresponds to equal lung and plasma;
L/P>1 indicates higher lung exposure compared to the plasma one.

The results for some compounds are provided below in Table 12.

**Table 12**

| **Example No** | **L/P (Cmax)** |
|---|---|
| 1 | 388 |
| 17 | 643 |

As it can be appreciated, the compounds of Table 12 show an high lung exposure compared to the plasma one, indicating a good inhalatory profile associated with a low systemic exposure.

## Claims

1. A compound of formula (I) 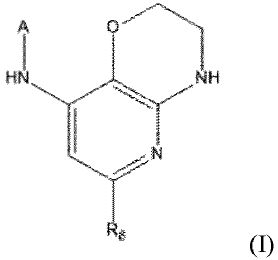 wherein
**A** is selected from the group consisting of **A1** and **A2** 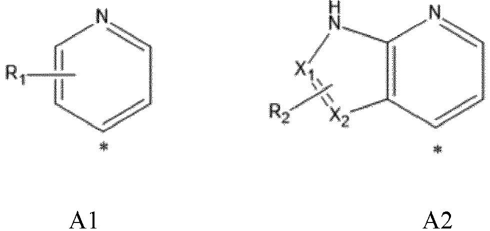
**X₁** and **X₂** are independently C or N;
**R₁** is H or is selected from the group consisting of -NR₃R₄, -C(O)NR₃R₅, - NR₃C(O)R₆ and -C(O)OR₇;
**R₂** is H or is selected from the group consisting of -C(O)OR₃ and -C(O)NR₃R₆; or **R₂** is absent when X₁ or X₂ is N;
**R₃** is H or -(C₁-C₆)alkyl;
**R₄** is H or -(C₁-C₆)alkyl;
**R₅** is H or is selected from the group consisting of -(C₁-C₆)alkyl, -(C₁₋C₆)alkylene-NR_{A}R_{B}, cycloalkyl, heterocycloalkyl optionally substituted by one or more -(C₁-C₆)alkyl, and -(C₁₋C₆)alkylene-heterocycloalkyl, wherein said heterocycloalkyl is optionally substituted by one or more groups selected from -(C₁-C₆)alkyl, -C(O)-(C₁₋C₆)alkyl and -SO₂-(C₁₋C₆)alkyl;
**R₆** is selected from the group consisting of -(C₁₋C₆)alkylene-NR₃R₄ and -(C₁₋C₆)alkylene-heterocycloalkyl, wherein said heterocycloalkyl is optionally substituted by one or more -(C₁-C₆)alkyl;
**R₇** is H or is selected from the group consisting of -(C₁-C₆)alkyl, -(C₁₋C₆)alkylene-NR₃R₄ and -(C₁₋C₆)alkylene-heterocycloalkyl, wherein said heterocycloalkyl is optionally substituted by one or more -(C₁-C₆)alkyl;
**R_{A}** is H or is selected from the group consisting of -(C₁₋C₆)alkyl and -(C₁-C₆)hydroxyalkyl;
**R_{B}** is selected from the group consisting of -(C₁-C₆)alkyl, -(C₁₋C₆)alkylene-C(O)O-(C₁₋C₆)alkyl, heterocycloalkyl and -(C₁-C₆)hydroxyalkyl;
**R₈** is selected from the group consisting of aryl and heteroaryl, wherein said aryl is optionally substituted by one or more groups selected from halogen atoms, -(C₁₋C₆)alkyl and -(C₁-C₄)haloalkyl, and said heteroaryl is optionally substituted by one or more groups selected from -(C₁-C₆)alkyl, halogen atoms and -O-(C₁-C₆)alkyl;
and pharmaceutically acceptable salts thereof.

2. The compound of formula (I) according to claim 1, wherein **A** is group **A1** 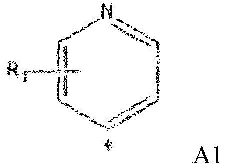 represented by the formula (Ia) 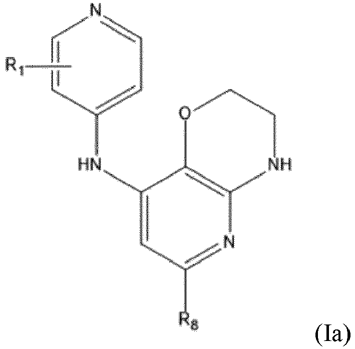
**R₁** is H or is selected from the group consisting of -NR₃R₄, -C(O)NR₃R₅, - NR₃C(O)R₆ and -C(O)OR₇;
**R₃** is H or -(C₁-C₆)alkyl;
**R₄** is H or -(C₁-C₆)alkyl;
**R₅** is H or is selected from the group consisting of -(C₁-C₆)alkyl, -(C₁₋ C₆)alkylene-NR_{A}R_{B}, cycloalkyl, heterocycloalkyl optionally substituted by a - (C₁₋C₆)alkyl, and -(C₁₋C₆)alkylene-heterocycloalkyl, wherein said heterocycloalkyl is optionally substituted by a group selected from -(C₁-C₆)alkyl, -C(O)-(C₁₋C₆)alkyl and -SO₂-(C₁₋C₆)alkyl;
**R₆** is selected from the group consisting of -(C₁₋C₆)alkylene-NR₃R₄ and -(C₁₋ C₆)alkylene-heterocycloalkyl, wherein said heterocycloalkyl is optionally substituted by a -(C₁-C₆)alkyl;
**R₇** is H or is selected from the group consisting of -(C₁-C₆)alkyl, -(C₁₋ C₆)alkylene-NR₃R₄ and -(C₁₋C₆)alkylene-heterocycloalkyl, wherein said heterocycloalkyl is optionally substituted by a -(C₁-C₆)alkyl;
**R_{A}** is H or is selected from the group consisting of -(C₁₋C₆)alkyl and -(C₁-C₆)hydroxyalkyl;
**R_{B}** is selected from the group consisting of -(C₁-C₆)alkyl, -(C₁₋C₆)alkylene-C(O)O-(C₁₋C₆)alkyl, heterocycloalkyl and -(C₁-C₆)hydroxyalkyl;
**R₈** is selected from the group consisting of aryl and heteroaryl, wherein said aryl is optionally substituted by one or more groups selected from halogen atoms, -(C₁₋C₆)alkyl and -(C₁-C₄)haloalkyl, and said heteroaryl is optionally substituted by one or more groups selected from -(C₁-C₆)alkyl, halogen atoms and -O-(C₁-C₆)alkyl;
and pharmaceutically acceptable salts thereof.

3. The compound of formula (Ia) according to claim 2 selected from at least one of:
N-[6-(5-chloro-2-fluorophenyl)-2H,3H,4H-pyrido[3,2-b][1,4]oxazin-8-yl]pyridin-4-amine;
4- f [6-(5-chloro-2-fluorophenyl)-2H,3H,4H-pyrido[3,2-b][1,4]oxazin-8-yl]amino}-N-methylpyridine-3-carboxamide;
N4-[6-(5-chloro-2-fluorophenyl)-2H,3H,4H-pyrido[3,2-b][1,4]oxazin-8-yl]pyridine-2,4-diamine;
N-[6-(4-methyl-1,3-thiazol-2-yl)-2H,3H,4H-pyrido[3,2-b][1,4]oxazin-8-yl]pyridin-4-amine;
N-[6-(6-methylpyridin-2-yl)-2H,3H,4H-pyrido[3,2-b][1,4]oxazin-8-yl]pyridin-4-amine;
4- f [6-(5-chloro-2-fluorophenyl)-2H,3H,4H-pyrido[3,2-b][1,4]oxazin-8-yl]amino}-N-methylpyridine-2-carboxamide;
N4-[6-(5-chloro-2-fluorophenyl)-2H,3H,4H-pyrido[3,2-b][1,4]oxazin-8-yl]pyridine-3,4-diamine;
N-[6-(2-fluoro-5-methylphenyl)-2H,3H,4H-pyrido[3,2-b][1,4]oxazin-8-yl]pyridin-4-amine;
4-{[6-(5-chloro-2-fluorophenyl)-2H,3H,4H-pyrido[3,2-b][1,4]oxazin-8-yl]amino}-N-[2-(morpholin-4-yl)ethyl]pyridine-3-carboxamide;
4-{[6-(5-chloro-2-fluorophenyl)-2H,3H,4H-pyrido[3,2-b][1,4]oxazin-8-yl]amino}-N-[2-(dimethylamino)ethyl]pyridine-3-carboxamide;
N-[6-(2-fluorophenyl)-2H,3H,4H-pyrido[3,2-b][1,4]oxazin-8-yl]pyridin-4-amine;
4-{[6-(5-chloro-2-fluorophenyl)-2H,3H,4H-pyrido[3,2-b][1,4]oxazin-8-yl]amino}-N-[2-(piperazin-1-yl)ethyl]pyridine-3-carboxamide;
N-[6-(6-chloropyridin-2-yl)-2H,3H,4H-pyrido[3,2-b][1,4]oxazin-8-yl]pyridin-4-amine;
N-[6-(2,5-difluorophenyl)-2H,3H,4H-pyrido[3,2-b][1,4]oxazin-8-yl]pyridin-4-amine;
N-[6-(3-chlorophenyl)-2H,3H,4H-pyrido[3,2-b][1,4]oxazin-8-yl]pyridin-4-amine;
4-{[6-(5-chloro-2-fluorophenyl)-2H,3H,4H-pyrido[3,2-b][1,4]oxazin-8-yl]amino}pyridine-3-carboxylate;
methyl 4-{[6-(5-chloro-2-fluorophenyl)-2H,3H,4H-pyrido[3,2-b][1,4]oxazin-8-yl]amino}pyridine-3-carboxylate;
N-[6-(3-methylphenyl)-2H,3H,4H-pyrido[3,2-b][1,4]oxazin-8-yl]pyridin-4-amine;
N-[6-(3-chloro-4-fluorophenyl)-2H,3H,4H-pyrido[3,2-b][1,4]oxazin-8-yl]pyridin-4-amine;
N-(4-{[6-(5-chloro-2-fluorophenyl)-2H,3H,4H-pyrido[3,2-b][1,4]oxazin-8-yl]amino}pyridin-2-yl)-3-(morpholin-4-yl)propenamide;
4-{[6-(5-chloro-2-fluorophenyl)-2H,3H,4H-pyrido[3,2-b][1,4]oxazin-8-yl]amino}-N-[2-(methylamino)ethyl]pyridine-3-carboxamide;
4-{[6-(5-chloro-2-fluorophenyl)-2H,3H,4H-pyrido[3,2-b][1,4]oxazin-8-yl]amino}-N-(propan-2-yl)pyridine-3-carboxamide;
4-{[6-(5-chloro-2-fluorophenyl)-2H,3H,4H-pyrido[3,2-b][1,4]oxazin-8-yl]amino}-N-cyclopropylpyridine-3-carboxamide;
4-{[6-(5-chloro-2-fluorophenyl)-2H,3H,4H-pyrido[3,2-b][1,4]oxazin-8-yl]amino}-N-(piperidin-4-yl)pyridine-3-carboxamide;
N-[6-(5-fluoropyridin-2-yl)-2H,3H,4H-pyrido[3,2-b][1,4]oxazin-8-yl]pyridin-4-amine;
2-(dimethylamino)ethyl 4-{ [6-(5-chloro-2-fluorophenyl)-2H,3H,4H-pyrido[3,2-b][1,4]oxazin-8-yl]amino}pyridine-3-carboxylate;
4-{[6-(5-chloro-2-fluorophenyl)-2H,3H,4H-pyrido[3,2-b][1,4]oxazin-8-yl]amino}-N-[2-(4-methylpiperazin-1-yl)ethyl]pyridine-3-carboxamide;
2-(4-methylpiperazin-1-yl)ethyl 4-{[6-(5-chloro-2-fluorophenyl)-2H,3H,4H-pyrido[3,2-b][1,4]oxazin-8-yl]amino}pyridine-3-carboxylate;
methyl 4- f [6-(6-methylpyridin-2-yl)-2H,3H,4H-pyrido[3,2-b][1,4]oxazin-8-yl]amino}pyridine-3-carboxylate;
4-{[6-(5-chloro-2-fluorophenyl)-2H,3H,4H-pyrido[3,2-b][1,4]oxazin-8-yl]amino}-N-(1-methylpiperidin-4-yl)pyridine-3-carboxamide;
N-(4-{[6-(5-chloro-2-fluorophenyl)-2H,3H,4H-pyrido[3,2-b][1,4]oxazin-8-yl]amino}pyridin-2-yl)-3-(4-methylpiperazin-1-yl)propenamide;
N-(4-{[6-(5-chloro-2-fluorophenyl)-2H,3H,4H-pyrido[3,2-b][1,4]oxazin-8-yl]amino}pyridin-2-yl)-3-(dimethylamino)propenamide;
N-{2-[bis(2-hydroxyethyl)amino]ethyl}-4-{[6-(5-chloro-2-fluorophenyl)-2H,3H,4H-pyrido[3,2-b][1,4]oxazin-8-yl]amino}pyridine-3-carboxamide;
4-{[6-(5-chloro-2-fluorophenyl)-2H,3H,4H-pyrido[3,2-b][1,4]oxazin-8-yl]amino}-N-[2-(dimethylamino)ethyl]pyridine-3-carboxamide;
N-(1-methylpiperidin-4-yl)-4-{[6-(6-methylpyridin-2-yl)-2H,3H,4H-pyrido[3,2-b][1,4]oxazin-8-yl]amino}pyridine-3-carboxamide;
4- f [6-(5-chloro-2-fluorophenyl)-2H,3H,4H-pyrido[3,2-b][1,4]oxazin-8-yl]amino}-N-{2-[methyl(oxetan-3-yl)amino]ethyl}pyridine-3-carboxamide;
methyl 2-({2-[(4-{ [6-(5-chloro-2-fluorophenyl)-2H,3H,4H-pyrido[3,2-b][1,4]oxazin-8-yl]amino}pyridin-3-yl)formamido]ethyl}(methyl)amino)acetate;
4-{[6-(5-chloro-2-fluorophenyl)-2H,3H,4H-pyrido[3,2-b][1,4]oxazin-8-yl]amino}-N-[2-(4-methanesulfonylpiperazin-1-yl)ethyl]pyridine-3-carboxamide;
N-[2-(4-acetylpiperazin-1-yl)ethyl]-4-{[6-(5-chloro-2-fluorophenyl)-2H,3H,4H-pyrido[3,2-b][1,4]oxazin-8-yl]amino}pyridine-3-carboxamide.

4. The compound of formula (Ia) according to claim 2, wherein **A** is **A1a** 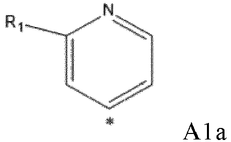 represented by the formula (Iaa) 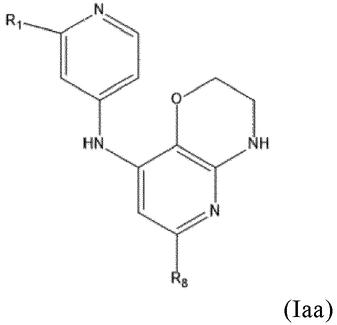
**R₁** is H;
**R₈** is phenyl substituted by fluorine and chlorine.

5. The compound of formula (Ia) according to claim 2, wherein **A** is **A1a** 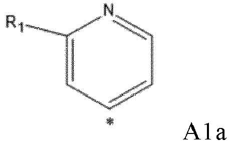 represented by the formula (Iaa) 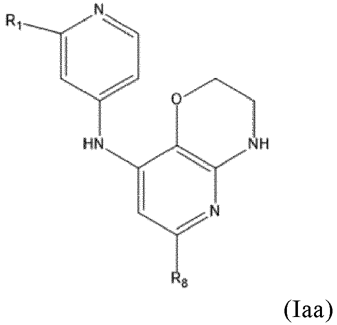
**R₁** is -NHC(O)R₆;
**R₆** is selected from the group consisting of 4-ethyl morpholine, 1-ethyl-4-methylpiperazine and N,N-dimethylethanamine.

6. The compound of formula (Ia) according to claim 2, wherein **A** is **A1b**, represented by the formula (Iab) 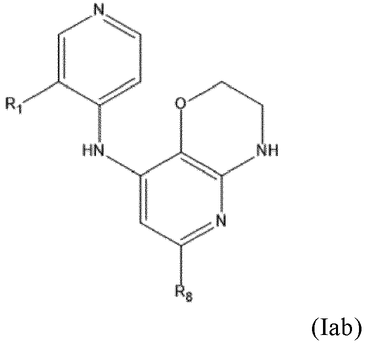
**R₁** is selected from the group consisting of -C(O)NHRs and -C(O)OR₇;
**R₅** is selected from the group consisting of methyl, 2-(methylamino)ethyl, cyclopropyl, 2-(4-methylpiperazin-1-yl)ethyl, 2-(dimethylamino)ethyl, 2-(piperazin-1-yl)ethyl, (piperidin-4-yl), 1-methylpiperidin-4-yl and 2-[bis(2-hydroxyethyl)amino] ethyl;
**R₇** is H or is selected from the group consisting of methyl and 2-(piperazin-1-yl)ethyl.

7. The compound of formula (I) according to claim 1, wherein A is group A2 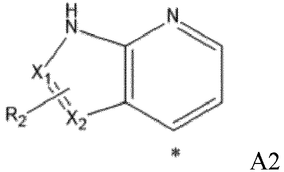 represented by the formula (Ib) 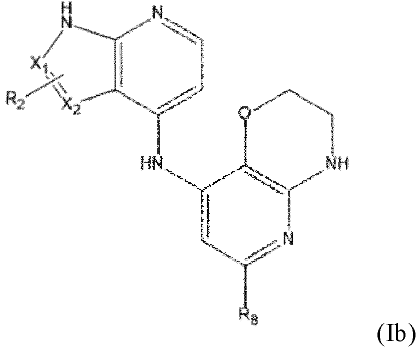
**X₁** and **X₂** are independently C or N;
**R₂** is H or is selected from the group consisting of -C(O)OR₃ and -C(O)NR₃R₆; or **R₂** is absent when X₁ or X₂ is N;
**R₃** is H or -(C₁-C₆)alkyl;
**R₆** is selected from the group consisting of -(C₁₋C₆)alkylene-NR₃R₄ and -(C₁₋ C₆)alkylene-heterocycloalkyl, wherein said heterocycloalkyl is optionally substituted by a -(C₁-C₆)alkyl;
**R₈** is selected from the group consisting of aryl and heteroaryl, wherein said aryl is optionally substituted by one or more groups selected from halogen atoms, -(C₁₋C₆)alkyl and -(C₁-C₄)haloalkyl, and said heteroaryl is optionally substituted by one or more groups selected from -(C₁-C₆)alkyl, halogen atoms and -O-(C₁-C₆)alkyl;
and pharmaceutically acceptable salts thereof.

8. The compound of formula (Ib) according to claim 7 selected from at least one of:
6-(5-chloro-2-fluorophenyl)-N-{1H-pyrazolo[3,4-b]pyridin-4-yl}-2H,3H,4H-pyrido[3,2-b][1,4]oxazin-8-amine;
6-(5-chloro-2-fluorophenyl)-N-{1H-pyrrolo[2,3-b]pyridin-4-yl}-2H,3H,4H-pyrido[3,2-b][1,4]oxazin-8-amine;
6-(5-chloro-2-fluorophenyl)-N-{3H-imidazo[4,5-b]pyridin-7-yl}-2H,3H,4H-pyrido[3,2-b][1,4]oxazin-8-amine;
methyl 4-{[6-(5-chloro-2-fluorophenyl)-2H,3H,4H-pyrido[3,2-b][1,4]oxazin-8-yl]amino}-1H-pyrrolo[2,3-b]pyridine-3-carboxylate;
methyl 4-{[6-(5-chloro-2-fluorophenyl)-2H,3H,4H-pyrido[3,2-b][1,4]oxazin-8-yl]amino}-1H-pyrrolo[2,3-b]pyridine-2-carboxylate;
N-{3H-imidazo[4,5-b]pyridin-7-yl}-6-(6-methylpyridin-2-yl)-2H,3H,4H-pyrido[3,2-b][1,4]oxazin-8-amine;
6-(6-methylpyridin-2-yl)-N-{1H-pyrazolo[3,4-b]pyridin-4-yl}-2H,3H,4H-pyrido[3,2-b][1,4]oxazin-8-amine;
6-(6-methylpyridin-2-yl)-N-{1H-pyrrolo[2,3-b]pyridin-4-yl}-2H,3H,4H-pyrido[3,2-b][1,4]oxazin-8-amine;
4-{[6-(5-chloro-2-fluorophenyl)-2H,3H,4H-pyrido[3,2-b][1,4]oxazin-8-yl]amino}-N-[2-(1-methylpiperidin-4-yl)ethyl]-1H-pyrrolo[2,3-b]pyridine-2-carboxamide;
4-{[6-(5-chloro-2-fluorophenyl)-2H,3H,4H-pyrido[3,2-b][1,4]oxazin-8-yl]amino}-N-[2-(dimethylamino)ethyl]-1H-pyrrolo[2,3-b]pyridine-2-carboxamide.

9. A pharmaceutical composition comprising a compound of formula (I) according to any one of claims 1 to 8, in admixture with one or more pharmaceutically acceptable carrier or excipient.

10. The pharmaceutical composition according to claim 9 for administration by inhalation.

11. A compound of formula (I) according to any one of claims 1 to 8 or a pharmaceutical composition according to claims 9 and 10 for use as a medicament.

12. A compound of formula (I) or a pharmaceutical composition for use according to claim 11 in the prevention and/or treatment of a disease, disorder or condition mediated by ALK5 signaling pathway in a mammal.

13. A compound of formula (I) or a pharmaceutical composition for use according to claims 11 and 12 in the prevention and/or treatment of fibrosis and/or diseases, disorders or conditions that involve fibrosis.

14. A compound of formula (I) or a pharmaceutical composition for use according to claim 13 in the prevention and/or treatment of fibrosis including pulmonary fibrosis, idiopathic pulmonary fibrosis (IPF), hepatic fibrosis, renal fibrosis, ocular fibrosis, cardiac fibrosis, arterial fibrosis and systemic sclerosis.

15. A compound of formula (I) or a pharmaceutical composition for use according to claim 14 in the prevention and/or treatment idiopathic pulmonary fibrosis (IPF).

## Patentansprüche

1. Eine Verbindung der Formel (I) 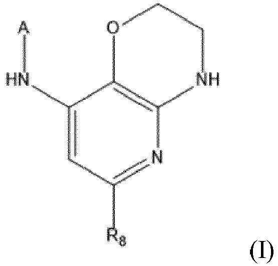 wobei
**A** ausgewählt ist aus der Gruppe bestehend aus **A1** und **A2** 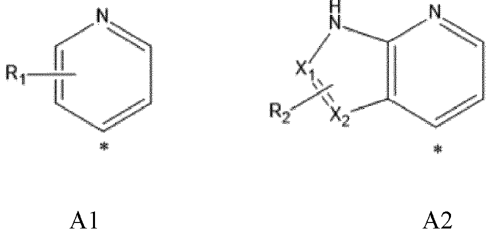
**X₁** und **X₂** unabhängig voneinander C oder N sind;
**R₁** H ist oder ausgewählt ist aus der Gruppe bestehend aus -NR₃R₄, - C(O)NR₃R₅, -NR₃C(O)R₆ und -C(O)OR₇;
**R₂** H ist oder ausgewählt ist aus der Gruppe bestehend aus -C(O)OR₃ und - C(O)NR₃R₆; oder **R₂** fehlt, wenn X₁ oder X₂ N ist;
**R₃ H** oder -(C₁-C₆)Alkyl ist;
**R₄ H** oder -(C₁-C₆)Alkyl ist;
**R₅** H ist oder ausgewählt ist aus der Gruppe bestehend aus -(C₁-C₆)Alkyl, - (C₁-C₆)Alkylen-NR_{A}R_{B}, Cycloalkyl, Heterocycloalkyl, optional substituiert durch ein oder mehrere -(C₁-C₆)Alkyle und -(C₁-C₆)Alkylen-Heterocycloalkyl, wobei das Heterocycloalkyl optional durch eine oder mehrere Gruppen substituiert ist, die ausgewählt sind aus -(C₁-C₆)Alkyl, - C(O)-(C₁-C₆)Alkyl und -SO₂-(C₁-C₆)Alkyl;
**R₆** ausgewählt ist aus der Gruppe bestehend aus -(C₁-C₆)Alkylen-NR₃R₄ und -(C₁-C₆)Alkylen-Heterocycloalkyl, wobei das Heterocycloalkyl optional durch ein oder mehrere -(C₁-C₆)Alkyle substituiert ist;
**R₇** H ist oder ausgewählt ist aus der Gruppe bestehend aus -(C₁-C₆)Alkyl, - (C₁-C₆)Alkylen-NR₃R₄ und -(C₁-C₆)Alkylen-Heterocycloalkyl, wobei das Heterocycloalkyl optional durch ein oder mehrere -(C₁-C₆)Alkyle substituiert ist;
**R_{A}** H ist oder ausgewählt ist aus der Gruppe bestehend aus -(C₁-C₆)Alkyl und -(C₁-C₆)Hydroxyalkyl;
**R_{B}** ausgewählt ist aus der Gruppe bestehend aus -(C₁-C₆)Alkyl, -(C₁-C₆)Alkylen-C(O)O-(C₁-C₆)Alkyl, Heterocycloalkyl und -(C₁-C₆)Hydroxyalkyl;
**R₈** ausgewählt ist aus der Gruppe bestehend aus Aryl und Heteroaryl, wobei das Aryl optional durch eine oder mehrere Gruppen substituiert ist, die aus Halogenatomen, -(C₁-C₆)Alkyl und -(C₁-C₄)Haloalkyl ausgewählt sind, und das Heteroaryl optional durch eine oder mehrere Gruppen substituiert ist, die aus -(C₁-C₆)Alkyl, Halogenatomen und -O-(C₁-C₆)Alkyl ausgewählt sind; und pharmazeutisch akzeptable Salze davon.

2. Die Verbindung der Formel (I) nach Anspruch 1, wobei **A** die Gruppe A1 ist 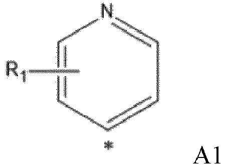 durch die Formel (Ia) dargestellt 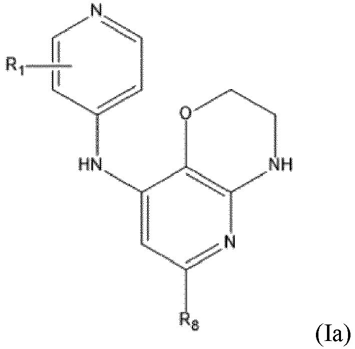
**R₁** H ist oder ausgewählt ist aus der Gruppe bestehend aus -NR₃R₄, - C(O)NR₃R₅, -NR₃C(O)R₆ und -C(O)OR₇;
**R₃** H oder -(C₁-C₆)Alkyl ist;
**R₄** H oder -(C₁-C₆)Alkyl ist;
**R₅** H ist oder ausgewählt ist aus der Gruppe bestehend aus -(C₁-C₆)Alkyl, - (C₁₋C₆)Alkylen-NR_{A}R_{B}, Cycloalkyl, Heterocycloalkyl, optional substituiert durch ein -(C₁-C₆)Alkyl und -(C₁-C₆)Alkylen-Heterocycloalkyl, wobei das Heterocycloalkyl optional substituiert ist durch eine Gruppe, die aus -(C₁-C₆)Alkyl, -C(O)-(C₁-C₆)Alkyl und -SO₂-(C₁-C₆)Alkyl ausgewählt ist;
**R₆** ausgewählt ist aus der Gruppe bestehend aus -(C₁-C₆)Alkylen-NR₃R₄ und -(C₁-C₆)Alkylen-Heterocycloalkyl, wobei das Heterocycloalkyl optional durch ein -(C₁-C₆)Alkyl substituiert ist;
**R₇** H ist oder ausgewählt ist aus der Gruppe bestehend aus -(C₁-C₆)Alkyl, - (C₁-C₆)Alkylen-NR₃R₄ und -(C₁-C₆)Alkylen-Heterocycloalkyl, wobei das Heterocycloalkyl optional durch ein -(C₁-C₆)Alkyl substituiert ist;
**R_{A}** H ist oder ausgewählt ist aus der Gruppe bestehend aus -(C₁-C₆)Alkyl und -(C₁-C₆)Hydroxyalkyl;
**R_{B}** ausgewählt ist aus der Gruppe bestehend aus -(C₁-C₆)Alkyl, -(C₁-C₆)Alkylen-C(O)O-(C₁-C₆)Alkyl, Heterocycloalkyl und -(C₁-C₆)Hydroxyalkyl;
**R₈** ausgewählt ist aus der Gruppe bestehend aus Aryl und Heteroaryl, wobei das Aryl optional durch eine oder mehrere Gruppen substituiert ist, die aus Halogenatomen, -(C₁-C₆)Alkyl und -(C₁-C₄)Haloalkyl ausgewählt sind, und das Heteroaryl optional durch eine oder mehrere Gruppen substituiert ist, die aus -(C₁-C₆)Alkyl, Halogenatomen und -O-(C₁-C₆)Alkyl ausgewählt sind; und pharmazeutisch akzeptable Salze davon.

3. Die Verbindung der Formel (Ia) nach Anspruch 2, ausgewählt aus mindestens einer der folgenden Verbindungen:
N-[6-(5-Chlor-2-fluorphenyl)-2H,3H,4H-pyrido[3,2-b][1,4]oxazin-8-yl]pyridin-4-amin;
4- f [6-(5-Chlor-2-fluorphenyl)-2H,3H,4H-pyrido[3,2-b][1,4]oxazin-8-yl]amino}-N-methylpyridin-3-carboxamid;
N-[6-(5-Chlor-2-fluorphenyl)-2H,3H,4H-pyrido[3,2-b][1,4]oxazin-8-yl]pyridin-2,4-diamin;
N-[6-(4-Methyl-1,3-thiazol-2-yl)-2H,3H,4H-pyrido[3,2-b][1,4]oxazin-8-yl]pyridin-4-amin;
N-[6-(6-Methylpyridin-2-yl)-2H,3H,4H-pyrido[3,2-b][1,4]oxazin-8-yl]pyridin-4-amin;
4-{[6-(5-Chlor-2-fluorphenyl)-2H,3H,4H-pyrido[3,2-b][1,4]oxazin-8-yl]amino}-N-methylpyridin-2-carboxamid;
N4-[6-(5-Chlor-2-fluorphenyl)-2H,3H,4H-pyrido[3,2-b][1,4]oxazin-8-yl]pyridin-3,4-diamin;
N-[6-(2-Fluoro-5-methylphenyl)-2H,3H,4H-pyrido[3,2-b][1,4]oxazin-8-yl]pyridin-4-amin;
4-{[6-(5-Chlor-2-fluorphenyl)-2H,3H,4H-pyrido[3,2-b][1,4]oxazin-8-yl]amino}-N-[2-(morpholin-4-yl)ethyl]pyridin-3-carboxamid;
4-{[6-(5-Chlor-2-fluorphenyl)-2H,3H,4H-pyrido[3,2-b][1,4]oxazin-8-yl]amino}-N-[2-(dimethylamino)ethyl]pyridin-3-carboxamid;
N-[6-(2-Fluorphenyl)-2H,3H,4H-pyrido[3,2-b][1,4]oxazin-8-yl]pyridin-4-amin;
4-{[6-(5-Chlor-2-fluorphenyl)-2H,3H,4H-pyrido[3,2-b][1,4]oxazin-8-yl]amino}-N-[2-(piperazin-1-yl)ethyl]pyridin-3-carboxamid;
N-[6-(6-Chloropyridin-2-yl)-2H,3H,4H-pyrido[3,2-b][1,4]oxazin-8-yl]pyridin-4-amin;
N-[6-(2,5-Difluorphenyl)-2H,3H,4H-pyrido[3,2-b][1,4]Oxazin-8-yl]pyridin-4-amin;
N-[6-(3-Chlorphenyl)-2H,3H,4H-pyrido[3,2-b][1,4]oxazin-8-yl]pyridin-4-amin;
4-{[6-(5-Chlor-2-fluorphenyl)-2H,3H,4H-pyrido[3,2-b][1,4]oxazin-8-yl]amino}pyridin-3-carboxylat;
Methyl-4-{[6-(5-Chlor-2-fluorphenyl)-2H,3H,4H-pyrido[3,2-b][1,4]oxazin-8-yl]amino}pyridin-3-carboxylat;
N-[6-(3-Methylphenyl)-2H,3H,4H-pyrido[3,2-b][1,4]oxazin-8-yl]pyridin-4-amin;
N-[6-(3-Chlor-4-fluorphenyl)-2H,3H,4H-pyrido[3,2-b][1,4]oxazin-8-yl]pyridin-4-amin;
N-(4-{[6-(5-Chlor-2-fluorphenyl)-2H,3H,4H-pyrido[3,2-b][1,4]oxazin-8-yl]amino}pyridin-2-yl)-3-(morpholin-4-yl)propenamid;
4-{[6-(5-Chlor-2-fluorphenyl)-2H,3H,4H-pyrido[3,2-b][1,4]oxazin-8-yl]amino}-N-[2-(methylamino)ethyl]pyridin-3-carboxamid;
4-{[6-(5-Chlor-2-fluorphenyl)-2H,3H,4H-pyrido[3,2-b][1,4]oxazin-8-yl]amino}-N-(propan-2-yl)pyridin-3-carboxamid;
4-{[6-(5-Chlor-2-fluorphenyl)-2H,3H,4H-pyrido[3,2-b][1,4]oxazin-8-yl]amino}-N-cyclopropylpyridin-3-carboxamid;
4-{[6-(5-Chlor-2-fluorphenyl)-2H,3H,4H-pyrido[3,2-b][1,4]oxazin-8-yl]amino}-N-(piperidin-4-yl)pyridin-3-carboxamid;
N-[6-(5-Fluorpyridin-2-yl)-2H,3H,4H-pyrido[3,2-b][1,4]oxazin-8-yl]pyridin-4-amin;
2-(Dimethylamino)ethyl-4-{[6-(5-chlor-2-fluorphenyl)-2H,3H,4H-pyrido[3,2-b][1,4]oxazin-8-yl]amino}pyridin-3-carboxylat;
4-{[6-(5-Chlor-2-fluorphenyl)-2H,3H,4H-pyrido[3,2-b][1,4]oxazin-8-yl]amino}-N-[2-(4-methylpiperazin-1-yl)ethyl]pyridin-3-carboxamid;
2-(4-Methylpiperazin-1-yl)ethyl-4-{[6-(5-chlor-2-fluorphenyl)-2H,3H,4H-pyrido[3,2-b][1,4]oxazin-8-yl]amino}pyridin-3-carboxylat;
Methyl-4-{[6-(6-methylpyridin-2-yl)-2H,3H,4H-pyrido[3,2-b][1,4]oxazin-8-yl]amino}pyridin-3-carboxylat;
4-{[6-(5-Chlor-2-fluorphenyl)-2H,3H,4H-pyrido[3,2-b][1,4]oxazin-8-yl]amino}-N-(1-methylpiperidin-4-yl)pyridin-3-carboxamid;
N-(4-{[6-(5-Chlor-2-fluorphenyl)-2H,3H,4H-pyrido[3,2-b][1,4]oxazin-8-yl]amino}pyridin-2-yl)-3-(4-methylpiperazin-1-yl)propenamid;
N-(4-{[6-(5-Chlor-2-fluorphenyl)-2H,3H,4H-pyrido[3,2-b][1,4]oxazin-8-yl]amino}pyridin-2-yl)-3-(dimethylamino)propenamid;
N-{2-[Bis(2-hydroxyethyl)amino]ethyl}-4-{[6-(5-chlor-2-fluorphenyl)-2H,3H,4H-pyrido[3,2-b][1,4]oxazin-8-yl]amino}pyridin-3-carboxamid;
4-{[6-(5-Chlor-2-fluorphenyl)-2H,3H,4H-pyrido[3,2-b][1,4]oxazin-8-yl]amino}-N-[2-(dimethylamino)ethyl]pyridin-3-carboxamid;
N-(1-Methylpiperidin-4-yl)-4-{[6-(6-methylpyridin-2-yl)-2H,3H,4H-pyrido[3,2-b][1,4]oxazin-8-yl]amino}pyridin-3-carboxamid;
4-{[6-(5-Chlor-2-fluorphenyl)-2H,3H,4H-pyrido[3,2-b][1,4]oxazin-8-yl]amino}-N-{2-[methyl(oxetan-3-yl)amino]ethyl}pyridin-3-carboxamid;
Methyl-2-({2-[(4-{[6-(5-Chlor-2-fluorphenyl)-2H,3H,4H-pyrido[3,2-b][1,4]oxazin-8-yl]amino}pyridin-3-yl)formamido]ethyl}(methyl)amino)acetat;
4-{[6-(5-Chlor-2-fluorphenyl)-2H,3H,4H-pyrido[3,2-b][1,4]oxazin-8-yl]amino}-N-[2-(4-methansulfonylpiperazin-1-yl)ethyl]pyridin-3-carboxamid;
N-[2-(4-Acetylpiperazin-1-yl)ethyl]-4-{ [6-(5-chlor-2-fluorphenyl)-2H,3H,4H-pyrido[3,2-b][1,4]oxazin-8-yl]amino}pyridin-3-carboxamid;

4. Die Verbindung der Formel (Ia) nach Anspruch 2, wobei **A** gleich **A1a** 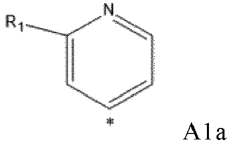 durch die Formel (Iaa) dargestellt 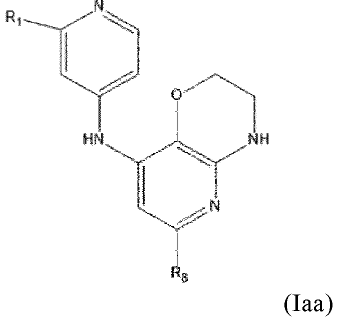
**R₁** H ist;
**R₈** durch Fluor und Chlor substituiertes Phenyl ist.

5. Die Verbindung der Formel (Ia) nach Anspruch 2, wobei **A** gleich **A1a** 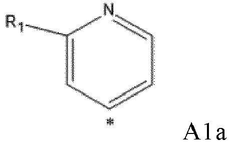 durch die Formel (Iaa) dargestellt 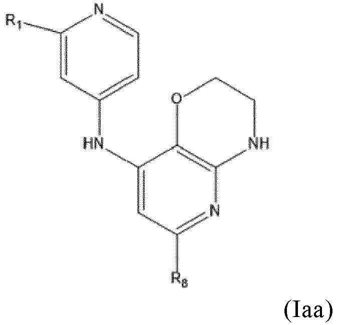
**R₁** -NHC(O)R₆ ist;
**R₆** ausgewählt ist aus der Gruppe bestehend aus 4-Ethylmorpholin, 1-Ethyl-4-methylpiperazin und N,N-Dimethylethanamin.

6. Die Verbindung der Formel (Ia) nach Anspruch 2, wobei **A** gleich **A1b**, dargestellt durch die Formel (Iab) 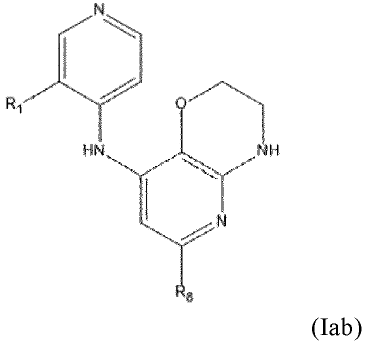
**R₁** ausgewählt ist aus der Gruppe bestehend aus -C(O)NHRs und -C(O)OR₇;
**R₅** ausgewählt ist aus der Gruppe bestehend aus Methyl, 2-(Methylamino)ethyl, Cyclopropyl, 2-(4-Methylpiperazin-1-yl)ethyl, 2-(Dimethylamino)ethyl, 2-(Piperazin-1-yl)ethyl, (Piperidin-4-yl), 1-Methylpiperidin-4-yl und 2-[Bis(2-hydroxyethyl)amino]ethyl;
**R₇** H ist oder ausgewählt ist aus der Gruppe bestehend aus Methyl und 2-(Piperazin-1-yl)ethyl.

7. Die Verbindung der Formel (I) nach Anspruch 1, wobei A die Gruppe A2 ist 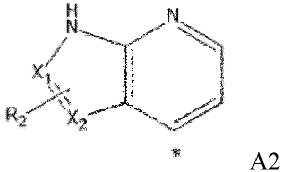 durch die Formel (Ib) dargestellt 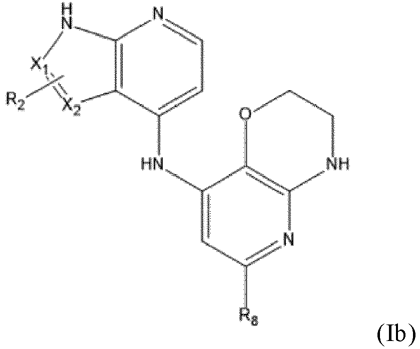
**X₁** und **X₂** unabhängig voneinander C oder N sind;
**R₂** H ist oder ausgewählt ist aus der Gruppe bestehend aus -C(O)OR₃ und - C(O)NR₃R₆; oder **R₂** fehlt, wenn X₁ oder X₂ N ist;
**R₃ H** oder -(C₁-C₆)Alkyl ist;
**R₆** ausgewählt ist aus der Gruppe bestehend aus -(C₁-C₆)Alkylen-NR₃R₄ und -(C₁-C₆)Alkylen-Heterocycloalkyl, wobei das Heterocycloalkyl optional durch ein -(C₁-C₆)Alkyl substituiert ist;
**R₈** ausgewählt ist aus der Gruppe bestehend aus Aryl und Heteroaryl, wobei das Aryl optional durch eine oder mehrere Gruppen substituiert ist, die aus Halogenatomen, -(C₁-C₆)Alkyl und -(C₁-C₄)Haloalkyl ausgewählt sind, und das Heteroaryl optional durch eine oder mehrere Gruppen substituiert ist, die aus -(C₁-C₆)Alkyl, Halogenatomen und -O-(C₁-C₆)Alkyl ausgewählt sind; und pharmazeutisch akzeptable Salze davon.

8. Die Verbindung der Formel (Ib) nach Anspruch 2, ausgewählt aus mindestens einer der folgenden Verbindungen:
6-(5-Chlor-2-fluorphenyl)-N-{1H-pyrazolo[3,4-b]pyridin-4-yl}-2H,3H,4H-pyrido[3,2-b][1,4]oxazin-8-amin;
6-(5-Chlor-2-fluorphenyl)-N-{1H-pyrrolo[2,3-b]pyridin-4-yl}-2H,3H,4H-pyrido[3,2-b][1,4]oxazin-8-amin;
6-(5-Chlor-2-fluorphenyl)-N-{3H-imidazo[4,5-b]pyridin-7-yl}-2H,3H,4H-pyrido[3,2-b][1,4]oxazin-8-amin;
Methyl -4-{[6-(5-Chl or-2-fluorphenyl)-2H, 3H,4H-pyrido[3,2-b] [1,4]oxazin-8-yl]amino}-1H-pyrrolo[2,3-b]pyridin-3-carboxylat;
Methyl-4-{[6-(5-Chlor-2-fluorphenyl)-2H,3H,4H-pyrido[3,2-b][1,4]oxazin-8-yl]amino}-1H-pyrrolo[2,3-b]pyridin-2-carboxylat;
N-{3H-Imidazo[4,5-b]pyridin-7-yl}-6-(6-methylpyridin-2-yl)-2H,3H,4H-pyrido[3,2-b][1,4]oxazin-8-amin;
6-(6-Methylpyridin-2-yl)-N-{1H-pyrazolo[3,4-b]pyridin-4-yl}-2H,3H,4H-pyrido[3,2-b][1,4]oxazin-8-amin;
6-(6-Methylpyridin-2-yl)-N-{1H-pyrrolo[2,3-b]pyridin-4-yl}-2H,3H,4H-pyrido[3,2-b][1,4]oxazin-8-amin;
4- f [6-(5-Chlor-2-fluorphenyl)-2H,3H,4H-pyrido[3,2-b][1,4]oxazin-8-yl]amino}-N-[2-(1-methylpiperidin-4-yl)ethyl]-1H-pyrrolo[2,3-b]pyridin-2-carboxamid;
4- f [6-(5-Chlor-2-fluorphenyl)-2H,3H,4H-pyrido[3,2-b][1,4]oxazin-8-yl]amino}-N-[2-(dimethylamino)ethyl]-1H-pyrrolo[2,3-b]pyridin-2-carboxamid;

9. Pharmazeutische Zusammensetzung, die eine Verbindung der Formel (I) umfasst, nach einem der Ansprüche 1 bis 8, unter Beigabe eines oder mehrerer pharmazeutisch annehmbarer Träger oder Hilfsstoffe.

10. Pharmazeutische Zusammensetzung nach Anspruch 9 zur Verabreichung durch Inhalation.

11. Verbindung der Formel (I) gemäß einem der Ansprüche 1 bis 8 oder pharmazeutische Zusammensetzung gemäß den Ansprüchen 9 und 10 zur Verwendung als Arzneimittel.

12. Verbindung der Formel (I) oder pharmazeutische Zusammensetzung zur Verwendung gemäß Anspruch 11 bei der Prävention und/oder Behandlung von Krankheiten, Störungen oder Zuständen, die durch den ALKS-Signalweg in einem Säugetier vermittelt werden.

13. Verbindung der Formel (I) oder pharmazeutische Zusammensetzung zur Verwendung gemäß den Ansprüchen 11 und 12 bei der Prävention und/oder Behandlung von Fibrose und/oder Krankheiten, Störungen oder Zuständen, die Fibrose beinhalten.

14. Verbindung der Formel (I) oder pharmazeutische Zusammensetzung zur Verwendung gemäß Anspruch 13 bei der Prävention und/oder Behandlung von Fibrose, einschließlich Lungenfibrose, idiopathischer pulmonaler Fibrose (IPF), Leberfibrose, Nierenfibrose, Augenfibrose, Herzfibrose, arterieller Fibrose und systemischer Sklerose.

15. Verbindung der Formel (I) oder pharmazeutische Zusammensetzung zur Verwendung gemäß Anspruch 14 bei der Prävention und/oder Behandlung von idiopathischer pulmonaler Fibrose (IPF).

## Revendications

1. Composé de formule (I) 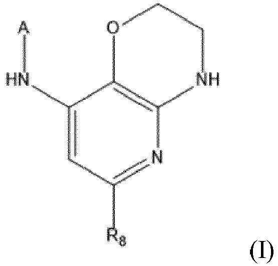 dans lequel
**A** est sélectionné dans le groupe constitué de **A1** et **A2** 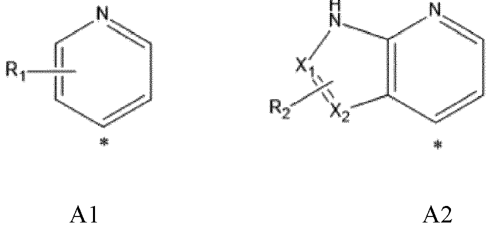
**X₁** et **X₂** sont indépendamment C ou N ;
**R₁** est H ou est sélectionné dans le groupe constitué de -NR₃R₄, -C(O)NR₃R₅, -NR₃C(O)R₆ et -C(O)OR₇ ;
**R₂** est H ou est sélectionné dans le groupe constitué de -C(O)OR₃ et - C(O)NR₃R₆ ; ou **R₂** est absent lorsque X₁ ou X₂ est N ;
**R₃** est H ou alkyle -(C₁-C₆) ;
**R₄** est H ou alkyle -(C₁-C₆) ;
**R₅** est H ou est sélectionné dans le groupe constitué d'alkyle -(C₁-C₆), alkylène -(C₁₋C₆)-NR_{A}R_{B}, cycloalkyle, hétérocycloalkyle éventuellement substitué par un ou plusieurs alkyles -(C₁₋C₆), et alkylène-hétérocycloalkyle - (C₁₋C₆), dans lequel cet hétérocycloalkyle est éventuellement substitué par un ou plusieurs groupes sélectionnés parmi l'alkyle -(C₁-C₆), l'alkyle -C(O)-(C₁₋ C₆) et l'alkyle -SO₂-(C₁₋C₆) ;
**R₆** est sélectionné dans le groupe constitué d'alkylène -(C₁₋C₆)-NR₃R₄ et alkylène-hétérocycloalkyle -(C₁₋C₆), dans lequel cet hétérocycloalkyle est éventuellement substitué par un ou plusieurs alkyles -(C₁-C₆) ;
**R₇** est H ou est sélectionné dans le groupe constitué d'alkyle -(C₁-C₆), alkylène -(C₁₋C₆)-NR₃R₄ et alkylène-hétérocycloalkyle -(C₁₋C₆), dans lequel cet hétérocycloalkyle est éventuellement substitué par un ou plusieurs alkyles -(C₁-C₆) ;
**R_{A}** est H ou est sélectionné dans le groupe constitué d'alkyle -(C₁₋C₆) et hydroxyalkyle -(C₁-C₆) ;
**R_{B}** est sélectionné dans le groupe constitué d'alkyle -(C₁₋C₆), alkylène -(C₁₋C₆)-alkyle C(O)O-(C₁₋C₆), hétérocycloalkyle et hydroxyalkyle -(C₁-C₆) ;
**R₈** est sélectionné dans le groupe constitué d'aryle et d'hétéroaryle, dans lequel cet aryle est éventuellement substitué par un ou plusieurs groupes sélectionnés parmi les atomes halogènes, alkyle -(C₁-C₆) et haloalkyle -(C₁-C₄), et cet hétéroaryle est éventuellement substitué par un ou plusieurs groupes choisis parmi les alkyles -(C₁-C₆), atomes halogènes et alkyles -O-(C₁-C₆) ; et ses sels acceptables du point de vue pharmaceutique.

2. Composé de formule (I) selon la revendication 1, dans lequel **A** est le groupe **A1** 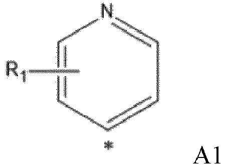 représentée par la formule (Ia) 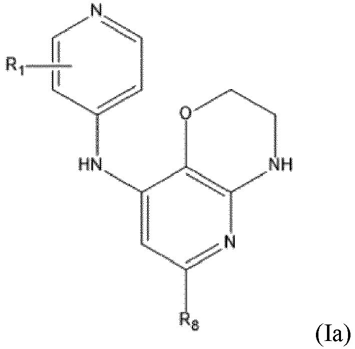
**R₁** est H ou est sélectionné dans le groupe constitué de -NR₃R₄, -C(O)NR₃R₅, -NR₃C(O)R₆ et -C(O)OR₇ ;
**R₃** est H ou alkyle -(C₁-C₆) ;
**R₄** est H ou alkyle -(C₁-C₆) ;
**R₅** est H ou est sélectionné dans le groupe constitué d'alkyle -(C₁-C₆), alkylène -(C₁₋C₆)-NR_{A}R_{B}, cycloalkyle, hétérocycloalkyle éventuellement substitué par un alkyle -(C₁₋C₆), et alkylène-hétérocycloalkyle -(C₁₋C₆), dans lequel cet hétérocycloalkyle est éventuellement substitué par un groupe sélectionné parmi l'alkyle -(C₁-C₆), l'alkyle -C(O)-(C₁₋C₆) et l'alkyle -SO₂-(C₁₋C₆) ;
**R₆** est sélectionné dans le groupe constitué d'alkylène -(C₁₋C₆)-NR₃R₄ et alkylène-hétérocycloalkyle -(C₁₋C₆), dans lequel cet hétérocycloalkyle est éventuellement substitué par un alkyle -(C₁-C₆) ;
**R₇** est H ou est sélectionné dans le groupe constitué d'alkyle -(C₁-C₆), alkylène -(C₁₋C₆)-NR₃R₄ et alkylène-hétérocycloalkyle -(C₁₋C₆), dans lequel cet hétérocycloalkyle est éventuellement substitué par un alkyle -(C₁-C₆) ;
**R_{A}** est H ou est sélectionné dans le groupe constitué d'alkyle -(C₁₋C₆) et hydroxyalkyle -(C₁-C₆) ;
**R_{B}** est sélectionné dans le groupe constitué d'alkyle -(C₁₋C₆), alkylène -(C₁₋C₆)-alkyle C(O)O-(C₁₋C₆), hétérocycloalkyle et hydroxyalkyle -(C₁-C₆) ;
**R₈** est sélectionné dans le groupe constitué d'aryle et d'hétéroaryle, dans lequel cet aryle est éventuellement substitué par un ou plusieurs groupes sélectionnés parmi les atomes halogènes, alkyle -(C₁-C₆) et haloalkyle -(C₁-C₄), et cet hétéroaryle est éventuellement substitué par un ou plusieurs groupes choisis parmi les alkyles -(C₁-C₆), atomes halogènes et alkyles -O-(C₁-C₆) ; et ses sels acceptables du point de vue pharmaceutique.

3. Composé de formule (Ia) selon la revendication 2 choisi parmi au moins l'un des :
N-[6-(5-chloro-2-fluorophényl)-2H,3H,4H-pyrido[3,2-b][1,4]oxazine-8-yl]pyridine-4-amine ;
4-{[6-(5-chloro-2-fluorophényl)-2H,3H,4H-pyrido[3,2-b][1,4]oxazine-8-yl]amino}-N-méthylpyridine-3-carboxamide ;
N4-[6-(5-chloro-2-fluorophényl)-2H,3H,4H-pyrido[3,2-b][1,4]oxazine-8-yl]pyridine-2,4-diamine ;
N-[6-(4-méthyle-1,3-thiazol-2-yl)-2H,3H,4H-pyrido[3,2-b][1,4]oxazine-8-yl]pyridine-4-amine ;
N-[6-(6-méthylpyridine-2-yl)-2H,3H,4H-pyrido[3,2-b][1,4]oxazine-8-yl]pyridine-4-amine ;
4-{[6-(5-chloro-2-fluorophényl)-2H,3H,4H-pyrido[3,2-b][1,4]oxazine-8-yl]amino}-N-méthylpyridine-2-carboxamide ;
N4-[6-(5-chloro-2-fluorophényl)-2H,3H,4H-pyrido[3,2-b][1,4]oxazine-8-yl]pyridine-3,4-diamine ;
N-[6-(2-fluoro-5-méthylphényl)-2H,3H,4H-pyrido[3,2-b][1,4]oxazine-8-yl]pyridine-4-amine ;
4-{[6-(5-chloro-2-fluorophényl)-2H,3H,4H-pyrido[3,2-b][1,4]oxazine-8-yl]amino}-N-[2-(morpholine-4-yl)éthyl]pyridine-3-carboxamide ;
4-{[6-(5-chloro-2-fluorophényl)-2H,3H,4H-pyrido[3,2-b][1,4]oxazine-8-yl]amino}-N-[2-(diméthylamino)éthyle]pyridine-3-carboxamide ;
N-[6-(2-fluorophényl)-2H,3H,4H-pyrido[3,2-b][1,4]oxazine-8-yl]pyridine-4-amine ;
4-{[6-(5-chloro-2-fluorophényl)-2H,3H,4H-pyrido[3,2-b][1,4]oxazine-8-yl]amino}-N-[2-(pipérazine-1-yl)éthyl]pyridine-3-carboxamide ;
N-[6-(6-chloropyridine-2-yl)-2H,3H,4H-pyrido[3,2-b][1,4]oxazine-8-yl]pyridine-4-amine ;
N-[6-(2,5-difluorophényl)-2H,3H,4H-pyrido[3,2-b][1,4]oxazine-8-yl]pyridine-4-amine ;
N-[6-(3-chlorophényl)-2H,3H,4H-pyrido[3,2-b][1,4]oxazine-8-yl]pyridine-4-amine ;
4-{[6-(5-chloro-2-fluorophényl)-2H,3H,4H-pyrido[3,2-b][1,4]oxazine-8-yl]amino}pyridine-3-carboxylate ;
méthyle 4-{ [6-(5-chloro-2-fluorophényl)-2H,3H,4H-pyrido[3,2-b][1,4]oxazine-8-yl]amino}pyridine-3-carboxylate ;
N-[6-(3-méthylphényl)-2H,3H,4H-pyrido[3,2-b][1,4]oxazine-8-yl]pyridine-4-amine ;
N-[6-(3-chloro-4-fluorophényl)-2H,3H,4H-pyrido[3,2-b][1,4]oxazine-8-yl]pyridine-4-amine ;
N-(4-{[6-(5-chloro-2-fluorophényl)-2H,3H,4H-pyrido[3,2-b][1,4]oxazine-8-yl]amino}pyridine-2-yl)-3-(morpholine-4-yl)propanamide ;
4-{[6-(5-chloro-2-fluorophényl)-2H,3H,4H-pyrido[3,2-b][1,4]oxazine-8-yl]amino}-N-[2-(méthylamino)éthyl]pyridine-3-carboxamide ;
4-{[6-(5-chloro-2-fluorophényl)-2H,3H,4H-pyrido[3,2-b][1,4]oxazine-8-yl]amino}-N-(propan-2-yl)pyridine-3-carboxamide ;
4-{[6-(5-chloro-2-fluorophényl)-2H,3H,4H-pyrido[3,2-b][1,4]oxazine-8-yl]amino}-N-cyclopropylpyridine-3-carboxamide ;
4-{[6-(5-chloro-2-fluorophényl)-2H,3H,4H-pyrido[3,2-b][1,4]oxazine-8-yl]amino}-N-(pipéridine-4-yl)pyridine-3-carboxamide ;
N-[6-(5-fluorophényl-2-yl)-2H,3H,4H-pyrido[3,2-b][1,4]oxazine-8-yl]pyridine-4-amine ;
2-(diméthylamino)éthyle 4-{ [6-(5-chloro-2-fluorophényl)-2H,3H,4H-pyrido[3,2-b][1,4]oxazine-8-yl]amino}pyridine-3-carboxylate ;
4-{[6-(5-chloro-2-fluorophényl)-2H,3H,4H-pyrido[3,2-b][1,4]oxazine-8-yl]amino}-N-[2-(4-méthylpipérazine-1-yl)éthyl]pyridine-3-carboxamide ;
2-(4-méthylpipérazine-1-yl)éthyl 4-{ [6-(5-chloro-2-fluorophényl)-2H,3H,4H-pyrido[3,2-b][1,4]oxazine-8-yl]amino}pyridine-3-carboxylate ;
méthyle 4-{[6-(6-méthylpyridine-2-yl)-2H,3H,4H-pyrido[3,2-b][1,4]oxazine-8-yl]amino}pyridine-3-carboxylate ;
4-{[6-(5-chloro-2-fluorophényl)-2H,3H,4H-pyrido[3,2-b][1,4]oxazine-8-yl]amino}-N-(1-méthylpipéridine-4-yl)pyridine-3-carboxamide ;
N-(4-{[6-(5-chloro-2-fluorophényl)-2H,3H,4H-pyrido[3,2-b][1,4]oxazine-8-yl]amino}pyridine-2-yl)-3-(4-méthylpipérazine-1-yl)propanamide ;
N-(4-{[6-(5-chloro-2-fluorophényl)-2H,3H,4H-pyrido[3,2-b][1,4]oxazine-8-yl]amino}pyridine-2-yl)-3-(diméthylamino)propanamide ;
N-{2-[bis(2-hydroxyéthyle)amino]éthyl}-4-{[6-(5-chloro-2-fluorophényl)-2H,3H,4H-pyrido[3,2-b][1,4]oxazine-8-yl]amino}pyridine-3-carboxamide ;
4-{[6-(5-chloro-2-fluorophényl)-2H,3H,4H-pyrido[3,2-b][1,4]oxazine-8-yl]amino}-N-[2-(diméthylamino)éthyle]pyridine-3-carboxamide ;
N-(1-méthylpipéridine-4-yl)-4-{[6-(6-méthylpyridine-2-yl)-2H,3H,4H-pyrido[3,2-b][1,4]oxazine-8-yl]amino}pyridine-3-carboxamide ;
4-{[6-(5-chloro-2-fluorophényl)-2H,3H,4H-pyrido[3,2-b][1,4]oxazine-8-yl]amino}-N-{2-[méthyle(oxétane-3-yl)amino]éthyl}pyridine-3-carboxamide ;
Acétate de méthyle 2-({2-[(4-{[6-(5-chloro-2-fluorophényl)-2H,3H,4H-pyrido[3,2-b][1,4]oxazine-8-yl]amino}pyridine-3-yl)formamide]éthyl}(méthyle)amino) ;
4-{[6-(5-chloro-2-fluorophényl)-2H,3H,4H-pyrido[3,2-b][1,4]oxazine-8-yl]amino}-N-[2-(4-méthanesulfonyl-pipérazine-1-yl)éthyl]pyridine-3-carboxamide ;
N-[2-(4-acétylpipérazine-1-yl)éthyl]-4-{[6-(5-chloro-2-fluorophényl)-2H,3H,4H-pyrido[3,2-b][1,4]oxazine-8-yl]amino}pyridine-3-carboxamide.

4. Composé de formule (Ia) selon la revendication 2, dans lequel **A** est **A1a** 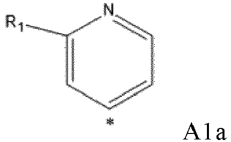 représenté par la formule (Iaa) 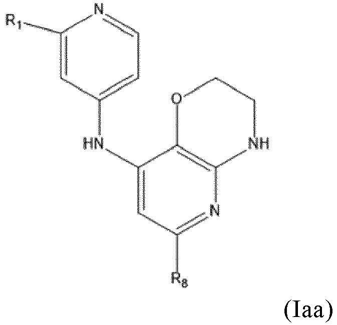
**R₁** est H ;
**R₈** est un phényle substitué par le fluor et le chlore.

5. Composé de formule (Ia) selon la revendication 2, dans lequel **A** est **A1a** 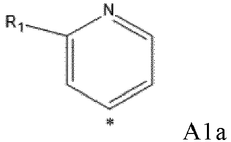 représenté par la formule (Iaa) 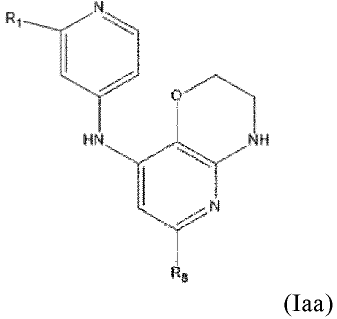
**R₁** est -NHC(O)R₆ ;
**R₆** est sélectionné dans le groupe constitué de 4-éthyl morpholine, 1-éthyl-4-méthylpipérazine et N,N-diméthyléthanamine.

6. Composé de formule (Ia) selon la revendication 2, dans lequel **A** est **A1b** , représenté par la formule (Iab) 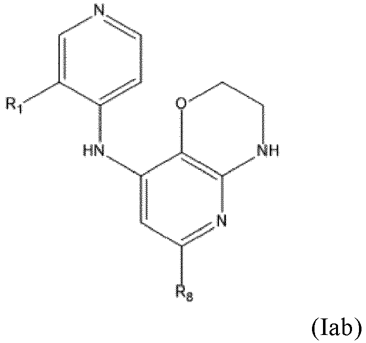
**R₁** est sélectionné dans le groupe constitué de -C(O)NHRs et -C(O)OR₇ ;
**R₅** est sélectionné dans le groupe constitué de méthyle, 2-(méthylamino)éthyle, cyclopropyle, 2-(4-méthylpipérazine-1-yl)éthyle, 2-(diméthylamino)éthyle, 2-(pipérazine-1-yl)éthyle, (pipéridine-4-yl), 1-méthylpipéridine-4-yl et 2-[bis(2-hydroxyéthyle)amino]éthyle ;
**R₇** est H ou est sélectionné dans le groupe constitué de méthyle et de 2-(pipérazine-1-yl)éthyle.

7. Composé de formule (I) selon la revendication 1, dans lequel A est le groupe A2 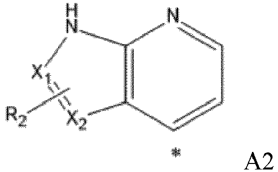 représentée par la formule (Ib) 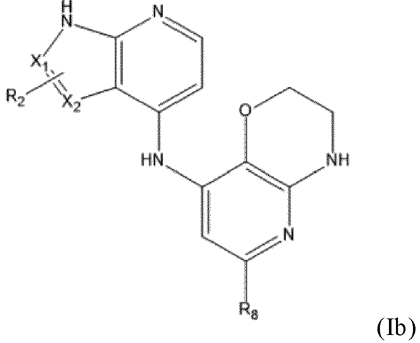
**X₁** et **X₂** sont indépendamment C ou N ;
**R₂** est H ou est sélectionné dans le groupe constitué de -C(O)OR₃ et - C(O)NR₃R₆ ; ou **R₂** est absent lorsque X₁ ou X₂ est N ;
**R₃** est H ou alkyle -(C₁-C₆) ;
**R₆** est sélectionné dans le groupe constitué d'alkylène -(C₁₋C₆)-NR₃R₄ et alkylène-hétérocycloalkyle -(C₁₋C₆), dans lequel cet hétérocycloalkyle est éventuellement substitué par un alkyle -(C₁-C₆) ;
**R₈** est sélectionné dans le groupe constitué d'aryle et d'hétéroaryle, dans lequel cet aryle est éventuellement substitué par un ou plusieurs groupes sélectionnés parmi les atomes halogènes, alkyle -(C₁-C₆) et haloalkyle -(C₁-C₄), et cet hétéroaryle est éventuellement substitué par un ou plusieurs groupes choisis parmi les alkyles -(C₁-C₆), atomes halogènes et alkyles -O-(C₁-C₆) ; et ses sels acceptables du point de vue pharmaceutique.

8. Composé de formule (Ib) selon la revendication 7 choisi parmi au moins l'un des :
6-(5-chloro-2-fluorophényl)-N-{1H-pyrazolo[3,4-b]pyridine-4-yl}-2H,3H,4H-pyrido[3,2-b][1,4]oxazine-8-amine ;
6-(5-chloro-2-fluorophényl)-N-{1H-pyrrolo[2,3-b]pyridine-4-yl}-2H,3H,4H-pyrido[3,2-b][1,4]oxazine-8-amine ;
6-(5-chloro-2-fluorophényl)-N-{3H-imidazo[4,5-b]pyridine-7-yl}-2H,3H,4H-pyrido[3,2-b][1,4]oxazine-8-amine ;
méthyle 4-{ [6-(5-chloro-2-fluorophényl)-2H,3H,4H-pyrido[3,2-b][1,4]oxazine-8-yl]amino}-1H-pyrrolo[2,3-b]pyridine-3-carboxylate ;
méthyle 4-{ [6-(5-chloro-2-fluorophényl)-2H,3H,4H-pyrido[3,2-b][1,4]oxazine-8-yl]amino}-1H-pyrrolo[2,3-b]pyridine-2-carboxylate ;
N-{3H-imidazo[4,5-b]pyridine-7-yl}-6-(6-méthylpyridine-2-yl)-2H,3H,4H-pyrido[3,2-b][1,4]oxazine-8-amine;
6-(6-méthylpyridine-2-yl)-N-{1H-pyrazolo[3,4-b]pyridine-4-yl}-2H,3H,4H-pyrido[3,2-b][1,4]oxazine-8-amine ;
6-(6-méthylpyridine-2-yl)-N-{1H-pyrrolo[2,3-b]pyridine-4-yl}-2H,3H,4H-pyrido[3,2-b][1,4]oxazine-8-amine ;
4-{[6-(5-chloro-2-fluorophényl)-2H,3H,4H-pyrido[3,2-b][1,4]oxazine-8-yl]amino}-N-[2-(1-méthylpipéridine-4-yl)éthyl]-1H-pyrrolo[2,3-b]pyridine-2-carboxamide ;
4-{[6-(5-chloro-2-fluorophényl)-2H,3H,4H-pyrido[3,2-b][1,4]oxazine-8-yl]amino}-N-[2-(diméthylamino)éthyle]-1H-pyrrolo[2,3-b]pyridine-2-carboxamide.

9. Composition pharmaceutique comprenant un composé de formule (I) selon l'une des revendications 1 à 8, en mélange avec un ou plusieurs transporteurs ou excipients acceptables du point de vue pharmaceutique.

10. Composition pharmaceutique selon la revendication 9 pour administration par inhalation.

11. Composé de formule (I) selon l'une des revendications 1 à 8 ou composition pharmaceutique selon les revendications 9 et 10 pour une utilisation comme médicament.

12. Composé de formule (I) ou composition pharmaceutique pour une utilisation selon la revendication 11 dans la prévention et/ou le traitement d'une maladie, d'un trouble ou d'une affection médié par la voie de signalisation ALK5 chez un mammifère.

13. Composé de formule (I) ou composition pharmaceutique pour une utilisation selon les revendications 11 et 12 dans la prévention et/ou le traitement de la fibrose et/ou de maladies, troubles ou affections impliquant la fibrose.

14. Composé de formule (I) ou composition pharmaceutique pour une utilisation selon la revendication 13 dans la prévention et/ou le traitement de la fibrose incluant la fibrose pulmonaire, la fibrose pulmonaire idiopathique (FPI), la fibrose hépatique, la fibrose rénale, la fibrose oculaire, la fibrose cardiaque, la fibrose artérielle et la sclérose systémique.

15. Composé de formule (I) ou composition pharmaceutique pour une utilisation selon la revendication 14 dans la prévention et/ou le traitement de la fibrose pulmonaire idiopathique (FPI).
